# EUROPEAN PATENT APPLICATION

(11) **EP 4 190 802 A1**
(43) Date of publication of application: **07.06.2023**
(21) Application number: 21842399.4
(22) Date of filing: 13.07.2021
(51) Int. Cl.: C07K 14/02, C07K 19/00, A61K 38/16, A61K 47/42, A61K 47/64

(54) **CELL-PENETRATING PEPTIDE AND USE THEREOF**

(30) Priority: 17.07.2020 CN 202010692443
(71) Applicant: Yang Sheng Tang Company, Ltd., Hangzhou, Zhejiang 310000 (CN); Xiamen University, Xiamen, Fujian 361005 (CN)
(72) Inventor: YUAN, Quan, Xiamen, Fujian 361005 (CN); WANG, Shaojuan, Xiamen, Fujian 361005 (CN); NIAN, Sheng, Xiamen, Fujian 361005 (CN); WEI, Min, Xiamen, Fujian 361005 (CN); ZHANG, Yali, Xiamen, Fujian 361005 (CN); WANG, Kai, Xiamen, Fujian 361005 (CN); CHEN, Yixin, Xiamen, Fujian 361005 (CN); ZHANG, Tianying, Xiamen, Fujian 361005 (CN); GE, Shengxiang, Xiamen, Fujian 361005 (CN); XIA, Ningshao, Xiamen, Fujian 361005 (CN)
(74) Representative: J A Kemp LLP
(86) International application number: PCT/CN2021/106043
(87) International publication number: WO 2022/012539

(57) **Abstract**

Provided is a cell-penetrating peptide, which can deliver a variety of biological macromolecules, such as proteins, antibodies, nucleic acids and so on into cells across a membrane. In addition, further provided are a fusion protein, conjugate and complex containing the cell-penetrating peptide, and a use of the cell-penetrating peptide.

## Description

### Technical Field

The present application relates to the technical field of biology, especially the technical field of transmembrane delivery. Specifically, the present application relates to a cell-penetrating peptide (cpp), which can efficiently perform the transmembrane delivery of a variety of biological macromolecules such as protein, antibody, nucleic acid, etc., into cells. In addition, the present application also relates to a fusion protein, conjugate and complex containing the cell-penetrating peptide, and a use of the cell-penetrating peptide.

### Background Art

Current biological drugs, especially antibodies, mainly target free targets and targets on the surface of cell membranes. However, in fact, many potential targets exist in cells. Due to the lack of accessibility of biological macromolecular drugs (e.g., antibodies) to these intracellular targets, the current drugs targeting these intracellular targets are mainly chemical drugs. However, there is still a gap between chemical drugs and antibodies or other biologically active macromolecules in the recognition of overexpressed antigens in certain diseases, the blocking and specificity of protein interactions, etc. For example, intracellular tumor-associated antigens or signaling pathways that promote tumorigenesis and development are potential targets for intervention, provided that active biological macromolecules can be efficiently introduced into cells to intervene on such targets.

Cell-penetrating peptides (cpp) are a class of short peptides that can cross cell membranes or tissue barriers. These peptides do not bind to specific receptors, and cross tissue barriers and cell membranes through energy-dependent or energy-independent mechanisms. Cpp can enter into cells mainly through two mechanisms: endocytosis and direct penetration, and has the advantages of high transmembrane efficiency and low cytotoxicity. Cpp can be mainly classified as cationic, amphiphilic, and hydrophobic types. Since 1988, two independent research groups reported that the HIV-1 trans-activator of transcription (TAT) can effectively cross the cell membrane and enter the cell in vitro, the study of cell-penetrating peptides has made great progress. It has been found that cpp can carry biologically active molecules (also collectively referred to as cargoes, such as proteins, polypeptides, DNA, siRNAs and small drugs, etc.) into cells. Therefore, delivery systems using cpp are also referred to as polypeptide delivery systems. Cell-penetrating peptides have been used in basic research such as transfection tools targeting multiple cell types and used for post-transfection translation studies. In addition, the transmembrane properties of cpp have been exploited to improve the delivery and therapeutic effects of drugs (including antibiotics, anti-inflammatory drugs, anti-tumor drugs, and some neuroprotective drugs) in difficult-to-access cells and tissues. Numerous preclinical studies on cell-penetrating peptides have shown promising therapeutic results in different disease models, and some of these drugs have been pushed to the clinical stage. These preclinical and clinical studies have led to unprecedented development of human therapeutics.

However, the current cell-penetrating peptides/drugs in clinical applications still have problems such as low delivery efficiency and poor targeting. Therefore, it is still necessary to develop new and efficient cell-penetrating peptides to further improve the efficiency of transmembrane delivery of biologically active macromolecules (e.g., antibodies, protein molecules such as gene editing system-related proteins, and nucleic acid molecules, etc.) into cells, thereby exerting the biological functions of the biologically active macromolecules. This new class of high-efficiency cell-penetrating peptides will provide a more efficient delivery means for therapeutic drugs targeting intracellular targets.

### Contents of the present invention

In the present invention, unless otherwise specified, scientific and technical terms used herein have the meanings commonly understood by those skilled in the art. In addition, the cell culture, molecular genetics, nucleic acid chemistry, and immunology laboratory operation steps used herein are all routine steps widely used in the corresponding fields. Meanwhile, for a better understanding of the present invention, definitions and explanations of related terms are provided below.

As used herein, the term "cell-penetrating peptide" refers to a peptide capable of performing transmembrane delivery into a cell of a molecule of interest to which it is attached. For example, the cell-penetrating peptide of the present application is capable of performing transmembrane delivery into a cell of a biological molecule of interest (e.g., a peptide of interest or nucleic acid of interest) to which it is attached. In the present application, the cell-penetrating peptide can be attached to a biological molecule of interest (e.g., a peptide of interest or a nucleic acid of interest) through covalent or non-covalent linkage.

For example, the cell-penetrating peptide of the present application can be attached to a peptide of interest or nucleic acid of interest by covalent linkage (optionally via a linker, for example, a peptide linker). Thus, in certain embodiments, the cell-penetrating peptide of the present application can be optionally fused to a peptide of interest via a peptide linker. In certain embodiments, the cell-penetrating peptide of the present application can optionally be conjugated to a peptide of interest or nucleic acid of interest through a linker (e.g., a peptide linker or a bifunctional linker). Methods for conjugating a peptide molecule to a peptide of interest or nucleic acid of interest are known in the art, for example, using various known bifunctional linkers.

In addition, the cell-penetrating peptide of the present application can be attached to a biological molecule of interest (e.g., a peptide of interest or nucleic acid of interest) in a non-covalent manner. Thus, in certain embodiments, the cell-penetrating peptide of the present application can be attached to a biological molecule of interest (e.g., a peptide of interest or nucleic acid of interest) through a specific intermolecular interaction/specific binding (e.g., interaction/binding between antigen and antibody; interaction/binding between DNA binding domain and DNA molecule).

As used herein, the term "specific binding" or "specific interaction" refers to a non-random binding reaction between two molecules, such as a reaction between an antibody and an antigen, a reaction between a DNA binding domain and a DNA molecule. For example, a non-random binding reaction between an antibody and an antigen can have a binding affinity (KD) of ≤ 10⁻⁶M. In the present application, KD refers to a ratio of dissociation velocity to association velocity (koff/kon), which can be determined by methods such as surface plasmon resonance, for example using instruments such as Biacore.

As used herein, the term "conservative substitution" refers to an amino acid substitution that does not adversely affect or alter the essential properties of a protein/polypeptide comprising an amino acid sequence. For example, conservative substitution can be introduced by standard techniques known in the art, such as site-directed mutagenesis and PCR-mediated mutagenesis. Conservative amino acid substitutions include substitutions of amino acid residues with amino acid residues having similar side chains, such as those that are physically or functionally similar to the corresponding amino acid residues (e.g., having similar size, shape, charge, chemical properties, including the ability to form covalent bonds or hydrogen bonds, etc.). Families of amino acid residues with similar side chains have been defined in the art. These families include: amino acid family with basic side chain (e.g., lysine, arginine, and histidine); amino acid family with acidic side chain (e.g., aspartic acid, glutamic acid); amino acid family with uncharged polar side chain (e.g., glycine, asparagine, glutamine, serine, threonine, tyrosine, cysteine, tryptophan); amino acid family with non-polar side chain (e.g., alanine, valine, leucine, isoleucine, proline, phenylalanine, methionine); amino acid family with β-branched side chain (e.g., threonine, valine, isoleucine); and, amino acid family with aromatic side chain (e.g., tyrosine, phenylalanine, tryptophan, histidine). Therefore, it is preferred to substitute the corresponding amino acid residue with another amino acid residue from the same side chain family. Methods for identifying conservative substitutions of amino acids are well known in the art (see, for example, Brummell et al., Biochem. 32:1180-1187 (1993); Kobayashi et al. Protein Eng. 12(10):879-884 (1999); and Burks et al. Proc. Natl Acad. Set USA 94:412-417 (1997), which are incorporated herein by reference). In the present application, the term "conservative substitution" generally refers to the replacement of a corresponding amino acid residue with another amino acid residue from the same side chain family.

As used herein, the term "N-terminal truncated by X amino acid residues" means that the N-terminal amino acid residues 1 to X of a peptide/polypeptide are deleted (X is an integer not less than 1). For example, the expression "N-terminal truncated by 5 amino acid residues" means that the N-terminal amino acid residues 1 to 5 of the peptide/polypeptide are deleted.

As used herein, the term "C-terminal truncated by X amino acid residues" means that the C-terminal last X amino acid residues of a peptide/polypeptide are deleted (X is an integer not less than 1). For example, the expression "C-terminal truncated by 8 amino acid residues" means that the C-terminal last 8 amino acid residues of the peptide/polypeptide are deleted.

In the present application, the terms "peptide", "polypeptide" and "protein" have the same meaning and are used interchangeably. And in the present application, amino acids are generally represented by one-letter and three-letter abbreviations well known in the art. For example, alanine can be represented by A or Ala.

As used herein, the term "isolated" means that a material has been artificially altered from its natural state. If a "separated" substance or component occurs in nature, it has been altered or departs from its original state, or both occur. For example, naturally occurring polynucleotides or polypeptides in a living animal are not isolated, but these polynucleotides or polypeptides can be considered "isolated" if they are sufficiently separated from the substance with which they exist in their natural state and exist in a sufficiently pure state.

As used herein, the term "vector" refers to a nucleic acid delivery vehicle into which a polynucleotide can be inserted. When the vector can express the protein encoded by the inserted polynucleotide, the vector is called an expression vector. Vectors can be used for transformation, transduction or transfection of host cells so that elements of the genetic material they carry are expressed in the host cells. For example, the vectors include: plasmid, phagemid, cosmid, artificial chromosome such as yeast artificial chromosome (YAC), bacterial artificial chromosome (BAC) or P1-derived artificial chromosome (PAC), bacteriophage such as λ-phage or M13 phage, and animal virus, etc. The types of animal viruses used as vectors are retroviruses (including lentivirus, adenovirus, adeno-associated virus, herpesvirus (e.g., herpes simplex virus), poxvirus, baculovirus, papillomavirus, papovavirus (e.g., SV40)). Vectors may contain a variety of elements to control expression, including promoter sequence, transcription initiation sequence, enhancer sequence, selection element, and reporter gene. In addition, the vector may also contain an origin of replication site. The vector may also contain a component to facilitate its entry into a cell, including, but not limited to, viral particle, liposome, or protein shell.

As used herein, the term "host cell" refers to a cell into which an exogenous polynucleotide and/or vector is introduced. The host cell described in the present application includes, but is not limited to, prokaryotic cell such as *Escherichia coli* or *Bacillus subtilis,* fungal cell such as yeast cell or *Aspergillus,* insect cell such as *S2 Drosophila* cell or Sf9, or animal cell such as fibroblast, CHO cell, COS cell, NSO cell, HeLa cell, BHK cell, HEK 293 cell or human cell.

As used herein, the term "pharmaceutically acceptable carrier or excipient" refers to a carrier or excipient that is pharmacologically and/or physiologically compatible with the subject and the active ingredient, which is well known in the art (see, for example, Remington's Pharmaceutical Sciences. Edited by Gennaro AR, 19th ed. Pennsylvania: Mack Publishing Company, 1995) and includes, but is not limited to: pH adjuster, surfactant, adjuvant, ionic strength enhancer. For example, the pH adjusting agent includes but is not limited to phosphate buffer; the surfactant includes but is not limited to cationic, anionic or nonionic surfactant such as Tween-80; the ionic strength enhancer includes but is not limited to sodium chloride.

In the present application, after intensive research, the inventors developed a new class of cell-penetrating peptides with a motif of PRRR***PRRRR*Q*PRRRR. It has been found that the cell-penetrating peptide containing such motif could efficiently deliver a biological molecule of interest (e.g., a peptide of interest or nucleic acid of interest) into a cell across a membrane and perform a function of the biological molecule in the cell.

Therefore, in one aspect, the present application provides a cell-penetrating peptide or a truncate thereof, the cell-penetrating peptide having the structure represented by Formula I:

X₁X₂X₃X₄X₅X₆X₇X₈X₉X₁₀X₁₁PRRRX₁₆X₁₇X₁₈PRRRRX₂₄QX₂₆PRRRRX₃₂X₃₃X₃₄X₃₅X₃₆X₃₇X₃₈X₃₉C Formula I

wherein,
X₁ to X₃ are each independently selected from (i) amino acid residue R; and (ii) amino acid residues (e.g., K or H) that are conservative substitutions relative to (i);
X₄ is selected from (i) amino acid residues R, C, G; and (ii) amino acid residues (e.g., K, H, N, Q, S, T, Y, W) that are conservatively substitutions relative to (i);
X₅ is selected from (i) amino acid residues R, N, G; and (ii) amino acid residues (e.g., K, H, C, Q, S, T, Y, W) that are conservatively substitutions relative to (i);
X₆ is selected from (i) amino acid residues R, G, P, S; and (ii) amino acid residues (e.g., K, H, N, Q, C, T, Y, W, A, V, L, I, M) that are conservative substitutions relative to (i);
X₇ is selected from (i) amino acid residues R, D, Q; and (ii) amino acid residues (e.g., K, H, N, G, C, S, T, Y, W, E) that are conservative substitutions relative to (i);
X₈ is selected from (i) amino acid residues R, A; and (ii) amino acid residues (e.g., K, H, V, L, I, M) that are conservative substitutions relative to (i);
X₉ is selected from (i) amino acid residues G, P, T; and (ii) amino acid residues (e.g., N, Q, S, C, Y, W, A, V, L, I, M) that are conservative substitutions relative to (i);
X₁₀ is selected from (i) amino acid residue R; and (ii) amino acid residues (e.g., K or H) that are conservative substitutions relative to (i);
X₁₁ is selected from (i) amino acid residues A, S, Q; and (ii) amino acid residues (e.g., V, L, I, M, N, G, T, C, Y, W) that are conservative substitutions relative to (i);
X₁₆ is selected from (i) amino acid residue T and (ii) amino acid residues (e.g., N, Q, G, S, C, Y, W) that are conservative substitutions relative to (i);
X₁₇ is selected from (i) amino acid residue P and (ii) amino acid residues (e.g., A, V, L, I, M) that are conservative substitutions relative to (i);
X₁₈ is selected from (i) amino acid residues S, Q and (ii) amino acid residues (e.g., N, G, T, C, Y, W) that are conservative substitutions relative to (i);
X₂₄ is selected from (i) amino acid residue S; and (ii) amino acid residues (e.g., N, Q, G, T, C, Y, W) that are conservative substitutions relative to (i);
X₂₆ is selected from (i) amino acid residues S, C, Q; and (ii) amino acid residues (e.g., N, G, T, Y, W) that are conservative substitutions relative to (i); X₃₂ is selected from (i) amino acid residues S, C; and (ii) amino acid residues (e.g., N, Q, G, T, Y, W) that are conservative substitutions relative to (i);
X₃₃ is selected from (i) amino acid residues Q, K; and (ii) amino acid residues (e.g., N, S, C, G, T, Y, W, R, H) that are conservative substitutions relative to (i);
X₃₄ is selected from (i) amino acid residues S, C; and (ii) amino acid residues (e.g., N, Q, G, T, Y, W) that are conservative substitutions relative to (i);
X₃₅ is selected from (i) amino acid residues R, P; and (ii) amino acid residues (e.g., K, H, A, V, L, I, M) that are conservative substitutions relative to (i);
X₃₆ is selected from (i) amino acid residues E, A; and (ii) amino acid residues (e.g., V, L, I, M, D) that are conservative substitutions relative to (i);
X₃₇ is selected from (i) amino acid residues P, S, C; and (ii) amino acid residues (e.g., A, V, L, I, M, N, Q, G, T, Y, W) that are conservative substitutions relative to (i);
X₃₈ is selected from (i) amino acid residues Q, S; and (ii) amino acid residues (e.g., N, C, G, T, Y, W) that are conservative substitutions relative to (i); and
X₃₉ is selected from (i) amino acid residues C, S, N; and (ii) amino acid residues (e.g., Q, G, T, Y, W) that are conservative substitutions relative to (i);
wherein, compared with the cell-penetrating peptide, the truncate is truncated by 1-10 (e.g., 1-5, for example, 1, 2, 3, 4, 5, 6, 7, 8, 9 or 10) amino acid residues at the N-terminal, and/or truncated by 1-14 (e.g., 1-8, for example, 1, 2, 3, 4, 5, 6, 7, 8, 9, 10, 11, 12, 13 or 14) amino acid residues at the C-terminal; and,
wherein, the cell-penetrating peptide or truncate thereof can perform transmembrane delivery of a biological molecule (e.g., a peptide of interest or nucleic acid of interest) into a cell.

In certain preferred embodiments, X₁ to X₃ are each independently selected from the groups consisting of amino acid residues R, K and H. In certain preferred embodiments, X₁ to X₃ are each independently selected from the group consisting of amino acid residues R and K. In certain preferred embodiments, X₁ to X₃ are each independently an amino acid residue R.

In certain preferred embodiments, X₄ is selected from the group consisting of amino acid residues R, C, G, K, H, N, Q, S, T, Y and W. In certain preferred embodiments, X₄ is selected from the group consisting of amino acid residues R, C, G, K, N, Q, S and T. In certain preferred embodiments, X₄ is selected from the group consisting of amino acid residues R, C, G and K. In certain preferred embodiments, X₄ is selected from the group consisting of amino acid residues R, C and G.

In certain preferred embodiments, X₅ is selected from the group consisting of amino acid residues R, N, G, K, H, C, Q, S, T, Y and W. In certain preferred embodiments, X₅ is selected from the group consisting of amino acid residues R, N, G, K, C, Q, S and T. In certain preferred embodiments, X₅ is selected from the group consisting of amino acid residues R, N, G, K, C and Q. In certain preferred embodiments, X₅ is selected from the group consisting of amino acid residues R, N and G.

In certain preferred embodiments, X₆ is selected from the group consisting of amino acid residues R, G, P, S, K, H, N, Q, C, T, Y, W, A, V, L, I and M. In certain preferred embodiments, X₆ is selected from the group consisting of amino acid residues R, G, P, S, K, N, Q, C and T. X₆ is selected from the group consisting of amino acid residues R, G, P, S, K, C and T. In certain preferred embodiments, X₆ is selected from the group consisting of amino acid residues R, G, P and S.

In certain preferred embodiments, X₇ is selected from the group consisting of amino acid residues R, D, Q, K, H, N, G, C, S, T, Y, W and E. In certain preferred embodiments, X₇ is selected from the group consisting of amino acid residues R, D, Q, K, N, G, C, S, T and E. In certain preferred embodiments, X₇ is selected from the group consisting of amino acid residues R, D, Q, K, N and E. In certain preferred embodiments, X₇ is selected from the group consisting of amino acid residues R, D and Q.

In certain preferred embodiments, X₈ is selected from the group consisting of amino acid residues R, A, K, H, V, L, I, and M. In certain preferred embodiments, X₈ is selected from the group consisting of amino acid residues R, A, K, V, L and I. In certain preferred embodiments, X₈ is selected from the group consisting of amino acid residues R and A.

In certain preferred embodiments, X₉ is selected from the group consisting of amino acid residues G, P, T, N, Q, S, C, Y, W, A, V, L, I and M. In certain preferred embodiments, X₉ is selected from the group consisting of amino acid residues G, P, T, N, Q, S and C. In certain preferred embodiments, X₉ is selected from the group consisting of amino acid residues G, P, T, S and C. In certain preferred embodiments, X₉ is selected from the group consisting of amino acid residues G, P and T.

In certain preferred embodiments, X₁₀ is selected from the group consisting of amino acid residues R, K and H. In certain preferred embodiments, X₁₀ is selected from the group consisting of amino acid residues R and K. In certain preferred embodiments, X₁₀ is amino acid residue R.

In certain preferred embodiments, X₁₁ is selected from the group consisting of amino acid residues A, S, V, L, I, M, N, Q, G, T, C, Y and W. In certain preferred embodiments, X₁₁ is selected from the group consisting of amino acid residues A, S, V, L, I, N, Q, G, T and C. In certain preferred embodiments, X₁₁ is selected from the group consisting of amino acid residues A, S, V, L, I and T. In certain preferred embodiments, X₁₁ is selected from the group consisting of amino acid residues A and S.

In certain preferred embodiments, X₁₆ is selected from the group consisting of amino acid residues T, N, Q, G, S, C, Y and W. In certain preferred embodiments, X₁₆ is selected from the group consisting of amino acid residues T, N, Q, G, S and C. In certain preferred embodiments, X₁₆ is selected from the group consisting of amino acid residues T and S. In certain preferred embodiments, X₁₆ is amino acid residue T.

In certain preferred embodiments, X₁₇ is selected from the group consisting of amino acid residues P, A, V, L, I and M. In certain preferred embodiments, X₁₇ is amino acid residue P.

In certain preferred embodiments, Xis is selected from the group consisting of amino acid residues S, N, Q, G, T, C, Y and W. In certain preferred embodiments, Xis is selected from the group consisting of amino acid residues S, N, Q, G, T and C. In certain preferred embodiments, Xis is selected from the group consisting of amino acid residues S, Q and T. In certain preferred embodiments, Xis is selected from the group consisting of amino acid residues S and Q.

In certain preferred embodiments, X₂₄ is selected from the group consisting of amino acid residues S, N, Q, G, T, C, Y and W. In certain preferred embodiments, X₂₄ is selected from the group consisting of amino acid residues S, N, Q, G, T and C. In certain preferred embodiments, X₂₄ is selected from the group consisting of amino acid residues S and T. In certain preferred embodiments, X₂₄ is amino acid residue S.

In certain preferred embodiments, X₂₆ is selected from the group consisting of amino acid residues S, N, Q, G, T, C, Y and W. In certain preferred embodiments, X₂₆ is selected from the group consisting of amino acid residues S, N, Q, G, T and C. In certain preferred embodiments, X₂₆ is selected from the group consisting of amino acid residues S, C and Q.

In certain preferred embodiments, X₃₂ is selected from the group consisting of amino acid residues S, C, N, Q, G, T, Y and W. In certain preferred embodiments, X₃₂ is selected from the group consisting of amino acid residues S, C, N, Q, G and T. In certain preferred embodiments, X₃₂ is selected from the group consisting of amino acid residues S, C, G and T. In certain preferred embodiments, X₃₂ is selected from the group consisting of amino acid residues S and C.

In certain preferred embodiments, X₃₃ is selected from the group consisting of amino acid residues Q, K, N, S, C, G, T, Y, W, R and H. In certain preferred embodiments, X₃₃ is selected from the group consisting of amino acid residues Q, K, N, S, C, G, T and R. In certain preferred embodiments, X₃₃ is selected from the group consisting of amino acid residues Q, K, N and R. In certain preferred embodiments, X₃₃ is selected from the group consisting of amino acid residues Q and K.

In certain preferred embodiments, X₃₄ is selected from the group consisting of amino acid residues S, C, N, Q, G, T, Y and W. In certain preferred embodiments, X₃₄ is selected from the group consisting of amino acid residues S, C, N, Q, G and T. In certain preferred embodiments, X₃₄ is selected from the group consisting of amino acid residues S, C, G and T. In certain preferred embodiments, X₃₄ is selected from the group consisting of amino acid residues S and C.

In certain preferred embodiments, X₃₅ is selected from the group consisting of amino acid residues R, P, K, H, A, V, L, I and M. In certain preferred embodiments, X₃₅ is selected from the group consisting of amino acid residues R, P, K and H. In certain preferred embodiments, X₃₅ is selected from the group consisting of amino acid residues R, P and K. In certain preferred embodiments, X₃₅ is selected from the group consisting of amino acid residues R and P.

In certain preferred embodiments, X₃₆ is selected from the group consisting of amino acid residues E, A, V, L, I, M and D. In certain preferred embodiments, X₃₆ is selected from the group consisting of amino acid residues E, A, V, L, I and D. In certain preferred embodiments, X₃₆ is selected from the group consisting of amino acid residues E and A.

In certain preferred embodiments, X₃₇ is selected from the group consisting of amino acid residues P, S, C, A, V, L, I, M, N, Q, G, T, Y and W. In certain preferred embodiments, X₃₇ is selected from the group consisting of amino acid residues P, S, C, N, Q, G and T. In certain preferred embodiments, X₃₇ is selected from the group consisting of amino acid residues P, S, C, G and T. In certain preferred embodiments, X₃₇ is selected from the group consisting of amino acid residues P, S and C.

In certain preferred embodiments, X₃₈ is selected from the group consisting of amino acid residues Q, S, N, C, G, T, Y and W. In certain preferred embodiments, X₃₈ is selected from the group consisting of amino acid residues Q, S, N, C, G and T. In certain preferred embodiments, X₃₈ is selected from the group consisting of amino acid residues Q, S, N and T. In certain preferred embodiments, X₃₈ is selected from the group consisting of amino acid residues Q and S.

In certain preferred embodiments, X₃₉ is selected from the group consisting of amino acid residues C, S, N, Q, G, T, Y and W. In certain preferred embodiments, X₃₉ is selected from the group consisting of amino acid residues C, S, N, Q, G and T. In certain preferred embodiments, X₃₉ is selected from the group consisting of amino acid residues C, S and N.

In certain preferred embodiments, the cell-penetrating peptide has the structure represented by Formula II:

RRRX₄X₅X₆X₇X₈X₉RX₁₁PRRRTPSPRRRRSQSPRRRRX₃₂X₃₃X₃₄X₃₅X₃₆X₃₇X₃₈X₃₉C Formula II

wherein,
X₄ is selected from (i) amino acid residues R, C, G; and (ii) amino acid residues (e.g., K, H, N, Q, S, T, Y, W) that are conservative substitutions relative to (i);
X₅ is selected from (i) amino acid residues R, N, G; and (ii) amino acid residues (e.g., K, H, C, Q, S, T, Y, W) that are conservative substitutions relative to (i);
X₆ is selected from (i) amino acid residues R, G, P, S; and (ii) amino acid residues (e.g., K, H, N, Q, C, T, Y, W, A, V, L, I, M) that are conservative substitutions relative to (i);
X₇ is selected from (i) amino acid residues R, D, Q; and (ii) amino acid residues (e.g., K, H, N, G, C, S, T, Y, W, E) that are conservative substitutions relative to (i);
X₈ is selected from (i) amino acid residues R, A; and (ii) amino acid residues (e.g., K, H, V, L, I, M) that are conservative substitutions relative to (i);
X₉ is selected from (i) amino acid residues G, P, T; and (ii) amino acid residues (e.g., N, Q, S, C, Y, W, A, V, L, I, M) that are conservative substitutions relative to (i);
X₁₁ is selected from (i) amino acid residues A, S, Q; and (ii) amino acid residues (e.g., V, L, I, M, N, G, T, C, Y, W) that are conservative substitutions relative to (i);
X₃₂ is selected from (i) amino acid residues S, C; and (ii) amino acid residues (e.g., N, Q, G, T, Y, W) that are conservative substitutions relative to (i);
X₃₃ is selected from (i) amino acid residues Q, K; and (ii) amino acid residues (e.g., N, S, C, G, T, Y, W, R, H) that are conservative substitutions relative to (i);
X₃₄ is selected from (i) amino acid residues S, C; and (ii) amino acid residues (e.g., N, Q, G, T, Y, W) that are conservative substitutions relative to (i);
X₃₅ is selected from (i) amino acid residues R, P; and (ii) amino acid residues (e.g., K, H, A, V, L, I, M) that are conservative substitutions relative to (i);
X₃₆ is selected from (i) amino acid residues E, A; and (ii) amino acid residues (e.g., V, L, I, M, D) that are conservative substitutions relative to (i);
X₃₇ is selected from (i) amino acid residues P, S, C; and (ii) amino acid residues (e.g., A, V, L, I, M, N, Q, G, T, Y, W) that are conservative substitutions relative to (i);
X₃₈ is selected from (i) amino acid residues Q, S; and (ii) amino acid residues (e.g., N, C, G, T, Y, W) that are conservative substitutions relative to (i); and
X₃₉ is selected from (i) amino acid residues C, S, N; and (ii) amino acid residues (e.g., Q, G, T, Y, W) that are conservative substitutions relative to (i).

In certain preferred embodiments, X₄, X₅, X₆, X₇, X₈, X₉, X₁₁, X₃₂, X₃₃, X₃₄, X₃₅, X₃₆, X₃₇, X₃₈, X₃₉ are each independently as defined above.

In certain preferred embodiments, the cell-penetrating peptide has the structure represented by Formula II:

RRRX₄X₅X₆X₇X₈X₉RX₁₁PRRRTPSPRRRRSQSPRRRRX₃₂X₃₃X₃₄X₃₅X₃₆X₃₇X₃₈X₃₉C Formula II

wherein, X₄ is selected from the group consisting of amino acid residues R, C, G;
X₅ is selected from the group consisting of amino acid residues R, N, G;
X₆ is selected from the group consisting of amino acid residues R, G, P, S;
X₇ is selected from the group consisting of amino acid residues R, D, Q;
X₈ is selected from the group consisting of amino acid residues R, A;
X₉ is selected from the group consisting of amino acid residues G, P, T;
X₁₁ is selected from the group consisting of amino acid residues A, S, Q;
X₃₂ is selected from the group consisting of amino acid residues S, C;
X₃₃ is selected from the group consisting of amino acid residues Q, K;
X₃₄ is selected from the group consisting of amino acid residues S, C;
X₃₅ is selected from the group consisting of amino acid residues R, P;
X₃₆ is selected from the group consisting of amino acid residues E, A;
X₃₇ is selected from the group consisting of amino acid residues P, S, C;
X₃₈ is selected from the group consisting of amino acid residues Q, S; and
X₃₉ is selected from the group consisting of amino acid residues C, S, N.

In certain preferred embodiments, compared to the cell-penetrating peptide, the truncate is truncated by 1-10 amino acid residues (e.g., 1-5 amino acid residues) at the N-terminal and/or truncated by 1-14 amino acid residues (e.g., 1-8 amino acid residues) at the C-terminal. In certain preferred embodiments, compared to the cell-penetrating peptide, the truncate is truncated by 1-10 amino acid residues at the N-terminal, for example, truncated by 1, 2, 3, 4, 5, 6, 7, 8, 9 or 10 amino acid residues at the N-terminal. In some preferred embodiments, compared to the cell-penetrating peptide, the truncate is truncated by 1-14 amino acid residues at the C-terminal, for example, truncated by 1, 2, 3, 4, 5, 6, 7, 8, 9, 10, 11, 12, 13 or 14 amino acid residues at the C-terminal. In certain preferred embodiments, compared to the cell-penetrating peptide, the truncate is truncated by 1-10 amino acid residues (e.g., 1-5 amino acid residues) at the N-terminal, and truncated by 1-14 amino acid residues (e.g., 1-8 amino acid residues) at the C-terminal. In certain embodiments, compared to the cell-penetrating peptide, the truncate is truncated by 5 or 10 amino acid residues at the N-terminal and truncated by 1-8 amino acid residues (e.g., 1, 3, 6 or 8 amino acid residues) at the C-terminal. In certain embodiments, compared to the cell-penetrating peptide, the truncate is truncated by 1-10 amino acid residues (e.g., 2, 3, 5, or 10 amino acid residues) at the N-terminal, and is not truncated or truncated by 1-8 amino acid residues (e.g., 1, 3, 6 or 8 amino acid residues) at the C-terminal. In some preferred embodiments, compared with the cell-penetrating peptide, the truncate is truncated by 5 amino acid residues at the N-terminal and truncated by 1-14 amino acid residues (e.g., 1, 3, 6, 8 or 14 amino acid residues) at the C-terminal. In certain preferred embodiments, compared to the cell-penetrating peptide, the truncate is truncated by 1-5 amino acid residues (e.g., 2, 3 or 5 amino acid residues) at the N-terminal, and is not truncated or truncated by 1-14 amino acid residues (e.g., 1, 3, 6, 8 or 14 amino acid residues) at the C-terminal. In certain preferred embodiments, compared to the cell-penetrating peptide, the truncate is truncated by 14 amino acid residues at the C-terminal, and X₂₆ is amino acid residue C. In certain preferred embodiments, the truncate does not have the amino acid sequence set forth in SEQ ID NO:14.

In certain preferred embodiments, the cell-penetrating peptide has the structure represented by Formula III:

RRRGX₅X₆RX₈X₉RSPRRRTPSPRRRRSQSPRRRRX₃₂QX₃₄X₃₅X₃₆X₃₇SNC Formula III

wherein,
X₅ is selected from (i) amino acid residues R, N, G; and (ii) amino acid residues (e.g., K, H, C, Q, S, T, Y, W) that are conservative substitutions relative to (i);
X₆ is selected from (i) amino acid residues R, G, P, S; and (ii) amino acid residues (e.g., K, H, N, Q, C, T, Y, W, A, V, L, I, M) that are conservative substitutions relative to (i);
X₈ is selected from (i) amino acid residues R, A; and (ii) amino acid residues (e.g., K, H, V, L, I, M) that are conservative substitutions relative to (i);
X₉ is selected from (i) amino acid residues G, P, T; and (ii) amino acid residues (e.g., N, Q, S, C, Y, W, A, V, L, I, M) that are conservative substitutions relative to (i);
X₃₂ and X₃₄ are each independently selected from (i) amino acid residues S, C; and (ii) amino acid residues (e.g., N, Q, G, T, Y, W) that are conservative substitutions relative to (i);
X₃₅ is selected from (i) amino acid residues R, P; and (ii) amino acid residues (e.g., K, H, A, V, L, I, M) that are conservative substitutions relative to (i);
X₃₆ is selected from (i) amino acid residues E, A; and (ii) amino acid residues (e.g., V, L, I, M, D) that are conservative substitutions relative to (i); and
X₃₇ is selected from (i) amino acid residues P, S, C; and (ii) amino acid residues (e.g., A, V, L, I, M, N, Q, G, T, Y, W) that are conservative substitutions relative to (i).

In certain preferred embodiments, X₅ is selected from the group consisting of amino acid residues R, N, G, K, H, C, Q, S, T, Y and W. In certain preferred embodiments, X₅ is selected from the group consisting of amino acid residues N, G, C, Q, S and T. In certain preferred embodiments, X₅ is selected from the group consisting of amino acid residues N, G, C and Q. In certain preferred embodiments, X₅ is selected from the group consisting of amino acid residues N and G.

In certain preferred embodiments, X₆ is selected from the group consisting of amino acid residues R, G, P, S, K, H, N, Q, C, T, Y, W, A, V, L, I and M. In certain preferred embodiments, X₆ is selected from the group consisting of amino acid residues P, S, N, Q, C and T. X₆ is selected from the group consisting of amino acid residues P, S and T. In certain preferred embodiments, X₆ is selected from the group consisting of amino acid residues P and S.

In certain preferred embodiments, X₈ is selected from the group consisting of amino acid residues R, A, K, H, V, L, I, and M. In certain preferred embodiments, X₈ is selected from the group consisting of amino acid residues R, A, K, V, L and I. In certain preferred embodiments, X₈ is selected from the group consisting of amino acid residues R and A. In certain preferred embodiments, X₈ is amino acid residue A.

In certain preferred embodiments, X₉ is selected from the group consisting of amino acid residues G, P, T, N, Q, S, C, Y, W, A, V, L, I and M. In certain preferred embodiments, X₉ is selected from the group consisting of amino acid residues G, P, T, N, Q, S and C. In certain preferred embodiments, X₉ is selected from the group consisting of amino acid residues P, T and S. In certain preferred embodiments, X₉ is selected from the group consisting of amino acid residues P and T.

In certain preferred embodiments, X₃₂ and X₃₄ are each independently selected from the group consisting of amino acid residues S, C, N, Q, G, T, Y and W. In certain preferred embodiments, X₃₂ and X₃₄ are each independently selected from the group consisting of amino acid residues S, C, N, Q, G and T. In certain preferred embodiments, X₃₂ and X₃₄ are each independently selected from the group consisting of amino acid residues S, C, G and T. In certain preferred embodiments, X₃₂ and X₃₄ are each independently selected from the group consisting of amino acid residues S and C. In certain preferred embodiments, X₃₂ and X₃₄ are each independently amino acid residue S.

In certain preferred embodiments, X₃₅ is selected from the group consisting of amino acid residues R, P, K, H, A, V, L, I and M. In certain preferred embodiments, X₃₅ is selected from the group consisting of amino acid residues R, P, K and H. In certain preferred embodiments, X₃₅ is selected from the group consisting of amino acid residues R, P and K. In certain preferred embodiments, X₃₅ is selected from the group consisting of amino acid residues R and P. In certain preferred embodiments, X₃₅ is amino acid residue P.

In certain preferred embodiments, X₃₆ is selected from the group consisting of amino acid residues E, A, V, L, I, M and D. In certain preferred embodiments, X₃₆ is selected from the group consisting of amino acid residues E, A, V, L, I and D. In certain preferred embodiments, X₃₆ is selected from the group consisting of amino acid residues E and A. In certain preferred embodiments, X₃₆ is amino acid residue A.

In certain preferred embodiments, X₃₇ is selected from the group consisting of amino acid residues P, S, C, A, V, L, I, M, N, Q, G, T, Y and W. In certain preferred embodiments, X₃₇ is selected from the group consisting of amino acid residues P, S, C, N, Q, G and T. In certain preferred embodiments, X₃₇ is selected from the group consisting of amino acid residues P, S, C, G and T. In certain preferred embodiments, X₃₇ is selected from the group consisting of amino acid residues P, S and C. In certain preferred embodiments, X₃₇ is selected from the group consisting of amino acid residues P and S.

In certain preferred embodiments, the cell-penetrating peptide has an amino acid sequence selected from the group consisting of SEQ ID NOs: 20-21.

In certain preferred embodiments, the truncate comprises the structure represented by Formula IV:

X₁₁PRRRTPX₁₈PRRRRSQX₂₆ Formula IV

wherein,
X₁₁ is selected from (i) amino acid residues S, Q, A; and (ii) amino acid residues (e.g., V, L, I, M, N, G, T, C, Y, W) that are conservative substitutions relative to (i);
Xis is selected from (i) amino acid residues S, Q; and (ii) amino acid residues (e.g., C, N, G, T, Y, W) that are conservative substitutions relative to (i);
X₂₆ is selected from (i) amino acid residues S, C, Q; and (ii) amino acid residues (e.g., N, G, T, Y, W) that are conservative substitutions relative to (i).

In certain preferred embodiments, X₁₁ is selected from the group consisting of amino acid residues A, S, V, L, I, M, N, Q, G, T, C, Y and W. In certain preferred embodiments, X₁₁ is selected from the group consisting of amino acid residues A, S, V, L, I, N, Q, G, T and C. In certain preferred embodiments, X₁₁ is selected from the group consisting of amino acid residues A, Q, S, V, L, I and T. In certain preferred embodiments, X₁₁ is selected from the group consisting of amino acid residues A, Q and S.

In certain preferred embodiments, Xis is selected from the group consisting of amino acid residues S, N, Q, G, T, C, Y and W. In certain preferred embodiments, Xis is selected from the group consisting of amino acid residues S, N, Q, G, T and C. In certain preferred embodiments, Xis is selected from the group consisting of amino acid residues S, Q and T. In certain preferred embodiments, Xis is selected from the group consisting of amino acid residues S and Q.

In certain preferred embodiments, X₂₆ is selected from the group consisting of amino acid residues S, N, Q, G, T, C, Y and W. In certain preferred embodiments, X₂₆ is selected from the group consisting of amino acid residues S, N, Q, G, T and C. In certain preferred embodiments, X₂₆ is selected from the group consisting of amino acid residues S, C and Q.

In certain preferred embodiments, the truncate comprises the structure represented by Formula V:

X₆X₇RGRX₁₁PRRRTPX₁₈PRRRRSQX₂₆PRRRRX₃₂ Formula V

wherein,
X₆ is selected from (i) amino acid residues R, G; and (ii) amino acid residues (e.g., K, H, C, Q, S, T, Y, W, N) that are conservative substitutions relative to (i); X₇ is selected from (i) amino acid residues R, D, Q; and (ii) amino acid residues (e.g., K, H, N, G, C, S, T, Y, W, E) that are conservative substitutions relative to (i);
X₁₁ is selected from (i) amino acid residues S, A, Q; and (ii) amino acid residues (e.g., V, L, I, M, N, G, T, C, Y, W) that are conservative substitutions relative to (i);
Xis and X₂₆ are each independently selected from (i) amino acid residues S, Q; and (ii) amino acid residues (e.g., N, G, T, C, Y, W) that are conservative substitutions relative to (i);
X₃₂ is selected from (i) amino acid residues S, C; and (ii) amino acid residues (e.g., N, Q, G, T, Y, W) that are conservative substitutions relative to (i).

In certain preferred embodiments, X₆ is selected from the group consisting of amino acid residues R, N, G, K, H, C, Q, S, T, Y and W. In certain preferred embodiments, X₆ is selected from the group consisting of amino acid residues R, N, G, K, C, Q, S and T. In certain preferred embodiments, X₆ is selected from the group consisting of amino acid residues R, N, G, K, C and Q. In certain preferred embodiments, X₆ is selected from the group consisting of amino acid residues R and G. In certain preferred embodiments, X₆ is selected from amino acid residue R.

In certain preferred embodiments, X₇ is selected from the group consisting of amino acid residues R, D, Q, K, H, N, G, C, S, T, Y, W and E. In certain preferred embodiments, X₇ is selected from the group consisting of amino acid residues R, D, Q, K, N, G, C, S, T and E. In certain preferred embodiments, X₇ is selected from the group consisting of amino acid residues R, D, Q, K, N and E. In certain preferred embodiments, X₇ is selected from the group consisting of amino acid residues R, D and Q. In certain preferred embodiments, X₇ is selected from amino acid residue R.

In certain preferred embodiments, X₁₁ is selected from the group consisting of amino acid residues A, S, V, L, I, M, N, Q, G, T, C, Y and W. In certain preferred embodiments, X₁₁ is selected from the group consisting of amino acid residues A, S, V, L, I, N, Q, G, T and C. In certain preferred embodiments, X₁₁ is selected from the group consisting of amino acid residues A, Q, S, V, L, I and T. In certain preferred embodiments, X₁₁ is selected from the group consisting of amino acid residues A, Q and S. In certain preferred embodiments, X₁₁ is selected from the group consisting of amino acid residues Q and S.

In certain preferred embodiments, Xis and X₂₆ are each independently selected from the group consisting of amino acid residues S, N, Q, G, T, C, Y and W. In certain preferred embodiments, Xis and X₂₆ are each independently selected from the group consisting of amino acid residues S, N, Q, G, T and C. In certain preferred embodiments, Xis and X₂₆ are each independently selected from the group consisting of amino acid residues S, Q and T. In certain preferred embodiments, Xis and X₂₆ are each independently selected from the group consisting of amino acid residues Q and S.

In certain preferred embodiments, X₃₂ is selected from the group consisting of amino acid residues S, C, N, Q, G, T, Y and W. In certain preferred embodiments, X₃₂ is selected from the group consisting of amino acid residues S, C, N, Q, G and T. In certain preferred embodiments, X₃₂ is selected from the group consisting of amino acid residues S, C, G and T. In certain preferred embodiments, X₃₂ is selected from the group consisting of amino acid residues S and C.

In certain preferred embodiments, the truncate comprises the structure represented by Formula VI:

X₆X₇RGRX₁₁PRRRTPX₁₈PRRRRSQX₂₆PRRRRSX₃₃SRX₃₆X₃₇ Formula VI

wherein,
X₆ is selected from (i) amino acid residues R, G; and (ii) amino acid residues (e.g., K, H, C, Q, S, T, Y, W, N) that are conservative substitutions relative to (i);
X₇ is selected from (i) amino acid residues R, D, Q; and (ii) amino acid residues (e.g., K, H, N, G, C, S, T, Y, W, E) that are conservative substitutions relative to (i);
X₁₁ is selected from (i) amino acid residues S, A, Q; and (ii) amino acid residues (e.g., V, L, I, M, N, G, T, C, Y, W) that are conservative substitutions relative to (i);
Xis and X₂₆ are each independently selected from (i) amino acid residues S, Q; and (ii) amino acid residues (e.g., N, G, T, C, Y, W) that are conservative substitutions relative to (i);
X₃₃ is selected from (i) amino acid residues Q, K; and (ii) amino acid residues (e.g., N, S, C, G, T, Y, W, R, H) that are conservative substitutions relative to (i);
X₃₆ is selected from (i) amino acid residues E, A; and (ii) amino acid residues (e.g., V, L, I, M, D) that are conservative substitutions relative to (i);
X₃₇ is selected from (i) amino acid residues S, C; and (ii) amino acid residues (e.g., N, Q, G, T, Y, W) that are conservative substitutions relative to (i).

In certain preferred embodiments, X₆ is selected from the group consisting of amino acid residues R, N, G, K, H, C, Q, S, T, Y and W. In certain preferred embodiments, X₆ is selected from the group consisting of amino acid residues R, N, G, K, C, Q, S and T. In certain preferred embodiments, X₆ is selected from the group consisting of amino acid residues R, N, G, K, C and Q. In certain preferred embodiments, X₆ is selected from the group consisting of amino acid residues R and G. In certain preferred embodiments, X₆ is selected from amino acid residue R.

In certain preferred embodiments, X₇ is selected from the group consisting of amino acid residues R, D, Q, K, H, N, G, C, S, T, Y, W and E. In certain preferred embodiments, X₇ is selected from the group consisting of amino acid residues R, D, Q, K, N, G, C, S, T and E. In certain preferred embodiments, X₇ is selected from the group consisting of amino acid residues R, D, Q, K, N and E. In certain preferred embodiments, X₇ is selected from the group consisting of amino acid residues R, D and Q. In certain preferred embodiments, X₇ is selected from amino acid residue R.

In certain preferred embodiments, X₁₁ is selected from the group consisting of amino acid residues A, S, V, L, I, M, N, Q, G, T, C, Y and W. In certain preferred embodiments, X₁₁ is selected from the group consisting of amino acid residues A, S, V, L, I, N, Q, G, T and C. In certain preferred embodiments, X₁₁ is selected from the group consisting of amino acid residues A, Q, S, V, L, I and T. In certain preferred embodiments, X₁₁ is selected from the group consisting of amino acid residues A, Q and S. In certain preferred embodiments, X₁₁ is selected from the group consisting of amino acid residues Q and S.

In certain preferred embodiments, Xis and X₂₆ are each independently selected from the group consisting of amino acid residues S, N, Q, G, T, C, Y and W. In certain preferred embodiments, Xis and X₂₆ are each independently selected from the group consisting of amino acid residues S, N, Q, G, T and C. In certain preferred embodiments, X₁₈ and X₂₆ are each independently selected from the group consisting of amino acid residues S, Q and T. In certain preferred embodiments, Xis and X₂₆ are each independently selected from the group consisting of amino acid residues Q and S.

In certain preferred embodiments, X₃₃ is selected from the group consisting of amino acid residues Q, K, N, S, C, G, T, Y, W, R and H. In certain preferred embodiments, X₃₃ is selected from the group consisting of amino acid residues Q, K, N, S, C, G, T and R. In certain preferred embodiments, X₃₃ is selected from the group consisting of amino acid residues Q, K, N and R. In certain preferred embodiments, X₃₃ is selected from the group consisting of amino acid residues Q and K. In certain preferred embodiments, X₃₃ is selected from amino acid residue Q.

In certain preferred embodiments, X₃₆ is selected from the group consisting of amino acid residues E, A, V, L, I, M and D. In certain preferred embodiments, X₃₆ is selected from the group consisting of amino acid residues E, A, V, L, I and D. In certain preferred embodiments, X₃₆ is selected from the group consisting of amino acid residues E and A. In certain preferred embodiments, X₃₆ is selected from amino acid residue E.

In certain preferred embodiments, X₃₇ is selected from the group consisting of amino acid residues S, C, N, Q, G, T, Y and W. In certain preferred embodiments, X₃₇ is selected from the group consisting of amino acid residues S, C, N, Q, G and T. In certain preferred embodiments, X₃₇ is selected from the group consisting of amino acid residues S, C, G and T. In certain preferred embodiments, X₃₇ is selected from the group consisting of amino acid residues S and C.

In certain preferred embodiments, the truncate has the structure represented by Formula VII:

RX₇RGRX₁₁PRRRTPSPRRRRSQSPRRRRX₃₂X₃₃X₃₄RX₃₆X₃₇QX₃₉ Formula VII

wherein,
X₇ is selected from (i) amino acid residues R, D, Q; and (ii) amino acid residues (e.g., K, H, N, G, C, S, T, Y, W, E) that are conservative substitutions relative to (i);
X₁₁ is selected from (i) amino acid residues A, S; and (ii) amino acid residues (e.g., V, L, I, M, N, Q, G, T, C, Y, W) that are conservative substitutions relative to (i);
X₃₂, X₃₄, X₃₇ and X₃₉ are each independently selected from (i) amino acid residues S, C; and (ii) amino acid residues (e.g., N, Q, G, T, Y, W) that are conservative substitutions with respect to (i);
X₃₃ is selected from (i) amino acid residues Q, K; and (ii) amino acid residues (e.g., N, S, C, G, T, Y, W, R, H) that are conservative substitutions relative to (i); and
X₃₆ is selected from (i) amino acid residues E, A; and (ii) amino acid residues (e.g., V, L, I, M, D) that are conservative substitutions relative to (i).

In certain preferred embodiments, X₇ is selected from the group consisting of amino acid residues R, D, Q, K, H, N, G, C, S, T, Y, W and E. In certain preferred embodiments, X₇ is selected from the group consisting of amino acid residues R, D, Q, K, N, G, C, S, T and E. In certain preferred embodiments, X₇ is selected from the group consisting of amino acid residues R, D, Q, K, N and E. In certain preferred embodiments, X₇ is selected from the group consisting of amino acid residues R, D and Q. In certain preferred embodiments, X₇ is selected from the group consisting of amino acid residues R and Q.

In certain preferred embodiments, X₁₁ is selected from the group consisting of amino acid residues A, S, V, L, I, M, N, Q, G, T, C, Y and W. In certain preferred embodiments, X₁₁ is selected from the group consisting of amino acid residues A, S, V, L, I, N, Q, G, T and C. In certain preferred embodiments, X₁₁ is selected from the group consisting of amino acid residues A, S, V, L, I and T. In certain preferred embodiments, X₁₁ is selected from the group consisting of amino acid residues A and S.

In certain preferred embodiments, X₃₂, X₃₄, X₃₇ and X₃₉ are each independently selected from the group consisting of amino acid residues S, C, N, Q, G, T, Y and W. In certain preferred embodiments, X₃₂, X₃₄, X₃₇ and X₃₉ are each independently selected from the group consisting of amino acid residues S, C, N, Q, G and T. In certain preferred embodiments, X₃₂, X₃₄, X₃₇ and X₃₉ are each independently selected from the group consisting of amino acid residues S, C, G and T. In certain preferred embodiments, X₃₂, X₃₄, X₃₇ and X₃₉ are each independently selected from the group consisting of amino acid residues S and C.

In certain preferred embodiments, X₃₃ is selected from the group consisting of amino acid residues Q, K, N, S, C, G, T, Y, W, R and H. In certain preferred embodiments, X₃₃ is selected from the group consisting of amino acid residues Q, K, N, S, C, G, T and R. In certain preferred embodiments, X₃₃ is selected from the group consisting of amino acid residues Q, K, N and R. In certain preferred embodiments, X₃₃ is selected from the group consisting of amino acid residues Q and K.

In certain preferred embodiments, X₃₆ is selected from the group consisting of amino acid residues E, A, V, L, I, M and D. In certain preferred embodiments, X₃₆ is selected from the group consisting of amino acid residues E, A, V, L, I and D. In certain preferred embodiments, X₃₆ is selected from the group consisting of amino acid residues E and A.

In certain preferred embodiments, the cell-penetrating peptide has an amino acid sequence selected from the group consisting of SEQ ID NOs: 20-21.

In certain preferred embodiments, the cell-penetrating peptide or truncate thereof has an amino acid sequence selected from the group consisting of: SEQ ID NOs: 10-13, 15, 17-18, 20-21, 23-29 and 32-37.

In certain preferred embodiments, the cell-penetrating peptide or truncate is isolated.

In another aspect, the present application provides a fusion protein, which comprises the cell-penetrating peptide or truncate thereof as described above, and a peptide of interest. The cell-penetrating peptide of the present application or truncate thereof can perform transmembrane delivery of the peptide of interest (the fusion protein) attached thereto into a cell.

In certain preferred embodiments, the peptide of interest is directly covalently linked (i.e., directly linked via a peptide bond) to the cell-penetrating peptide or truncate thereof. In certain preferred embodiments, the peptide of interest is covalently linked to the cell-penetrating peptide or truncate thereof through a peptide linker (e.g., a flexible peptide linker). In certain preferred embodiments, the peptide linker has the amino acid sequence set forth in SEQ ID NO:39. However, it is readily understood that in the present application, various known peptide linkers can be used to fuse the peptide of interest to the cell-penetrating peptide or truncate thereof.

In certain preferred embodiments, the cell-penetrating peptide or truncate thereof is linked to the N-terminal of the peptide of interest (optionally, via a peptide linker). In certain preferred embodiments, the cell-penetrating peptide or truncate thereof is linked to the C-terminal of the peptide of interest (optionally, via a peptide linker).

It is easy to understand that the peptide of interest in the present application can be any desired protein or polypeptide. For example, in certain circumstances, it may be desirable to deliver an antibody into a cell (e.g., tumor cell, immune cell, etc.) and perform its function (e.g., inhibiting viral infection, replication and/or assembly, inhibiting or enhancing intracellular signaling, etc.). In this case, the cell-penetrating peptide or truncate thereof of the present application can be fused to the antibody of interest to facilitate transmembrane delivery of the antibody. Thus, in certain preferred embodiments, the peptide of interest is an antibody. In certain preferred embodiments, the antibody is selected from anti-HBV antibody (e.g., anti-HBsAg antibody, anti-HBcAg antibody, anti-HBeAg antibody, etc.), anti-influenza virus antibody (e.g., anti-HA1 antibody, anti-HA2 antibody), anti-tumor antigen antibody (e.g., anti-p53 antibody, anti-kras antibody, anti-PRL-3 antibody), anti-immune checkpoint antibody (e.g., anti-PD1 or PDL1 antibody), anti-melanin synthesis-related antibody (e.g., tyrosinase-related protein TYRP1 antibody), anti-coronavirus antibody (e.g., anti-coronavirus S protein antibody, such as anti-S protein RBD or S1 or S2 antibody).

In some cases, it may be desirable to deliver a gene editing-related protein into a cell and perform its function (e.g., to edit a gene of interest in a cell, or to inhibit gene editing in a cell, etc.). In this case, the cell-penetrating peptide or truncate thereof of the present application can be fused to a gene editing-related protein to facilitate transmembrane delivery of the protein. Therefore, in certain preferred embodiments, the peptide of interest is a protein associated with gene editing. In certain preferred embodiments, the protein is selected from the group consisting of Cas9 protein, AcrIIA4 protein, Cas13 protein, Cre recombinase and Flip recombinase.

In some cases, it may be desirable to deliver an active or traceable protein into a cell and perform its function (e.g., to study or alter signaling pathway, localize cell, etc.). In this case, the cell-penetrating peptide or truncate thereof of the present application may be fused to such protein to facilitate transmembrane delivery of the protein. Thus, in certain preferred embodiments, the peptide of interest is an active or traceable protein. In certain preferred embodiments, the protein is selected from the group consisting of fluorescent protein (e.g., green fluorescent protein), toxin protein (e.g., endotoxin), cytokine (e.g., interleukin or interferon, for example, IL10 or IFNγ), immunomodulatory protein (e.g., PD1), enzyme (e.g., luciferase, nuclease, recombinase, methylase, protein kinase, etc.), signaling pathway-related molecular protein (e.g., β-catenin protein), cyclin (e.g., Cyclin D1 protein), transcription activator and transcription repressor.

In certain circumstances, it may be desirable to deliver a DNA molecule into a cell and perform its functions (e.g., to express foreign protein, interfere with endogenous gene expression, etc.). In this case, the cell-penetrating peptide or truncate thereof of the present application may be fused to a protein capable of binding a DNA molecule (hereinafter also referred to as DNA binding protein) to facilitate transmembrane delivery of the protein and DNA molecule. Thus, in certain preferred embodiments, the peptide of interest is a protein capable of binding a DNA molecule. In certain preferred embodiments, the protein is selected from the group consisting of zinc finger protein and transcription activator-like effector nuclease (TALEN protein).

In certain preferred embodiments, the peptide of interest has an amino acid sequence selected from the group consisting of SEQ ID NOs: 1, 6-7, 43-44 and 46-53.

In certain preferred embodiments, the fusion protein further comprises an additional domain. In certain preferred embodiments, the fusion protein further comprises a tag domain (e.g., 6*His tag, HA tag, hapten tag, Strep tag, MBP tag, GST tag, etc.) to facilitate the preparation and purification of the fusion protein. In certain preferred embodiments, the fusion protein further comprises an antibody heavy chain constant region. In certain preferred embodiments, the additional domain (e.g., tag domain) is located at the N-terminal of the fusion protein. In certain preferred embodiments, the additional domain (e.g., tag domain) is located at the C-terminal of the fusion protein.

In another aspect, the present application provides a conjugate, which comprises the cell-penetrating peptide or truncate thereof as described above, and a molecule of interest (e.g., a protein of interest or nucleic acid of interest).

In certain preferred embodiments, the molecule of interest is directly covalently linked (i.e., directly linked via a chemical bond) to the cell-penetrating peptide or truncate thereof. In certain preferred embodiments, the peptide of interest is covalently linked to the cell-penetrating peptide or truncate thereof through a linker (e.g., a bifunctional linker or peptide linker). In certain preferred embodiments, the peptide linker has the amino acid sequence set forth in SEQ ID NO:39. However, it is readily understood that in the present application, various known linkers can be used to covalently link the molecule of interest to the cell-penetrating peptide or truncate thereof. In certain preferred embodiments, the molecule of interest is non-covalently linked to the cell-penetrating peptide or truncate thereof.

It is easy to understand that the cell-penetrating peptide or truncate thereof in the present application can be linked to various molecule of interests. These molecule of interests can be linked to the cell-penetrating peptide or truncate thereof by other means such as covalent binding, affinity binding, intercalation, coordinate binding, complexation, binding, mixing or addition. In certain embodiments, the cell-penetrating peptide or truncate thereof disclosed in the present application can be engineered to contain an additional specific site that can be used to bind one or more molecules of interest. For example, such site may contain one or more reactive amino acid residues, such as cysteine residues and histidine residues, to facilitate covalent attachment to the molecule of interest. In certain embodiments, the cell-penetrating peptide or truncate thereof can be indirectly linked to the molecule of interest. For example, the cell-penetrating peptide or truncate thereof can bind biotin and then indirectly bind a second molecule of interest, which is linked to avidin.

It is easy to understand that the molecule of interest in the present application can be any desired molecule. For example, in certain embodiments, the molecule of interest can be a detectable label. Examples of detectable label may include fluorescent label (e.g., fluorescein, rhodamine, dansyl, phycoerythrin, or Texas red), enzyme label (e.g., horseradish peroxidase, alkaline phosphatase, luciferase, glucoamylase, lysozyme, glucose oxidase or β-D galactosidase), stable isotope or radioisotope, chromophore moiety, digoxigenin, biotin/avidin, DNA molecule or gold for easy detection. In certain embodiments, the molecule of interest can be a cytotoxic agent. The "cytotoxic agent" can be any agent that is detrimental to a cell or that may damage or kill a cell. Examples of cytotoxic agent include, but are not limited to, paclitaxel, cytochalasin B, gramicidin D, ethidium bromide, ipecamine, mitomycin, etoposide, teniposide, vincristine, vinblastine, colchicine, doxorubicin, daunorubicin, dihydroxyanthracin diketone, mitoxantrone, mithramycin, actinomycin D, 1-dehydrotestosterone, glucocorticoid, procaine, tetracaine, lidocaine, propranolol, puromycin and analog thereof, antimetabolite (e.g., methotrexate, 6-mercaptopurine, 6-mercaptoguanine, cytosine arabinoside, 5-fluorouracil dcrbzine), alkylating agent (e.g., mustine, thiotepa chlorambucil, melphalan, carmustine (BSNU) and lomustine (CCNU), cyclophosphamide, Busulfan, dibromomannitol, streptozotocin, mitomycin C, and cis-dichlorodiamineplatinum (DDP)), anthracycline (e.g., idarubicin (formerly daunorubicin) and doxorubicin), antibiotic (e.g., genshammycin (formerly known as actinomycin), bleomycin, mithramycin, and anthiamycin (AMC)), as well as antimitotic agent (e.g., vincristine and vinblastine).

In certain embodiments, the molecule of interest can be any desired protein or polypeptide. For example, in certain circumstances it may be desirable to deliver an antibody into a cell (e.g., tumor cell, immune cell, etc.) and perform its function (e.g., to inhibit viral infection, replication and/or assembly, to inhibit or enhance intracellular signaling, etc.). Thus, in certain preferred embodiments, the molecule of interest is an antibody. In certain preferred embodiments, the antibody is selected from anti-HBV antibody (e.g., anti-HBsAg antibody, anti-HBcAg antibody, anti-HBeAg antibody, etc.), anti-influenza virus antibody (e.g., anti-HA1 antibody, anti-HA2 antibody), antibody against tumor antigen (e.g., anti-p53 antibody, anti-kras antibody, anti-PRL-3 antibody), anti-immune checkpoint antibody (e.g., anti-PD1 or PDL1 antibody), anti-melanin synthesis-related antibody (e.g., tyrosinase-related protein TYRP1 antibody), anti-coronavirus antibody (e.g., anti-coronavirus S protein antibody, such as anti-S protein RBD or S1 or S2 antibody).

In some cases, it may be desirable to deliver a gene editing-related protein into a cell and perform its function (e.g., to edit a gene of interest in a cell, or to inhibit gene editing in a cell, etc.). Therefore, in certain preferred embodiments, the molecule of interest is a protein associated with gene editing. In certain preferred embodiments, the protein is selected from the group consisting of Cas9 protein, AcrIIA4 protein, Cas13 protein, Cre recombinase and Flip recombinase.

In some cases, it may be desirable to deliver an active or traceable protein into a cell and perform its function (e.g., to study or alter signaling pathway, localize cells, etc.). Thus, in certain preferred embodiments, the molecule of interest is an active or traceable protein. In certain preferred embodiments, the protein is selected from the group consisting of fluorescent protein (e.g., green fluorescent protein), toxin protein (e.g., endotoxin), cytokine (e.g., interleukin or interferon, for example, IL10 or IFNγ), immunomodulatory protein (e.g., PD1), enzyme (e.g., luciferase, nuclease, recombinase, methylase, protein kinase, etc.), signaling pathway-related molecular protein (e.g., β-catenin protein), cyclin (e.g., Cyclin D1 protein), transcription activator and transcription repressor.

In certain circumstances, it may be desirable to deliver a DNA molecule into a cell and perform its function (e.g., to express foreign protein, interfere with endogenous gene expression, etc.). Thus, in certain preferred embodiments, the molecule of interest is a protein capable of binding DNA molecule. In certain preferred embodiments, the protein is selected from the group consisting of zinc finger protein and transcription activator-like effector nuclease (TALEN protein).

In certain preferred embodiments, the molecule of interest is a polypeptide having an amino acid sequence selected from the group consisting of SEQ ID NOs: 1, 6-7, 43-44 and 46-53.

In another aspect, the present application provides a multimer, which comprises two or more fusion proteins or conjugates as described above. Without being bound by any theory, in some cases, a multimeric form may be advantageous, which can further facilitate the intracellular delivery of the fusion proteins or conjugates.

In another aspect, the present application provides a complex, which comprises the fusion protein or conjugate or multimer as described above, and a component non-covalently bound or complexed with the fusion protein or conjugate or multimer. In certain preferred embodiments, the fusion protein or conjugate or multimer comprises a protein capable of binding a DNA molecule that is linked to the cell-penetrating peptide or truncate thereof of the present application; and, the complex comprises a DNA molecule that is non-covalently bound or complexed with the protein capable of binding the DNA molecule. In certain preferred embodiments, the protein capable of binding the DNA molecule is covalently linked (optionally, via a linker) to the cell-penetrating peptide or truncate thereof. In certain preferred embodiments, the protein capable of binding the DNA molecule is non-covalently linked (optionally, via a linker) to the cell-penetrating peptide or truncate thereof. In certain preferred embodiments, the protein capable of binding the DNA molecule is linked to the N-terminal of the cell-penetrating peptide or truncate thereof. In certain preferred embodiments, the protein capable of binding the DNA molecule is linked to the C-terminal of the cell-penetrating peptide or truncate thereof. In certain preferred embodiments, the DNA molecule comprises a nucleotide sequence that is recognized and bound by the protein capable of binding the DNA molecule. In certain preferred embodiments, the protein capable of binding the DNA molecule is selected from the group consisting of zinc finger protein and transcription activator-like effector nuclease (TALEN proteins). In certain preferred embodiments, the nucleotide sequence is the binding sequence of zinc finger protein (e.g., SEQ ID NO: 54).

In another aspect, the present application provides an isolated nucleic acid molecule, which comprises a nucleotide sequence encoding the cell-penetrating peptide or truncate thereof or the fusion protein.

In another aspect, the present application provides a vector (e.g., a cloning vector or an expression vector), which comprises the isolated nucleic acid molecule as described above. In certain embodiments, the vector of the present application is selected from plasmid, cosmid, artificial chromosome such as yeast artificial chromosome (YAC), bacterial artificial chromosome (BAC) or P1-derived artificial chromosome (PAC), bacteriophage such as λ-phage or M13 phage, and animal virus, etc.

In another aspect, the present application provides a host cell, which comprises the isolated nucleic acid molecule or the vector as described above. In certain embodiments, the host cell is a prokaryotic cell, which includes, but is not limited to, Gram-negative bacteria or Gram-positive bacteria, for example, *Enterobacteriaceae* (e.g., *Escherichia coli*)*, Enterobacter, Erwinia, Klebsiella, Proteus, Salmonella,* such as, *Salmonella typhimurium, Serratia,* such as *Serratia marcescens,* and *Shigella,* and *Bacillus* such as *Bacillus subtilis* and *Bacillus licheniformis, Pseudomonas* such as *Pseudomonas aeruginosa* and *Streptomyces.* In certain embodiments, the host cell is, for example, an *E. coli* or *B. subtilis* cell.

In certain embodiments, the host cell is an eukaryotic cell, for example, fungal cell such as yeast cell or *Aspergillus,* insect cell such as *S2 Drosophila* cell or *Sf9*, or animal cell such as fibroblast, CHO cell, COS cell, NSO cell, HeLa cell, BHK cell, HEK 293 cell or human cell. In certain embodiments, the host cell is a mammalian cell. In certain embodiments, the host cell is a human, murine, ovine, equine, dog or feline cell. In certain embodiments, the host cell is a Chinese hamster ovary cell.

In another aspect, the present application provides a method for preparing the cell-penetrating peptide or truncate thereof or fusion protein, which comprises, under conditions that allow the expression of the cell-penetrating peptide or truncate thereof or fusion protein, the host cell as described above is cultured, and the cell-penetrating peptide or truncate thereof or the fusion protein is recovered from a culture of the cultured host cell.

In another aspect, the present application provides a composition, which comprises a fusion protein or conjugate or multimer or complex or nucleic acid molecule or vector as described above.

In another aspect, the present application provides a pharmaceutical composition, which comprises the fusion protein or conjugate or multimer or complex as described above, and a pharmaceutically acceptable carrier or excipient, wherein the fusion protein or conjugate or multimer or complex comprises a therapeutically active polypeptide linked to the cell-penetrating peptide or truncate thereof of the present application. In certain preferred embodiments, the therapeutically active polypeptide is covalently linked (optionally, via a linker) to the cell-penetrating peptide or truncate thereof. In certain preferred embodiments, the therapeutically active polypeptide is non-covalently linked (optionally, via a linker) to the cell-penetrating peptide or truncate thereof. In certain preferred embodiments, the therapeutically active polypeptide is linked to the N-terminal of the cell-penetrating peptide or truncate thereof. In certain preferred embodiments, the therapeutically active polypeptide is linked to the C-terminal of the cell-penetrating peptide or truncate thereof. It is readily understood that the therapeutically active polypeptide can be any polypeptide desired to be delivered into a cell through transmembrane delivery, examples thereof include toxin protein (e.g., endotoxin), cytokine (e.g., interleukin or interferon such as IL10 or IFNγ), immunomodulatory protein (e.g., PD1), antibody, etc. In certain preferred embodiments, the antibody is selected from anti-HBV antibody (e.g., anti-HBsAg antibody, anti-HBcAg antibody, anti-HBeAg antibody, etc.), anti-influenza virus antibody (e.g., anti-HA1 antibody, anti-HA2 antibody), anti-tumor antigen antibody (e.g., anti-p53 antibody, anti-kras antibody, anti-PRL-3 antibody), anti-immune checkpoint antibody (e.g., anti-PD1 or PDL1 antibody), anti-melanin synthesis-related antibody (e.g., tyrosinase-related protein TYRP1 antibody), anti-coronavirus antibody (e.g., anti-coronavirus S protein antibody, such as anti-S protein RBD or S1 or S2 antibody).

In another aspect, the present application provides a method for transmembrane delivery of a molecule of interest into a cell, comprising linking the above-described cell-penetrating peptide or truncate thereof to the molecule of interest, and then contacting the molecule of interest with the cell.

In certain preferred embodiments, the method comprises: (1) linking the molecule of interest with the cell-penetrating peptide or truncate thereof to obtain a conjugate; (2) contacting the conjugate with the cell, thereby delivering the molecule of interest into the cell through transmembrane delivery.

It is easy to understand that in the present application, the cell-penetrating peptide or truncate thereof can be linked to the molecule of interest in various ways. For example, the molecule of interest can be linked to the cell-penetrating peptide or truncate thereof by other means such as covalent binding, affinity binding, intercalation, coordinate binding, complexation, binding, mixing or addition. In certain preferred embodiments, in step (1), the molecule of interest is directly covalently linked to the cell-penetrating peptide or truncate thereof (i.e., directly linked by a chemical bond). In certain preferred embodiments, in step (1), the peptide of interest is covalently linked to the cell-penetrating peptide or truncate thereof through a linker (e.g., a bifunctional linker or peptide linker). In certain preferred embodiments, in step (1), the molecule of interest is linked to the cell-penetrating peptide or truncate thereof in a non-covalent manner. In certain preferred embodiments, the molecule of interest and the cell-penetrating peptide or truncate thereof are as defined above, respectively.

In certain preferred embodiments, the molecule of interest is a peptide of interest, and the method comprises: (1) linking the molecule of interest with the cell-penetrating peptide or a truncate thereof to obtain a fusion protein; (2) contacting the fusion protein with the cell, thereby delivering the molecule of interest into the cell through transmembrane delivery.

It is easy to understand that in the present application, the cell-penetrating peptide or truncate thereof can be linked with the peptide of interest in various ways to obtain a fusion protein. In certain preferred embodiments, in step (1), the peptide of interest is directly covalently linked (i.e., directly linked by a peptide bond) to the cell-penetrating peptide or truncate thereof to obtain a fusion protein. In certain preferred embodiments, in step (1), the peptide of interest is covalently linked to the cell-penetrating peptide or truncate thereof through a peptide linker (e.g., a flexible peptide linker) to obtain a fusion protein. In certain preferred embodiments, the peptide linker has the amino acid sequence set forth in SEQ ID NO:39. However, it is readily understood that in the present application, various known peptide linkers can be used to fuse the peptide of interest to the cell-penetrating peptide or truncate thereof. In certain preferred embodiments, in step (1), the cell-penetrating peptide or truncate thereof is linked to the N-terminal of the peptide of interest (optionally, via a peptide linker). In certain preferred embodiments, in step (1), the cell-penetrating peptide or truncate thereof is linked to the C-terminal of the peptide of interest (optionally, via a peptide linker). In certain preferred embodiments, the peptide of interest and the cell-penetrating peptide or truncate thereof are as defined above, respectively.

In certain preferred embodiments, the molecule of interest is a nucleic acid of interest, and the method comprises: (1) linking a protein capable of binding the nucleic acid of interest to the cell-penetrating peptide or truncate thereof; (2) contacting the product of step (1) with the nucleic acid of interest to obtain a complex; and (3) contacting the complex with a cell, thereby delivering the nucleic acid of interest into the cell through transmembrane delivery.

In certain preferred embodiments, the molecule of interest is a nucleic acid of interest, and the method comprises: (1) adding a nucleotide sequence to the nucleic acid of interest, which can be recognized and bound by a DNA-binding protein; (2) linking the DNA-binding protein with the cell-penetrating peptide or truncate thereof; (3) contacting the product of step (1) with the product of step (2) to obtain a complex; and (4) contacting the complex with a cell, thereby delivering the nucleic acid of interest into the cell through transmembrane delivery. It is readily understood that in the present application, various known DNA binding proteins, as well as the nucleotide sequences they recognize and bind, can be used. In certain preferred embodiments, the DNA-binding protein, the nucleotide sequence it recognizes and binds, and the cell-penetrating peptide or truncate thereof are as defined above, respectively.

In another aspect, the present application relates to a use of the cell-penetrating peptide or truncate thereof as described above for transmembrane delivery of a molecule of interest into a cell. In another aspect, the present application relates to a use of the cell-penetrating peptide or truncate thereof as described above in the manufacture of a kit for transmembrane delivery of a molecule of interest into a cell.

### Beneficial Effects of Invention

The cell-penetrating peptide or truncate thereof of the present application can deliver a biological molecule of interest (e.g., peptide of interest or nucleic acid of interest) into a cell through transmembrane delivery, and exert the function of the biological molecule in the cell. Compared with the prior art, the delivery efficiency of the cell-penetrating peptide or truncate thereof of the present application is high (significantly higher than the TAT peptide derived from HIV virus), so it has good application prospect and value.

The embodiments of the present invention will be described in detail below with reference to the drawings and examples, but those skilled in the art will understand that the following drawings and examples are only used to illustrate the present invention, rather than limit the scope of the present invention. Various objects and advantageous aspects of the present invention will become apparent to those skilled in the art from the accompanying drawings and the following detailed description of the preferred embodiments.

### Brief Description of the Drawings

Fig. 1A shows schematically the working principle of the reporting system constructed in Example 1.
Fig. 1B shows schematically the main structural elements contained in the plasmid EHIPS-U2MG constructed in Example 1.
Fig. 1C shows the fluorescence microscopy observation results of engineered cell lines transfected with pcDNA3.1-GFP plasmid or negative control plasmid.
Fig. 2 shows the SDS-PAGE detection results of purified target proteins (GFP, GFP-CPP28 and GFP-TAT). M: protein molecular weight marker.
Figs. 3A to 3B show the observation results of the 293β5 cells cultured for 36 h in medium with or without the protein of interest (GFP, GFP-CPP28 or GFP-TAT) using a laser confocal high-content imaging analysis system (Fig. 3A) and the results of quantitative analysis of the mCherry fluorescence intensity of the cells (Fig. 3B), wherein the 293β5 cells were transfected with EHIPS-U2MG plasmid.
Figs. 4A to 4B show the observation results of the 293β5 cells cultured in medium containing GFP-CPP28 (30, 10, 5, 2.5 µg/mL) for 36 h using a laser confocal high-content imaging analysis system (Fig. 4A) and the results of quantitative analysis of mCherry fluorescence intensity of the cells (Fig. 4B), wherein the 293β5 cells were transfected with the EHIPS-U2MG plasmid.
Fig. 5 shows the analysis results of various types of cells (MDCK, VERO and Hacat cells) and their culture supernatants cultured in medium containing target proteins (GFP, GFP-CPP28, or GFP-TAT) for 36h using a laser confocal high-content imaging analysis system and a luciferase detection system, respectively, wherein the various types of cells were transfected with EHIPS-U2MG plasmid.
Fig. 6A shows the observation results of Hela-p63mRb3 cells at the indicated time points using of a confocal microscopy, wherein the Hela-p63mRb3 cells were cultured in medium containing GFP-CPP28 (60 µg/mL).
Fig. 6B shows the detection results of the GFP protein content in Hela-p63mRb3 cells at the indicated time points using a double-antibody sandwich assay, wherein the Hela-p63mRb3 cells were cultured in medium containing GFP-CPP28 (60 µg/mL).
Figs. 7A to 7B show confocal microscopy observation results (Fig. 7A) and GFP fluorescence intensity analysis results (Fig. 7B) of the Hela-p63mRb3 cells, wherein the Hela-p63mRb3 cells were first treated with the indicated endocytic pathway inhibitor or low temperature (4°C) for 2 h, and then treated with 30 µg/ml GFP-cpp28 protein for 1 h.
Figs. 8A to 8B show the observation results of the 293β5 cells cultured in media containing various recombinant proteins at the indicated concentrations for 36 h using a laser confocal high-content imaging analysis system, wherein the 293β5 cells were transfected with EHIPS-U2MG plasmid.
Figs. 9A to 9B show the results of GFP fluorescence intensity analysis (Fig. 9A) and confocal microscopy observation results (Fig. 9B) of the Hela-p63mRb3 cells at the indicated time points, the Hela-p63mRb3 cells were cultured in media containing GFP-cpp28, GFP-cpp28a, GFP-cpp28b, GFP-cpp28c, GFP-cpp28d and GFP-TAT (60 µg/mL).
Figs. 10A to 10B show the intracellular GFP fluorescent spot count results (Fig. 10A) and confocal microscopy observation results (Fig. 10B) of Hela-p63mRb3 cells cultured for 1 h in media containing GFP-cpp28, GFP-cpp28oS, GFP-cpp28a, GFP-cpp28aC, GFP-cpp28b and GFP-cpp28bC (60 µg/mL).
Figs. 11A to 11B show the confocal microscopy observation results (Fig. 11A) and intracellular GFP fluorescence intensity results (Fig. 11B) of the Hela-p63mRb3 cells cultured for 1 h in the media containing GFP-cpp28, GFP-cpp28aC, GFP-cpp28bC, GFP-cpp28cC, GFP-cpp28dC, GFP-cpp28dREC and GFP-cpp-oS at different concentrations (6.25 µg/mL, 12.5 µg/mL, 25 µg/mL, 50 µg/mL, 100 µg/mL, 200 µg/mL).
Figs. 12A to 12B show the confocal microscopy observation results (Fig. 12A ) and intracellular GFP fluorescence intensity results (Fig. 12B) of the Hela-p63mRb3 cells cultured for 1 h in the media containing GFP-cpp28, GFP-cpp28a, GFP-cpp28b, GFP-cpp28c, GFP-cpp28d, GFP-cpp28oNT-1, GFP-cpp28oNT-2 and GFP-cpp28oNT-3 at different concentrations (6.25 µg/mL, 12.5 µg/mL, 25 µg/mL, 50 µg/mL, 100 µg/mL, 200 µg/mL).
Figs. 13A to 13B show the confocal microscopy observation results (Fig. 13A) and intracellular GFP fluorescence intensity results (Fig. 13B) of the Hela-p63mRb3 cells cultured for 1 h in the media containing GFP-cpp28, GFP-cpp28aCQ1, GFP-cpp28aCQ2, GFP-cpp28aCQ3, GFP-cpp28aCNT, GFP-cpp28bCNT, GFP-cpp28cCNT, and GFP-cpp28dCNT at different concentrations (6.25 µg/mL, 12.5 µg/mL, 25 µg/mL, 50 µg/mL, 100 µg/mL, 200 µg/mL).
Fig. 14A shows the analysis results of high performance liquid fluorescence chromatography (HPLC) of GFP, GFP-cpp28e and GFP-TAT.
Fig. 14B shows the analytical ultracentrifuge Optima XL-100 (AUC) analysis results of GFP-cpp28, GFP and GFP-TAT.
Fig. 15A shows the results of preparative molecular sieve purification of GFP-TAT, GFP-cpp28, GFP-cpp28e, GFP-cpp28g, GFP-cpp28h, GFP-cpp28j.
Fig. 15B shows the results of SDS-PAGE analysis of various protein fractions (multimeric fractions and monomeric fractions) obtained by the preparative molecular sieve purification method.
Fig. 15C shows the results of quantitative analysis of mCherry fluorescence intensity of 293β5 cells using a laser confocal high-content imaging analysis system, wherein the cells were transfected with EHIPS-U2MG plasmid, and cultured in the media containing the indicated protein fractions (multimeric fractions and monomeric fractions) at the indicated concentrations.
Fig. 16A shows the main structural elements contained in the Reporter plasmid constructed in Example 6.
Figs. 16B to 16C show the results of imaging (Fig. 16B) and fluorescence intensity analysis (Fig. 16C) of the 293β5 cells transfected with the indicated plasmids or plasmid combinations using a laser confocal high-content imaging analysis system.
Fig. 17A shows the observation results of 293β5 cells using a confocal high-content imaging analysis system, wherein the cells were transfected with the indicated plasmids or plasmid combinations and treated with the media with or without various recombinant proteins at the indicated concentrations for 12 h.
Fig. 17B shows the results of measuring intracellular EGFP protein content by Native PAGE (native gel electrophoresis), wherein the cells were transfected with the indicated plasmids or plasmid combinations and treated with the media with or without various recombinant proteins at the indicated concentrations for 12 h.
Fig. 17C shows the detection results of intracellular EGFP protein content by Western Blot, wherein the cells were transfected with the indicated plasmids or plasmid combinations and treated with the media with or without various recombinant proteins at the indicated concentrations for 12h.
Fig. 17D shows the results of sequencing analysis of the PCR amplification products obtained in Example 6, wherein Cas9: plasmid expressing Cas9 protein; Vector con: control vector (empty plasmid); aCRISPR-pl: plasmid expressing anti-CRISPR protein; aCRISPR-pro: anti-CRISPR protein; aCRISPR-cpp28-pro: anti-CRISPR-cpp28ori protein.
Fig. 18 shows the results of SDS-PAGE analysis (Fig. 18A ) and the results of Western blot analysis (Fig. 18B ) of the antibodies or recombinant proteins purified in Example 7.
Fig. 19 shows the results of immunofluorescence detection of the cells (HepG2-N10, HepG2-C3A, Hela, MNT-1 and A375) treated with the media containing 100 µg/ml of the indicated antibodies or recombinant proteins for 6 h.
Fig. 20 shows the results of Western blot detection of TYRP-1 antigen in lysates of MNT-1 cells, wherein the MNT-1 cells were treated with 2A7-cpp28ori, TA99 or TA99-cpp28ori at the indicated concentrations for 6 h.
Fig. 21A shows the results of Western blot detection of HBcAg, TRIM21 and antibody levels in lysates of HepG2-N10 cells, wherein the HepG2-N10 cells were treated with 100 µg/ml of 2A7-cpp28ori, 2A7, 16D5-cpp28ori, 16D5, TA99 -cpp28ori or TA99 antibody for 6h.
Fig. 21B shows the detection results of HBeAg antigen and HBV DNA levels in the culture supernatants of the HepG2-N10 cells, wherein the HepG2-N10 cells were treated with 100 µg/ml of 2A7-cpp28ori, 2A7, 16D5-cpp28ori, 16D5, TA99-cpp28ori or TA99 antibody for 6h.
Fig. 22 shows the results of observing mRuby3 fluorescence in 293β5 cells using a laser confocal high-content imaging analysis system, wherein the cells were transfected with plasmid pTTS-mRuby3-ZF motif using ZF-cpp28ori or PEI transfection reagent.

### Sequence Information

The information on the sequences involved in the present application is summarized in Table 1.

**Table 1: Sequence Information**

| SEQ ID NO: | Sequence Description |
|---|---|
| 1 | Amino acid sequence of GFP |
| 2 | Amino acid sequence of DNA-binding domain of Gal4 |
| 3 | Amino acid sequence of Anti-GFP VHH2 |
| 4 | Amino acid sequence of VP16 activation domain |
| 5 | Amino acid sequence of Anti-GFP VHH6 |
| 6 | Amino acid sequence of RFP |
| 7 | Amino acid sequence of luciferase |
| 8 | UAS sequence |
| 9 | Amino acid sequence of iRFP670 |
| 10 | Amino acid sequence of cpp28 |
| 11 | Amino acid sequence of cpp28a |
| 12 | Amino acid sequence of cpp28b |
| 13 | Amino acid sequence of cpp28c |
| 14 | Amino acid sequence of cpp28d |
| 15 | Amino acid sequence of cpp28e |
| 16 | Amino acid sequence of cpp28f |
| 17 | Amino acid sequence of cpp28g |
| 18 | Amino acid sequence of cpp28h |
| 19 | Amino acid sequence of cpp28i |
| 20 | Amino acid sequence of cpp28j |
| 21 | Amino acid sequence of cpp28k |
| 22 | Amino acid sequence of cpp28L |
| 23 | Amino acid sequence of cpp28-Os |
| 24 | Amino acid sequence of cpp28aC |
| 25 | Amino acid sequence of cpp28bC |
| 26 | Amino acid sequence of cpp28cC |
| 27 | Amino acid sequence of cpp28dC |
| 28 | Amino acid sequence of cpp28dREC |
| 29 | Amino acid sequence of cpp28oNT-1 |
| 30 | Amino acid sequence of cpp28oNT-2 |
| 31 | Amino acid sequence of cpp28oNT-3 |
| 32 | Amino acid sequence of cpp28aCQ1 |
| 33 | Amino acid sequence of cpp28aCQ2 |
| 34 | Amino acid sequence of cpp28aCQ3 |
| 35 | Amino acid sequence of cpp28aCNT |
| 36 | Amino acid sequence of cpp28bCNT |
| 37 | Amino acid sequence of cpp28cCNT |
| 38 | Amino acid sequence of cpp28dCNT |
| 39 | Amino acid sequence of flexible peptide linker |
| 40 | Amino acid sequence of GFP-CPP28 |
| 41 | Amino acid sequence of GFP-TAT |
| 42 | Amino acid sequence of cell-penetrating peptide TAT |
| 43 | Amino acid sequence of mRuby3 protein |
| 44 | Amino acid sequence of anti-CRISPR protein |
| 45 | Amino acid sequence of anti-CRISPR-cpp28ori protein |
| 46 | Amino acid sequence of Cas9 protein |
| 47 | Amino acid sequence of light chain variable region of antibody 2A7 |
| 48 | Amino acid sequence of heavy chain variable region of antibody 2A7 |
| 49 | Amino acid sequence of light chain variable region of antibody 16D5 |
| 50 | Amino acid sequence of heavy chain variable region of antibody 16D5 |
| 51 | Amino acid sequence of light chain variable region of antibody TA99 |
| 52 | Amino acid sequence of heavy chain variable region of antibody TA99 |
| 53 | Amino acid sequence of ZF protein |
| 54 | Binding sequence of ZF protein |

1. Amino acid sequence of GFP (SEQ ID NO: 1) 
MVSKGEELFTGVVPILVELDGDVNGHKFSVSGEGEGDATYGKLTLKFICTTGKL
PVPWPTLVTTLTYGVQCFSRYPDHMKQHDFFKSAMPEGYVQERTIFFKDDGNYKTR
AEVKFEGDTLVNRIELKGIDFKEDGNILGHKLEYNYNSHNVYIMADKQKNGIKVNFK
IRHNIEDGSVQLADHYQQNTPIGDGPVLLPDNHYLSTQSALSKDPNEKRDHMVLLEF
VTAAGITLGMDELYK
2. Amino acid sequence of DNA-binding domain of Gal4 (SEQ ID NO: 2) 
MKLLSSIEQACDICRLKKLKCSKEKPKCAKCLKNNWECRYSPKTKRSPLTRAHLTE
VESRLERLEQLFLLIFPREDLDMILKMDSLQDIKALLTGLFVQDNVNKDAVTDRLASVET
DMPLTLRQHRISATSSSEESSNKGQRQLTVS
3. Amino acid sequence of Anti-GFP VHH2 (SEQ ID NO: 3) 
MADVQLQESGGGSVQAGEALRLSCVGSGYTSINPYMAWFRQAPGKEREGVAAISS
GGQYTYYADSVKGRFTISRDNAKNTMYLQMPSLKPDDSAKYYCAADFRRGGSWNVDP
LRYDYQHWGQGTQVTVSS
4. Amino acid sequence of VP16 activation domain (SEQ ID NO: 4) 
APPTDVSLGDELHLDGEDVAMAHADALDDFDLDMLGDGDSPGPGFTPHDSAPYG
ALDMADFEFEQMFTDALGIDEYGG
5. Amino acid sequence of Anti-GFP VHH6 (SEQ ID NO: 5) 
MADVQLQESGGGSVQTGGSLRLSCAVSPYIGSRISLGWFRQAPGKVREGVALINSR
DGSTYYADTVKGRFTISQGDANTVYLQMNSLKPEDTAIYYCAARWGQITDIQALAVAS
FPDWGQGTQVTVSS
6. Amino acid sequence of RFP (SEQ ID NO: 6) 
MVSKGEEDNMAIIKEFMRFKVHMEGSVNGHEFEIEGEGEGRPYEGTQTAKLKV
TKGGPLPFAWDILSPQFMYGSKAYVKHPADIPDYLKLSFPEGFKWERVMNFEDGGV
VTVTQDSSLQDGEFIYKVKLRGTNFPSDGPVMQKKTMGWEASSERMYPEDGALKG
EIKQRLKLKDGGHYDAEVKTTYKAKKPVQLPGAYNVNIKLDITSHNEDYTIVEQYER
AEGRHSTGGMDGCTS
7. Amino acid sequence of luciferase (SEQ ID NO: 7) 
MGVKVLFALICIAVAEAKPTENNEDFNIVAVASNFATTDLDADRGKLPGKKLPLEV
LKELEANARKAGCTRGCLICLSHIKCTPKMKKFIPGRCHTYEGDKESAQGGIGEAIVDIP
EIPGFKDLEPLEQFIAQVDLCVDCTTGCLKGLANVQCSDLLKKWLPQRCATFASKIQGQ
VDKIKGAGGD
8. UAS sequence (SEQ ID NO: 8) 
AGCTTGCATGCCTGCAGGTCGGAGTACTGTCCTCCGAGCGGAGTACTGTCCTCC
GAGCGGAGTACTGTCCTCCGAGCGGAGTACTGTCCTCCGAGCGGAGTACTGTCCTCC
GAGCGGAGACTCTAGCGAGCGCCGGAGTATAAATAGAGGCGCTTCGTCTACGGAGC
GACAATTCAATTCAAACAAGCAAAGTGAACACGTCGCTAAGCGAAAGCTAAGCAAA
TAAACAAGCGCAGCTGAACAAGCTAAACAATCTGCAGTAAAGTGCAAGTTAAAGTG
AATCAATTAAAAGTAACCAGCAACCAAGTAAATCAACTGCAACTACTGAAATCTGC
CAAGAAGTAATTATTGAATACAA
9. Amino acid sequence of iRFP670 (SEQ ID NO: 9) 
MARKVDLTSCDREPIHIPGSIQPCGCLLACDAQAVRITRITENAGAFFGRETPRVGEL
LADYFGETEAHALRNALAQSSDPKRPALIFGWRDGLTGRTFDISLHRHDGTSIIEFEPAA
AEQADNPLRLTRQIIARTKELKSLEEMAARVPRYLQAMLGYHRVMLYRFADDGSGMVI
GEAKRSDLESFLGQHFPASLVPQQARLLYLKNAIRVVSDSRGISSRIVPEHDASGAALDL
SFAHLRSISPCHLEFLRNMGVSASMSLSIIIDGTLWGLIICHHYEPRAVPMAQRVAAEMF
ADFLSLHFTAAHHQR
10. Amino acid sequence of cpp28 (SEQ ID NO: 10)
   RRRGRSPRRRTPSPRRRRSQSPRRRRSQSRESQC
11. Amino acid sequence of cpp28a (SEQ ID NO: 11)
   RRRGRSPRRRTPSPRRRRSQSPRRRRSQSRES
12. Amino acid sequence of cpp28b (SEQ ID NO: 12)
   RRRGRSPRRRTPSPRRRRSQSPRRRRSQS
13. Amino acid sequence of cpp28c (SEQ ID NO: 13)
   RRRGRSPRRRTPSPRRRRSQSPRRRRS
14. Amino acid sequence of cpp28d (SEQ ID NO: 14)
   RRRGRSPRRRTPSPRRRRSQS
15. Amino acid sequence of cpp28e (SEQ ID NO: 15)
   RRRDRGRSPRRRTPSPRRRRSQSPRRRRSQSRESQC
16. Amino acid sequence of cpp28f (SEQ ID NO: 16)
   RRRGRSPRRRTPSPRRRRSKSPRRRRSKSRESQC
17. Amino acid sequence of cpp28g (SEQ ID NO: 17)
   RQRGRAPRRRTPSPRRRRSQSPRRRRSQSRASQC
18. Amino acid sequence of cpp28h (SEQ ID NO: 18)
   RCRGRRGRSPRRRTPSPRRRRSQSPRRRRSKSRESQC
19. Amino acid sequence of cpp28i (SEQ ID NO: 19)
   RRRGGARASRSPRRRTPSPRRRRSQSPRRRRSQSRSANC
20. The amino acid sequence of cpp28j (SEQ ID NO: 20)
   RRRGNPRAPRSPRRRTPSPRRRRSQSPRRRRSQSPAPSNC
21. Amino acid sequence of cpp28k (SEQ ID NO: 21)
   RRRGGSRATRSPRRRTPSPRRRRSQSPRRRRSQSPASSNC
22. The amino acid sequence of cpp28L (SEQ ID NO: 22)
   RRRPASRRSTPSPRRRRSQSPRRRRSPSPRPASNC
23. The amino acid sequence of cpp28-Os (SEQ ID NO: 23)
   RRRGRSPRRRTPSPRRRRSQSPRRRRSQSRESQS
24. Amino acid sequence of cpp28aC (SEQ ID NO: 24)
   RRRGRSPRRRTPSPRRRRSQSPRRRRSQSREC
25. Amino acid sequence of cpp28bC (SEQ ID NO: 25)
   RRRGRSPRRRTPSPRRRRSQSPRRRRSQC
26. The amino acid sequence of cpp28cC (SEQ ID NO: 26)
   RRRGRSPRRRTPSPRRRRSQSPRRRRC
27. Amino acid sequence of cpp28dC (SEQ ID NO: 27)
   RRRGRSPRRRTPSPRRRRSQC
28. Amino acid sequence of cpp28dREC (SEQ ID NO: 28)
   RRRGRSPRRRTPSPRRRRSQRREC
29. Amino acid sequence of cpp28oNT-1 (SEQ ID NO: 29)
   SPRRRTPSPRRRRSQSPRRRRSQSRESQC
30. The amino acid sequence of cpp28oNT-2 (SEQ ID NO: 30)
   SPRRRRSQSPRRRRSQSRESQC
31. Amino acid sequence of cpp28oNT-3 (SEQ ID NO: 31)
   SPRRRRSQSRESQC
32. Amino acid sequence of cpp28aCQ1 (SEQ ID NO: 32)
   RRRGRQPRRRTPSPRRRRSQSPRRRRSQSREC
33. Amino acid sequence of cpp28aCQ2 (SEQ ID NO: 33)
   RRRGRQPRRRTPQPRRRRSQSPRRRRSQSREC
34. The amino acid sequence of cpp28aCQ3 (SEQ ID NO: 34)
   RRRGRQPRRRTPQPRRRRSQQPRRRRSQSREC
35. Amino acid sequence of cpp28aCNT (SEQ ID NO: 35)
   SPRRRTPSPRRRRSQSPRRRRSQSREC
36. Amino acid sequence of cpp28bCNT (SEQ ID NO: 36)
   SPRRRTPSPRRRRSQSPRRRRSQC
37. Amino acid sequence of cpp28cCNT (SEQ ID NO: 37)
   SPRRRTPSPRRRRSQSPRRRRC
38. Amino acid sequence of cpp28dCNT (SEQ ID NO: 38)
   SPRRRTPSPRRRRSQC
39. Amino acid sequence of flexible peptide linker (SEQ ID NO: 39)
   GGGGSGGGGSGGGGS
40. Amino acid sequence of GFP-cpp28 (SEQ ID NO: 40) 
MVSKGEELFTGVVPILVELDGDVNGHKFSVSGEGEGDATYGKLTLKFICTTGKLPV
PWPTLVTTLTYGVQCFSRYPDHMKQHDFFKSAMPEGYVQERTIFFKDDGNYKTRAEVK
FEGDTLVNRIELKGIDFKEDGNILGHKLEYNYNSHNVYIMADKQKNGIKVNFKIRHNIED
GSVQLADHYQQNTPIGDGPVLLPDNHYLSTQSALSKDPNEKRDHMVLLEFVTAAGITLG
MDELYKGGGGSGGGGSGGGGSRRRGRSPRRRTPSPRRRRSQSPRRRRSQSRESQC
41. Amino acid sequence of GFP-TAT (SEQ ID NO: 41) 
MVSKGEELFTGVVPILVELDGDVNGHKFSVSGEGEGDATYGKLTLKFICTTGKLPV
PWPTLVTTLTYGVQCFSRYPDHMKQHDFFKSAMPEGYVQERTIFFKDDGNYKTRAEVK
FEGDTLVNRIELKGIDFKEDGNILGHKLEYNYNSHNVYIMADKQKNGIKVNFKIRHNIED
GSVQLADHYQQNTPIGDGPVLLPDNHYLSTQSALSKDPNEKRDHMVLLEFVTAAGITLG
MDELYKGGGGSGGGGSGGGGSGRKKRRQRRRPPQ
42. Amino acid sequence of cell-penetrating peptide TAT (SEQ ID NO: 42)
   GRKKRRQRRRPPQ
43. Amino acid sequence of mRuby3 protein (SEQ ID NO: 43) 
MVSKGEELIKENMRMKVVMEGSVNGHQFKCTGEGEGRPYEGVQTMRIKVIEG
GPLPFAFDILATSFMYGSRTFIKYPADIPDFFKQSFPEGFTWERVTRYEDGGVVTVTQ
DTSLEDGELVYNVKVRGVNFPSNGPVMQKKTKGWEPNTEMMYPADGGLRGYTDIA
LKVDGGGHLHCNFVTTYRSKKTVGNIKMPGVHAVDHRLERIEESDNETYVVQREVA
VAKYSNLGGGMDELYK
44. Amino acid sequence of anti-CRISPR protein (SEQ ID NO: 44)
45. Amino acid sequence of anti-CRISPR-cpp28ori protein (SEQ ID NO: 45) 
MNINDLIREIKNKDYTVKLSGTDSNSITQLIIRVNNDGNEYVISESENESIVEKFIS
AFKNGWNQEYEDEEEFYNDMQTITLKSELNGGGGSGGGGSGGGGSRRRGRSPRRRT
PSPRRRRSQSPRRRRSQSRESQC
46. Amino acid sequence of cas9 protein (SEQ ID NO: 46)
47. Amino acid sequence of light chain variable region of antibody 2A7 (SEQ ID NO: 47) 
DIQMTQTSSSLSASPGDRVTISCRASQGINNYLNWYKQKTDGTFKLLIYYTSYLHSG
VPSRFSGRGSGTDYSLTISNLEPEDVATYYCQQYGKLPWTFGGGTKLEIK
48. Amino acid sequence of heavy chain variable region of antibody 2A7 (SEQ ID NO: 48) 
QVQLQQPGAELVKPGASVKLSCKASGYTFTRYWMHWVMQRPGQDLEWIGEINPI
NGRTNYNEKFRRKATLTVDKSSSTVYIQFSSLTSEDSAVYFCTREGYRNDYYYAMDFW
GRGTSVTVSS
49. Amino acid sequence of light chain variable region of antibody 16D5 (SEQ ID NO: 49) 
DIVLTQSPGSLAVFLGQRATISCRASQSVSGSIYSYMHWYQQKPGQPPKLLIKFASN
LESGVPARFSGGGSGTDFTLNIHPVEEEDAATYYCQHSWEIPYTFGGGTKLEIK
50. Amino acid sequence of heavy chain variable region of antibody 16D5 (SEQ ID NO: 50) 
EVQLQQSGAEVVKPGASVKLSCTASGFKIEDTYIHWVKQRPEQGLEWIGRIDPANG
NSRYDPNFQGKATIIADTSSYTIYLQLSSLTSEDTAVYYCSSPLSLLRLGGFAYWGQGTLI
TVSA
51. Amino acid sequence of light chain variable region of antibody TA99 (SEQ ID NO: 51) 
AIQMSQSPASLSASVGETVTITCRASGNIYNYLAWYQQKQGKSPHLLVYDAKTLAD
GVPSRFSGSGSGTQYSLKISSLQTEDSGNYYCQHFWSLPFTFGSGTKLEIK
52. Amino acid sequence of heavy chain variable region of antibody TA99 (SEQ ID NO: 52) 
VQLQQSGAELVRPGALVKLSCKTSGFNIKDYFLHWVRQRPDQGLEWIGWINPDNG
NTVYDPKFQGTASLTADTSSNTVYLQLSGLTSEDTAVYFCTRRDYTYEKAALDYWGQG
ASVIVSS
53. Amino acid sequence of ZF protein (SEQ ID NO: 53) 
VSRPGERPFQCRICMRNFSDKTKLRVHTRTHTGEKPFQCRICMRNFSVRHNLTRHL
RTHTGEKPFQCRICMRNFSQSTSLQRHLKTHLRGS
54. Binding sequence of ZF protein (SEQ ID NO: 54)
   tGTAGATGGAg

### Specific Models for Carrying Out the present invention

The present invention will now be described with reference to the following examples, which are intended to illustrate, but not limit, the present invention.

Unless otherwise specified, the molecular biology experimental methods and immunoassays used in the present invention are performed by basically referring to the methods described in J. Sambrook et al., Molecular Cloning: A Laboratory Manual, 2nd Edition, Cold Spring Harbor Laboratory Press, 1989, and F. M. Ausubel et al., Refined Molecular Biology Laboratory Manual, 3rd Edition, John Wiley & Sons, Inc., 1995; and the restriction enzymes were used according to the conditions recommended by the product manufacturers. Those skilled in the art appreciate that the examples describe the present invention by way of example and are not intended to limit the scope sought to be protected by the present invention.

### Example 1: Establishment of reporter system for evaluating efficiency of cell-penetrating peptide in delivery of proteins into cells

In this example, in order to simply and efficiently evaluate the efficiency of cell-penetrating peptides in mediating the entry of target proteins into cells, the inventors used green fluorescent protein (GFP; SEQ ID NO: 1) as a model protein to establish a reporter system capable of quantifying the efficiency of cell-penetrating peptide in the delivery of proteins into cells.

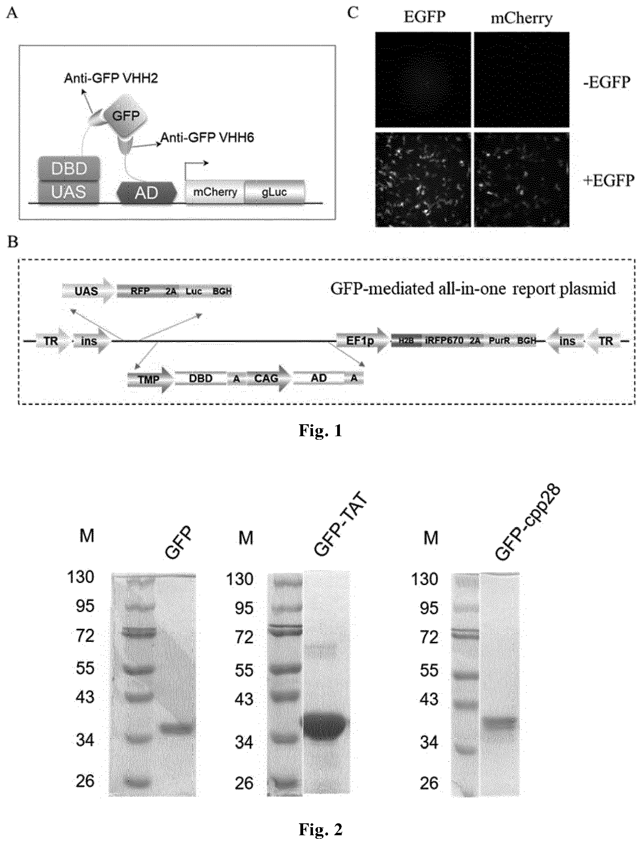

The working principle of the reporting system constructed in this example was shown in Fig. 1A. Briefly, GFP was used as a cargo molecule to be fused with the cell-penetrating peptide to be tested. Furthermore, the DNA binding domain (DBD; SEQ ID NO: 2) of transcriptional regulator Gal4 was fused with Anti-GFP VHH2 (first anti-GFP single domain antibody; SEQ ID NO: 3) to form a first fusion protein DBD-VHH2; VP16 activation domain (AD; SEQ ID NO: 4) was fused with Anti-GFP VHH6 (secondary anti-GFP single domain antibody; SEQ ID NO: 5) to form a second fusion protein AD-VHH6; and the mCherry gene encoding red fluorescent protein (RFP; SEQ ID NO: 6) and the Luc gene encoding luciferase (Luc; SEQ ID NO: 7) were placed under the control of the UAS sequence (upstream activating sequence; SEQ ID NO: 8) recognized by Gal4. Thus, when the cell-penetrating peptide to be tested could deliver the cargo molecule GFP into the cell, the DBD-VHH2 and AD-VHH6 expressed in the cell would specifically recognize and bind to the GFP molecule entered into the cell, and made the DBD and AD close to each other to form a complex that could recognize the UAS sequence and initiated transcription; the complex would initiate the transcription and expression of the downstream genes (mCherry gene and Luc gene) regulated by the UAS sequence (Cell. 2013 Aug 15; 154(4): 928 -939). Therefore, by detecting the intensity of the fluorescence emitted by the mCherry protein and/or the activity level of luciferase Luc, the number of GFP molecules entered into the cell could be determined, thereby determine the efficiency of the cell-penetrating peptide fused with GFP in the delivery of the protein into the cell. In this reporter system, the transcriptional activation of the mCherry gene and Luc gene depended on the presence of intracellular GFP, thus, the intracellular GFP level could be determined by detecting the intensity of mCherry fluorescence and/or the activity of luciferase Luc. The stronger the fluorescence emitted by the mCherry protein or the higher the activity of the luciferase Luc, the more GFP molecules in the cell and the higher the delivery efficiency of the cell-penetrating peptide to be tested.

For simple and efficient evaluation, the inventors constructed the elements involved in the above system in the same plasmid and named it as EHIPS-U2MG. The main structural elements contained in the plasmid EHIPS-U2MG were shown in Fig. 1B, wherein UAS: upstream activation sequence recognized by Gal4; RFP: nucleotide sequence encoding red fluorescent protein; 2A: ligation nucleotide sequence; Luc: nucleotide sequence encoding luciferase; BGH: transcription termination sequence; TMP/CAG/EF1p: promoter sequence; DBD: nucleotide sequence encoding DNA binding domain; AD: nucleotide sequence encoding VP16 activation domain; VHH2/VHH6: nucleotide sequence encoding anti-GFP single domain antibody VHH2/VHH6; H2B: nucleotide sequence encoding nuclear localization signal; iRFP670: nucleotide sequence encoding far-red fluorescent protein 670 (SEQ ID NO: 9); PurR: puromycin resistance gene; ins: insulation sequence; TR: transposition sequence.

The plasmid EHIPS-U2MG carried piggyBac transposon system and puromycin resistance gene (PurR). After the plasmid was transfected into 293T cells, puromycin could be used for resistance screening to obtain an engineered cell line that stably integrated the above structural elements in the genome. The pcDNA3.1-GFP plasmid (the plasmid pcDNA3.1 carrying the nucleotide sequence encoding GFP) or the negative control plasmid was transfected into the engineered cell line, and observed with a fluorescence microscope. The results showed that in the cells transfected with the pcDNA3.1-GFP plasmid, the green fluorescence emitted by GFP and the red fluorescence emitted by mCherry could be observed; in the cells of the negative control group, no green fluorescence signal and red fluorescence signal were detected (Fig. 1C).

Therefore, using the reporter system and the engineered cell line constructed in this example, the presence and amount of intracellular GFP protein could be determined by detecting the red fluorescence signal of mCherry and intensity thereof.

### Example 2: Design of cell-penetrating peptide cpp28 and preparation of recombinant protein containing cpp28 and GFP

In this example, the inventors designed a new cell-penetrating peptide, and prepared a recombinant protein containing the cell-penetrating peptide and green fluorescent protein.

The cell-penetrating peptide cpp28 designed in this example had the following amino acid sequence: RRRGRSPRRRTPSPRRRRSQSPRRRRSQSRESQC (SEQ ID NO: 10). Based on this, a recombinant protein GFP-CPP28 (SEQ ID NO: 40) was designed, wherein the C-terminal of GFP was ligated to the cell-penetrating peptide cpp28 through a flexible linker GGGGS-GGGGS-GGGGS (SEQ ID NO: 39). The nucleotide sequence encoding GFP-CPP28 was cloned into the pTO-T7 prokaryotic expression vector (which carried the nucleotide sequence encoding 6×His tag; Luo Wenxin et al., Chinese Journal of Biological Engineering, 2000, 16:53-57), thereby obtaining an expression plasmid pTO-T7-GFP-CPP28.

In addition, a recombinant protein GFP-TAT (SEQ ID NO: 41) was designed, in which the C-terminal of GFP was ligated to the cell-penetrating peptide TAT (HIV-derived cell-penetrating peptide; SEQ ID NO: 42) through a flexible linker (SEQ ID NO: 39). The nucleotide sequence encoding GFP-TAT was cloned into the pTO-T7 prokaryotic expression vector to obtain the expression plasmid pTO-T7-GFP-TAT.

The above-constructed expression plasmids were transformed into *Escherichia coli* Shuffle T7 strain respectively, and the recombinant proteins were expressed. Subsequently, the recombinant proteins expressed in *E. coli* were purified by nickel column affinity chromatography to obtain the purified target proteins GFP-CPP28 and GFP-TAT. In addition, the GFP protein was also expressed and purified by a similar method. The purified target proteins (GFP, GFP-CPP28 and GFP-TAT) were detected by SDS-PAGE, and the results were shown in Fig. 2. The results showed that the obtained purified proteins all had a purity of greater than 95%.

### Example 3: Evaluation of efficiency of cpp28 in delivery of proteins into cells

In this example, using the reporter system constructed in Example 1, the efficiency of the cell-penetrating peptide cpp28 in delivering green fluorescent protein into cells was evaluated.

293β5 cells were inoculated at a density of 20,000 cells per well in 96-well plates. After 12 h, the EHIPS-U2MG plasmid constructed in Example 1 was transfected into cells with Lipofectamine^{™} 2000 (Thermo Fisher, 11668019) transfection reagent. 12 h after transfection, the medium was removed, and media containing different recombinant proteins (DMEM, Gibco+10% Gibco FBS) were added, and the concentration of the recombinant proteins (GFP, GFP-CPP28 or GFP-TAT) was 60 µg/mL. After culturing for 36 hours, the mCherry fluorescence of the cells was photographed and analyzed by a laser confocal high-content imaging analysis system. In addition, negative and positive controls were also set as follows: in the negative control group, the cells were transfected with EHIPS-U2MG plasmid, but no recombinant protein was added to the medium; in the positive control group, the cells were simultaneously transfected with EHIPS-U2MG and pcDNA3.1-GFP plasmid, and no recombinant protein was added to the medium.

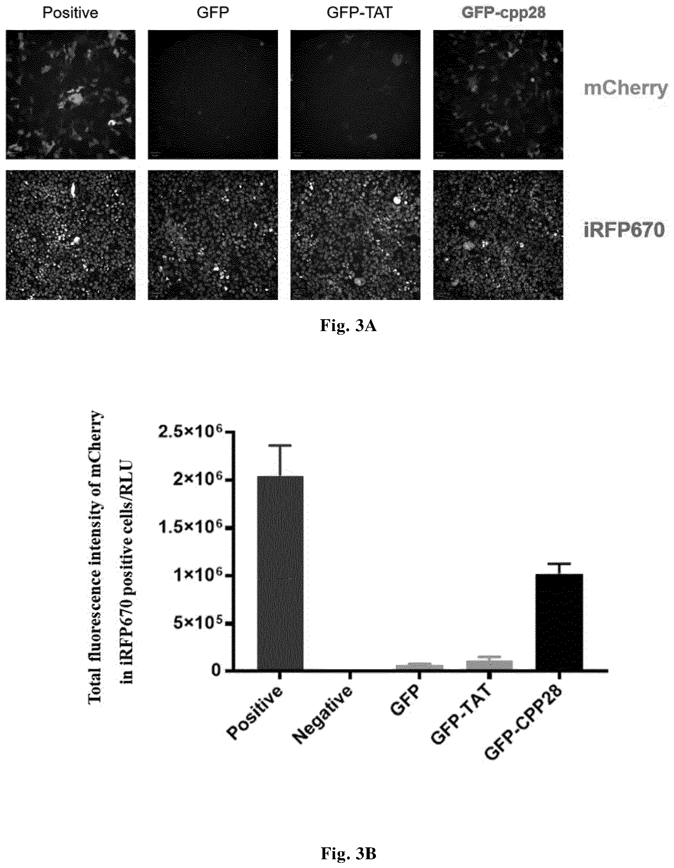

The experimental results were shown in Figs. 3A to 3B. The results showed: (1) in each group of cells, the fluorescence emitted by iRFP670 could be observed, which indicated that the EHIPS-U2MG plasmid had been successfully transfected into the cells; (2) in the negative control group, the fluorescence of mCherry was basically undetectable (Fig. 3B); and, in the positive control group, the strongest mCherry fluorescence was detected (Figs. 3A to 3B); (3) GFP alone basically could not enter into the cells across membrane, and could not induce the cells to express mCherry (the fluorescence of mCherry basically could not be detected in the cells of this group); (4) both cpp28 and TAT could deliver GFP into the cells, and then the expression of downstream mCherry gene was activated through the fluorescent reporter system, so that the cells emit mCherry fluorescence; (5) the efficiency of cpp28 in delivering GFP through transmembrane delivery into the cells was significantly higher than that of TAT.

Furthermore, the concentration gradient experiments were also performed on cpp28 to evaluate its efficiency of penetrating membrane. Briefly, 293β5 cells were inoculated at a density of 20,000 cells per well in 96-well plates. After 12 h, the EHIPS-U2MG plasmid constructed in Example 1 was transfected into the cells with Lipofectamine^{™} 2000 (Thermo Fisher, 11668019) transfection reagent. 12 h after the transfection, the medium was removed, and the medium containing GFP-CPP28 (DMEM, Gibco+10% Gibco FBS) was added, wherein the concentration of GFP-CPP28 was 30, 10, 5, 2.5 µg/mL. After culturing for 36 hours, the mCherry fluorescence of the cells was photographed and analyzed by a laser confocal high-content imaging analysis system.

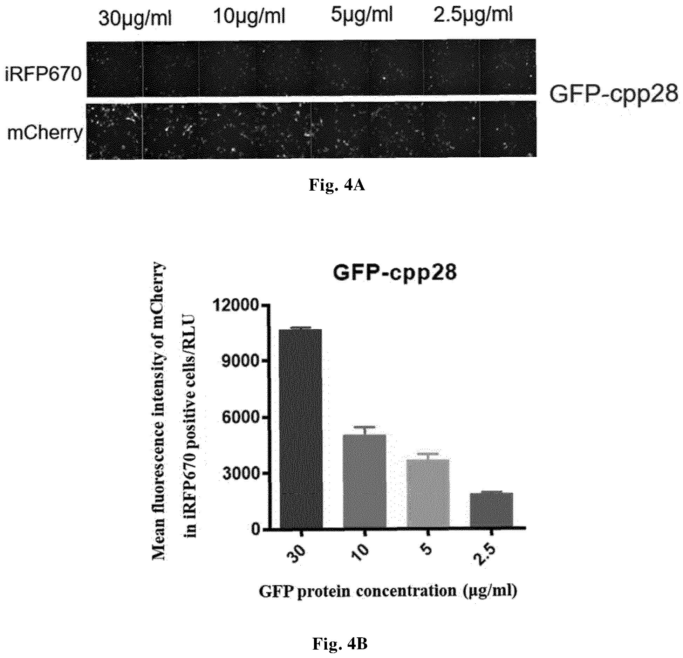

The experimental results were shown in Figs. 4A to 4B. The results showed that cpp28 could effectively deliver GFP protein into the cells, and the expression of downstream mCherry gene was activated through the fluorescent reporter system, so that the cells emit mCherry fluorescence; and, with the increase of GFP-cpp28 protein concentration, the expression amount of mCherry gradually increased, and the fluorescence intensity of the mCherry fluorescence gradually increased.

Further, the efficiency of cpp28 in delivering GFP molecules into cells was assessed in multiple cell types. Briefly, cells of the indicated type (MDCK, VERO or Hacat) were inoculated at a density of 20,000 cells per well in 96-well plates. After 12 h, the EHIPS-U2MG plasmid constructed in Example 1 was transfected into the cells with Lipofectamine^{™} 2000 (Thermo Fisher, 11668019) transfection reagent. 12 h after the transfection, the medium was removed, and medium (DMEM, Gibco+10% Gibco FBS) containing different recombinant protein was added, and the concentration of the recombinant protein (GFP, GFP-CPP28 or GFP-TAT) was 60 µg/mL. After culturing for 36 hours, the mCherry fluorescence of the cells was photographed and analyzed by a laser confocal high-content imaging analysis system; and the enzymatic activity of the gLuc protein secreted into the cell supernatant was measured by a Pierce^{™} Gaussia Luciferase luciferase detection system.

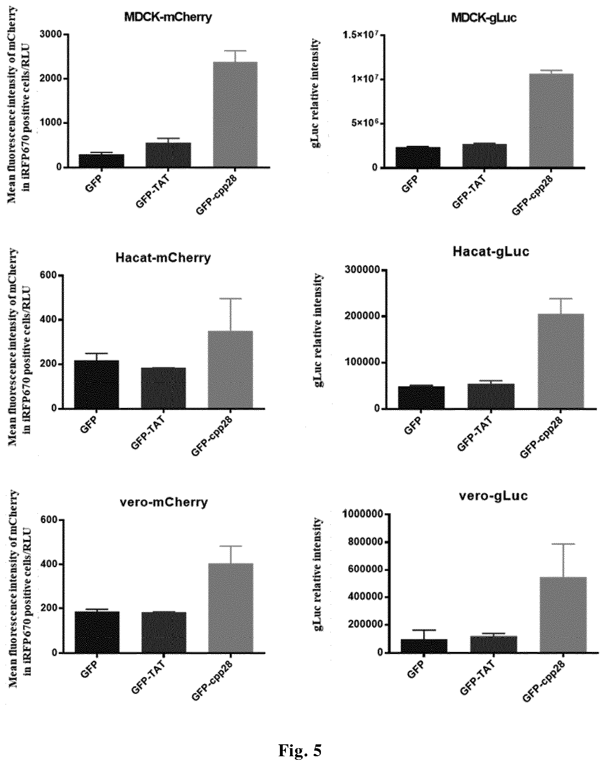

The experimental results were shown in Fig. 5. The results showed that for various cell types (MDCK, VERO and Hacat cells), the cells cultured in medium containing GFP-CPP28 had a significantly higher mCherry fluorescence intensity and a significantly higher luciferase activity. This indicated that cpp28 was able to deliver GFP molecules into various types of cells (MDCK, VERO and Hacat cells), and its delivery efficiency was significantly higher than that of TAT.

### Example 4: Dynamic process and pathway of cpp28 in delivery of protein into cells

In this example, the inventors studied the dynamic process and pathway of cpp28 in delivery of protein into cells.

In order to study the dynamic process of cpp28 in delivery of protein into cells, the inventors constructed a Hela cell line (named Hela-p63mRb3), which was able to express mRuby3 protein (SEQ ID NO: 43; emitting red fluorescence) on the cytoplasmic membrane, and able to express iRFP670 protein (emitting far-red fluorescence) in the nucleus. Through the image acquisition and analysis of the cell line with a laser confocal high-content imaging analysis system, the intracellular green fluorescence, red fluorescence and far-red fluorescence could be detected, localized and quantified, so that the intracellular GFP protein, cytoplasmic membranes and nuclei could be directly observed and analyzed and calculated.

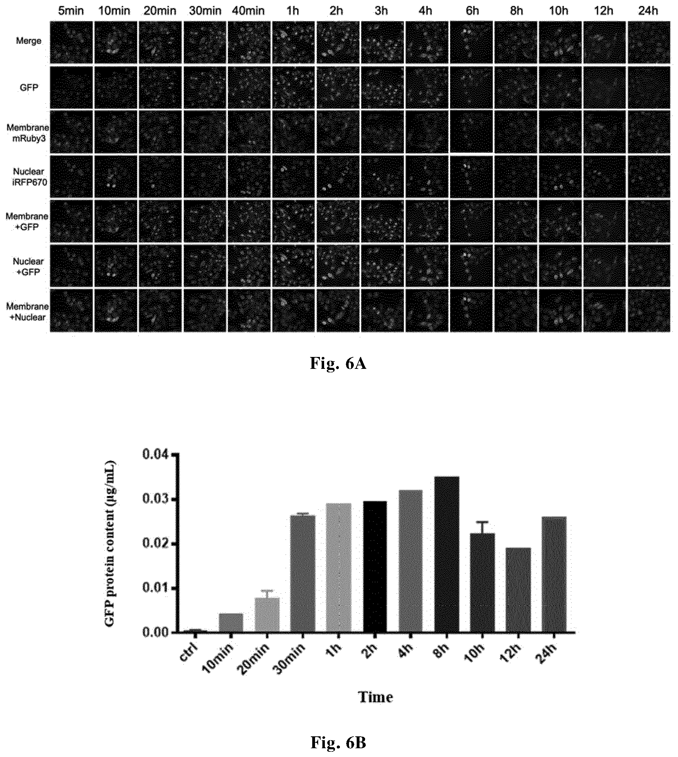

Briefly, Hela-p63mRb3 cells were inoculated at a density of 10,000 cells per well in PerkinElmer 24-well black plates. After the cells adhered, the medium was removed, and a medium (DMEM, Gibco+10% Gibco FBS) containing 60 µg/mL of GFP-cpp28 was added. After 5 minutes, 10 minutes, 20 minutes, 30 minutes, 40 minutes, 1 hour, 2 hours, 3 hours, 4 hours, 6 hours, 8 hours, 10 hours, 12 hours, and 24 hours, the cells were washed with 50 µM heparin (to remove the GFP protein non-specifically adsorbed on the cell membrane), then the cells were fixed with 3.7% paraformaldehyde, and then the cells were photographed with a laser confocal microscope to observe green fluorescence, red fluorescence and far-red fluorescence, to determine the intracellular localization of the GFP protein. The experimental results were shown in Fig. 6A.

In addition, another aliquot of cells were taken and underwent the same treatment in parallel, after being washed with 50 µM heparin, the cells were lysed with DDM (n-Dodecyl β-D-maltoside (sigma) 4 mg/mL, 20 mM Hepes, 1 mM EGTA, 100 mM NaCl, 5 mM MgCl₂), and then the double antibody sandwich method was used to detect the content of GFP protein in the cells. Briefly, the ELISA plates coated with 400 ng/well of GFP-binding protein GBP4 and the HRP-labeled anti-GFP antibody were used to detect GFP protein in serially diluted cell lysates (100 µL), and the contents of GFP protein in the cell lysates were calculated according to a pre-drawn standard curve. The experimental results were shown in Fig. 6B.

The results in Fig. 6A showed that the GFP protein could be detected in the cells after contacting GFP-cpp28 with the cells for a few minutes, and the green fluorescence intensity in the cells reached a peak (plateau phase) within 1-4 hours. Over time, the amount of GFP protein in the cells decreased. In addition, from the co-localization results of green fluorescence and red fluorescence, it could be seen that within a period of time after the GFP protein entered the cells, most of the GFP protein co-localized with the cytoplasmic membrane labeled with mRuby3, which indicated that within a period of time after cpp28 carried GFP and entered the cells, it was mainly located in the endosome. Over time, the co-localization signal of GFP and mRuby3 decreased, which indicated that the GFP protein escaped from the endosome and entered the cytoplasm. The detection results of Fig. 6B were consistent with the observation results of confocal microscopy (Fig. 6A).

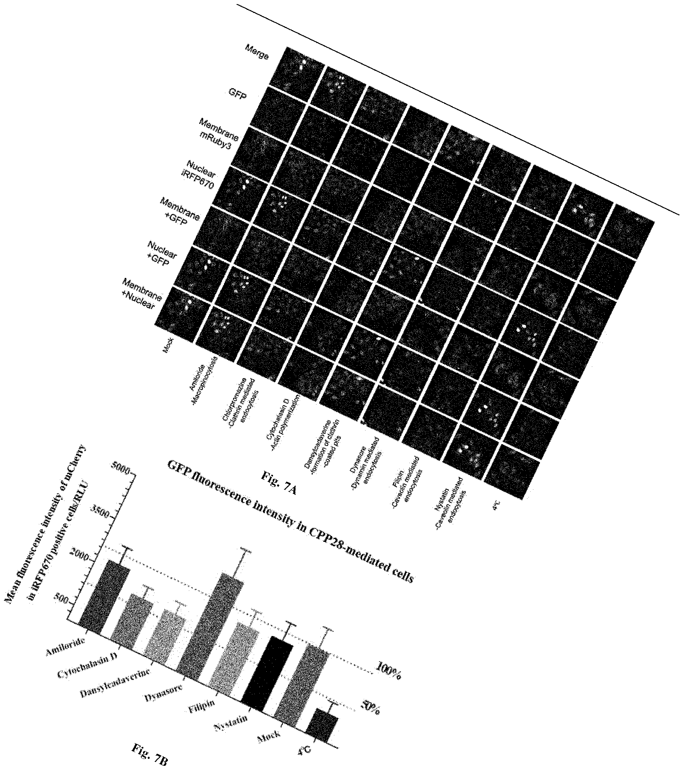

In addition, the above-mentioned Hela-p63mRb3 cells were used to analyze the pathway of cpp28 delivering GFP protein into cells. Briefly, Hela-p63mRb3 cells were inoculated at a density of 10,000 cells per well in PerkinElmer 24-well black plates. After the cells adhered, various endocytic pathway inhibitors (Amiloride, Chlorpromazine, filipin, Cytochalasin D, Dansylcadaverine, Dynasore, nystatin) or 4°C alone were used to treat the cells for 2h. Subsequently, 30 µg/ml GFP-cpp28 protein was added, and treatment was carried out for 1 h. After treatment, the cells were washed three times with heparin, then fixed with 3.7% paraformaldehyde, and photographed and observed with a laser confocal microscope. The experimental results were shown in Figs. 7A to 7B.

From the results of the fluorescence photography (Fig. 7A) and the analysis of the intracellular GFP fluorescence intensity (Fig. 7B), it could be seen that low temperature (4°C) treatment significantly inhibited the entry of GFP-cpp28 into the cells, indicating that it was an energy-dependent process that cpp28 carried GFP protein and entered the cells. In addition, two inhibitors, cytochalasin D and dansylcadaverine, also inhibited the transmembrane delivery of cpp28 to a certain extent, but the inhibitory effect was weaker than that of the low temperature treatment. This suggested that the transmembrane delivery of cpp28 involved clathrin-mediated endocytosis.

### Example 5: Evaluation of variants of cpp28 and their efficiency in delivery of protein into cells

In this example, the inventors designed multiple variants of cpp28 (also referred to as cpp28ori in the present application) (the cpp28 variants were listed in Table 2), and evaluated the efficiency of these variants in delivering proteins into cells.

**Table 2: Amino acid sequences of cpp28 and variants thereof**

| SEQ ID NO | Name | Sequence information |
|---|---|---|
| 10 | cpp28 | RRRGRSPRRRTPSPRRRRSQSPRRRRSQSRESQC |
| 11 | cpp28a | RRRGRSPRRRTPSPRRRRSQSPRRRRSQSRES |
| 12 | cpp28b | RRRGRSPRRRTPSPRRRRSQSPRRRRSQS |
| 13 | cpp28c | RRRGRSPRRRTPSPRRRRSQSPRRRRS |
| 14 | cpp28d | RRRGRSPRRRTPSPRRRRSQS |
| 15 | cpp28e | RRRDRGRSPRRRTPSPRRRRSQSPRRRRSQSRESQC |
| 16 | cpp28f | RRRGRSPRRRTPSPRRRRSKSPRRRRSKSRESQC |
| 17 | cpp28g | RQRGRAPRRRTPSPRRRRSQSPRRRRSQSRASQC |
| 18 | cpp28h | RCRGRRGRSPRRRTPSPRRRRSQSPRRRRSKSRESQC |
| 19 | cpp28i | RRRGGARASRSPRRRTPSPRRRRSQSPRRRRSQSRSANC |
| 20 | cpp28j | RRRGNPRAPRSPRRRTPSPRRRRSQSPRRRRSQSPAPSNC |
| 21 | cpp28k | RRRGGSRATRSPRRRTPSPRRRRSQSPRRRRSQSPASSNC |
| 22 | cpp28L | RRRPASRRSTPSPRRRRSQSPRRRRSPSPRPASNC |
| 23 | cpp28oS | RRRGRSPRRRTPSPRRRRSQSPRRRRSQSRESQS |
| 24 | cpp28aC | RRRGRSPRRRTPSPRRRRSQSPRRRRSQSREC |
| 25 | cpp28bC | RRRGRSPRRRTPSPRRRRSQSPRRRRSQC |
| 26 | cpp28cC | RRRGRSPRRRTPSPRRRRSQSPRRRRC |
| 27 | cpp28dC | RRRGRSPRRRTPSPRRRRSQC |
| 28 | cpp28dREC | RRRGRSPRRRTPSPRRRRSQRREC |
| 29 | cpp28oNT-1 | SPRRRTPSPRRRRSQSPRRRRSQSRESQC |
| 30 | cpp28oNT-2 | SPRRRRSQSPRRRRSQSRESQC |
| 31 | cpp28oNT-3 | SPRRRRSQSRESQC |
| 32 | cpp28aCQ1 | RRRGRQPRRRTPSPRRRRSQSPRRRRSQSREC |
| 33 | cpp28aCQ2 | RRRGRQPRRRTPQPRRRRSQSPRRRRSQSREC |
| 34 | cpp28aCQ3 | RRRGRQPRRRTPQPRRRRSQQPRRRRSQSREC |
| 35 | cpp28aCNT | SPRRRTPSPRRRRSQSPRRRRSQSREC |
| 36 | cpp28bCNT | SPRRRTPSPRRRRSQSPRRRRSQC |
| 37 | cpp28cCNT | SPRRRTPSPRRRRSQSPRRRRC |
| 38 | cpp28dCNT | SPRRRTPSPRRRRSQC |

Among the cpp28 variants listed in Table 2, cpp28a, cpp28b, cpp28c, and cpp28d were all truncates of cpp28, and their C-terminals were truncated by 2, 5, 7, and 13 amino acid residues, respectively, as compared with cpp28; cpp28e, cpp28h, cpp28i, cpp28j and cpp28k had an addition of 2-5 amino acid residues at the N-terminal as compared to cpp28; cpp28oNT-1, cpp28oNT-2 and cpp28oNT-3 were all N-terminal truncates of cpp28, and their N-terminals were truncated by 5, 12, and 20 amino acids, respectively, as compared with cpp28; cpp28aC, cpp28bC, cpp28cC, cpp28dC were variants of cpp28a, cpp28b, cpp28c and cpp28d, respectively, in which the C-terminal serine S was mutated to cysteine C; cpp28dREC was a variant obtained on the basis of cpp28dC by adding before the C-terminal cysteine with two positively charged arginine Rs and one glutamic acid E, which tended to produce a helical structure; cpp28oS was a variant obtained by mutating the C-terminal cysteine C of cpp28 to serine S; cpp28aCQ1, cpp28aCQ2 and cpp28aCQ3 were the variants obtained by mutating 1, 2 or 3 serine Ss that tended to produce disordered structure in the SPRRR structure of cpp28aC to glutamine Qs that tend to produce helical structure, respectively; cpp28aCNT, cpp28bCNT, cpp28cCNT and cpp28dCNT were variants obtained by truncation of 5 amino acids at the N-terminal of cpp28aC, cpp28bC, cpp28cC, and cpp28dC, respectively.

According to the method described in Example 2, a variety of recombinant proteins (GFP-cpp28a, GFP-cpp28b, GFP-cpp28c, GFP-cpp28d, GFP-cpp28e, GFP-cpp28f, GFP-cpp28g, GFP-cpp28h, GFP-cpp28i, GFP-cpp28j, GFP-cpp28k, GFP-cpp28L, GFP-cpp28oS, GFP-cpp28aC, GFP-cpp28bC, GFP-cpp28cC, GFP-cpp28dC, GFP-cpp28dREC, GFP-cpp28oNT-1, GFP-cpp28oNT-2, GFP-cpp28oNT-3, GFP-cpp28aCQ1, GFP-cpp28aCQ2, GFP-cpp28aCQ3, GFP-cpp28aCNT, GFP-cpp28bCNT, GFP-cpp28cCNT and GFP-cpp28dCNT) were constructed, expressed and purified based on the cpp28 variants designed in Table 2, wherein each of the cpp28 variants was linked to the C-terminal of GFP via a linker (SEQ ID NO: 39).

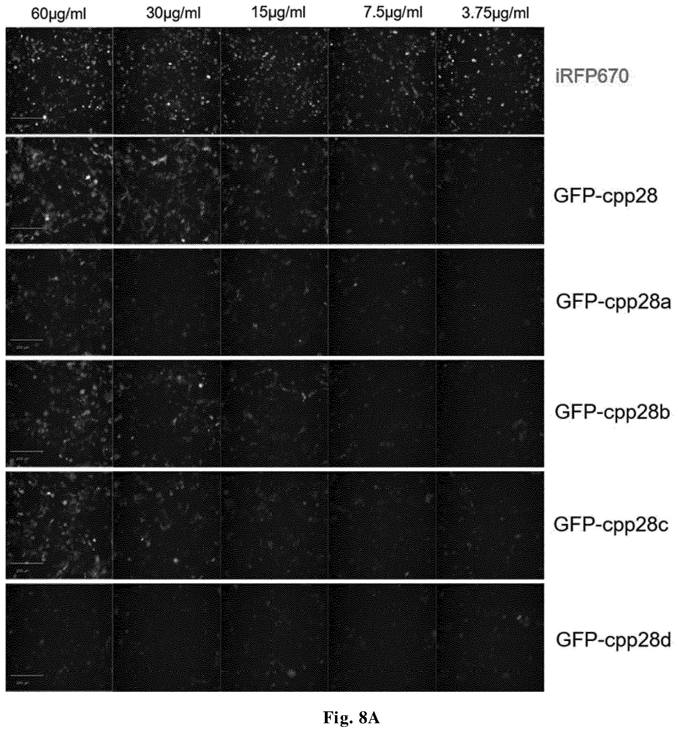

Then, using the reporter system constructed in Example 1, as described in Example 3, the efficiencies of the cpp28 variants (cpp28a, cpp28b, cpp28c, cpp28d, cpp28e, cpp28f, cpp28g, cpp28h, cpp28i, cpp28j, cpp28k and cpp28L) in delivering green fluorescent protein into cells were evaluated. The experimental results were shown in Figs. 8A to 8B.

The results in Fig. 8A showed that cpp28a, cpp28b and cpp28c all had the ability to deliver GFP into the cells, but their delivery efficiencies were lower than that of cpp28. In addition, cpp28d essentially lost its ability to deliver GFP into the cells. These results suggested that cpp28 could tolerate a C-terminal deletion to a certain extent; wherein, the cpp28 truncates with a truncation of 1-7 amino acid residues at the C-terminal were still able to deliver GFP molecules through transmembrane delivery.

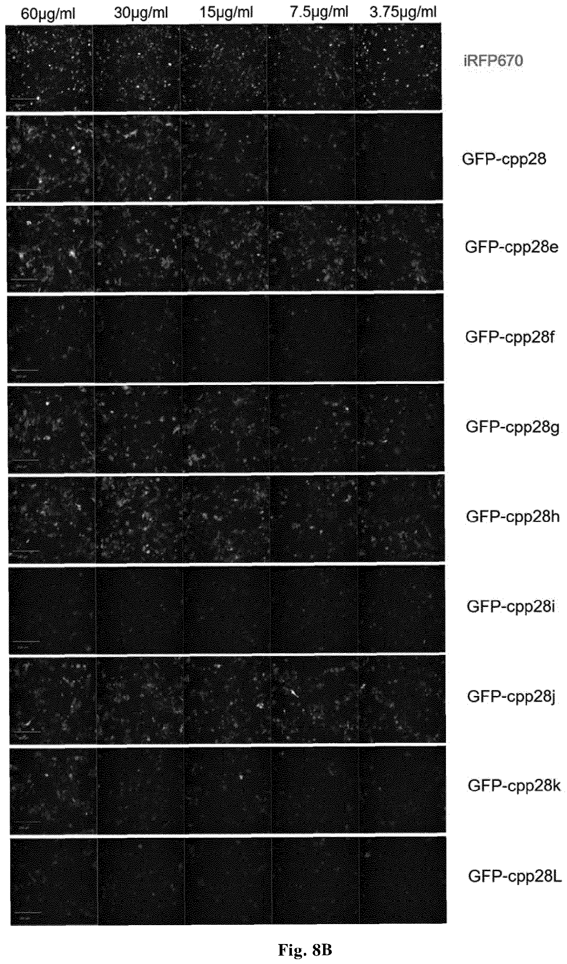

The results in Fig. 8B showed that cpp28, cpp28e, cpp28g, cpp28h, cpp28j, and cpp28k all had the ability to deliver GFP into the cells, wherein the delivery efficiencies of cpp28e, cpp28g, cpp28h and cpp28j were even better than that of cpp28, and cpp28f, cpp28i and cpp28L essentially lost their ability to deliver GFP into the cells.

In addition, the results of Figs. 8A to 8B also showed that cpp28a, cpp28b, cpp28c, cpp28e, cpp28g, cpp28h, cpp28j, cpp28k were all able to deliver GFP into the cells in a dose-dependent manner.

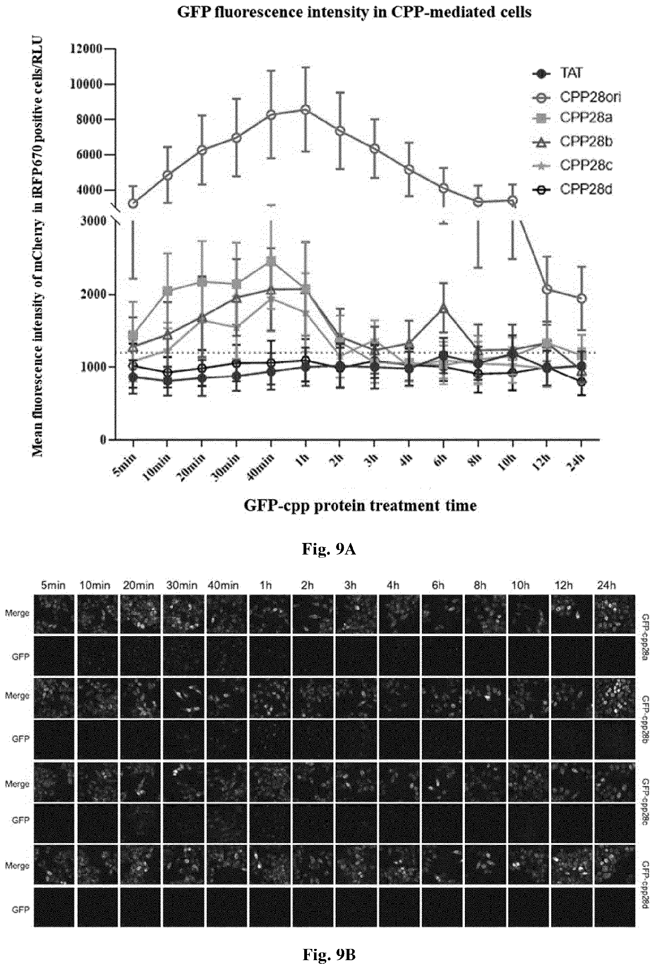

Further, the process of cpp28, cpp28a, cpp28b, cpp28c, cpp28d and TAT delivering GFP protein into cells was also observed by the method described in Example 4 using the constructed Hela-p63mRb3 cell model. The experimental results were shown in Figs. 9A to 9B.

The results of Figs. 9A to 9B showed that cpp28a, cpp28b and cpp28c all had the ability to deliver GFP into the cells, wherein although their delivery efficiencies were lower than that of cpp28, but all significantly higher than those of cpp28d and TAT. This result was basically consistent with that of Fig. 8A.

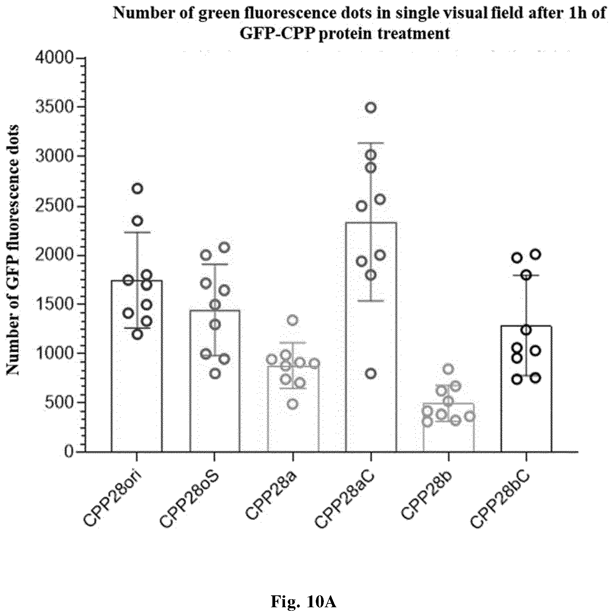

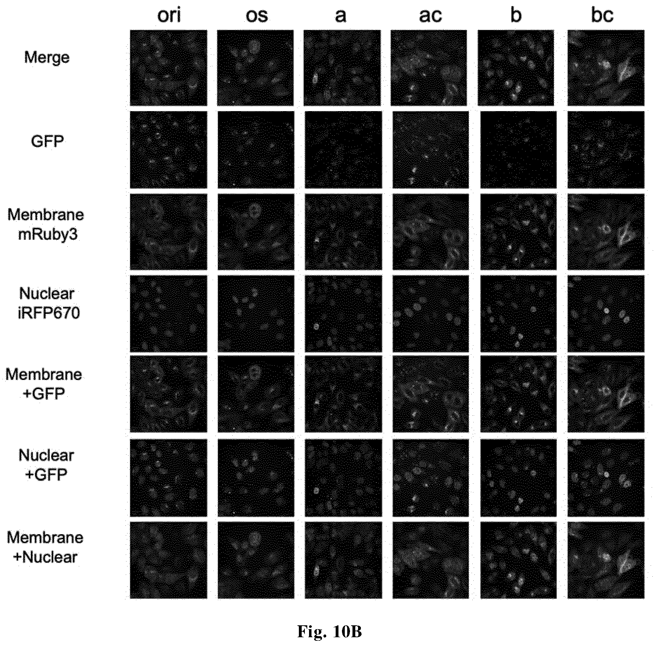

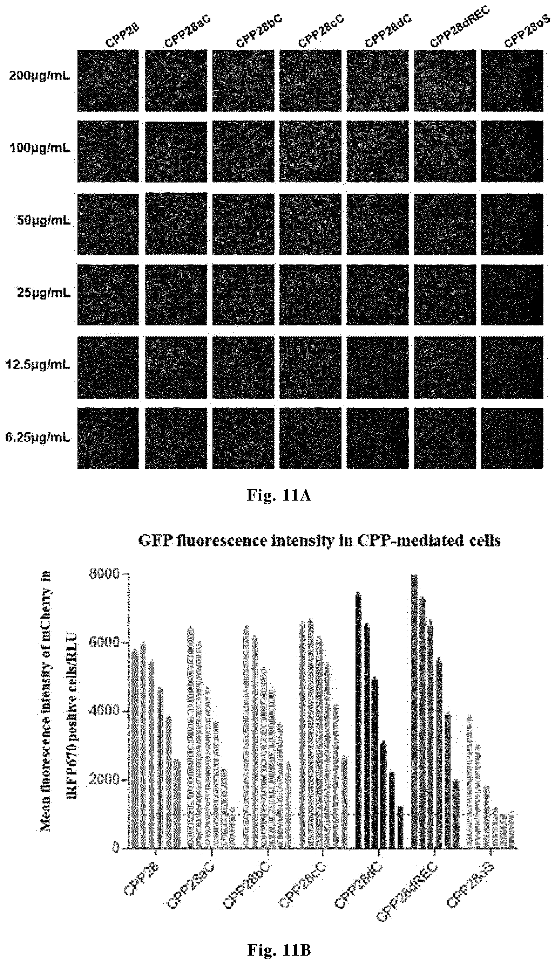

In addition, by the method described in Example 4, the process of cpp28, cpp28-oS, cpp28a, cpp28aC, cpp28b, cpp28bC, cpp28cC, cpp28dC and cpp28dREC delivering GFP protein into cells was observed by using the constructed Hela-p63mRb3 cell model, and their efficiencies and dose-dependent properties in delivery of protein into cells were evaluated. The experimental results were shown in Figs. 10A to 10B and Figs. 11A to 11B.

The results of Figs. 10A to 10B and Figs. 11A to 11B showed that after mutating the C-terminal cysteine of cpp28 to serine, cpp28-oS could still deliver GFP into the cells, but the delivery efficiency decreased in certain extent; further, after the C-terminal serine of cpp28a, cpp28b, cpp28c and cpp28d was mutated to cysteine, the activity of cpp28aC, cpp28bC, cpp28cC and cpp28dC to deliver GFP protein into the cells was significantly enhanced. These results suggested that the C-terminal cysteine was beneficial for the transmembrane delivery function of cpp28. Moreover, Figs. 11A to 11B showed that the activity of cpp28dREC in delivering GFP protein into the cells was improved in certain extent as compared with cpp28dC, and it still maintained a good transmembrane activity at a low concentration of 12.5 µg/mL.

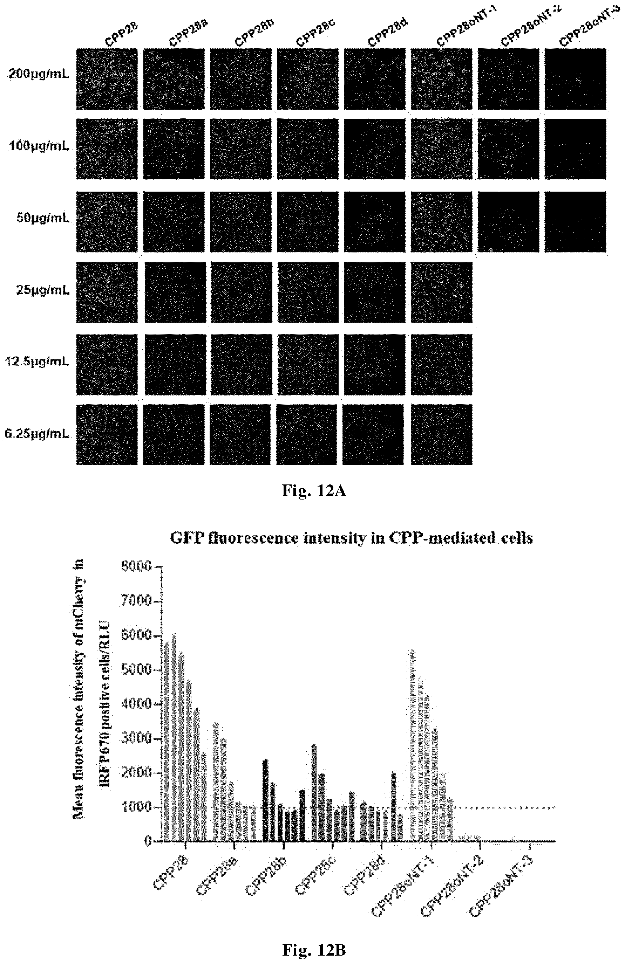

At the same time, by the method described in Example 4, the process of cpp28, cpp28a, cpp28b, cpp28c, cpp28d, cpp28oNT-1, cpp28oNT-2 and cpp28oNT-3 delivering GFP protein into cells was observed by using the constructed Hela-p63mRb3 cell model, and their efficiencies and dose-dependent characteristics in delivery of protein into cells were evaluated. The experimental results were shown in Figs. 12A to 12B.

The results of Figs. 12A to 12B showed that cpp28a, cpp28b, cpp28c and cpp28oNT-1 all had the ability to deliver GFP into the cells with a dose-dependent effect, and the delivery efficiencies of cpp28oNT-1 and cpp28 were basically equivalent. In addition, cpp28d, cpp28oNT-2 and cpp28oNT-3 essentially lost their ability to deliver GFP into the cells. These results suggested that cpp28 could tolerate N-terminal or C-terminal deletion to a certain extent; wherein the cpp28 truncates with a truncation of 1-7 amino acid residues at the C-terminal could still deliver GFP molecules across membrane, and N-terminal deletions of 1-5 amino acids basically did not affect its transmembrane delivery efficiency, while cpp28oNT-2 and cpp28oNT-3 lacking one SPRRR structure or two SPRRR structures basically lost the ability to deliver GFP into the cells.

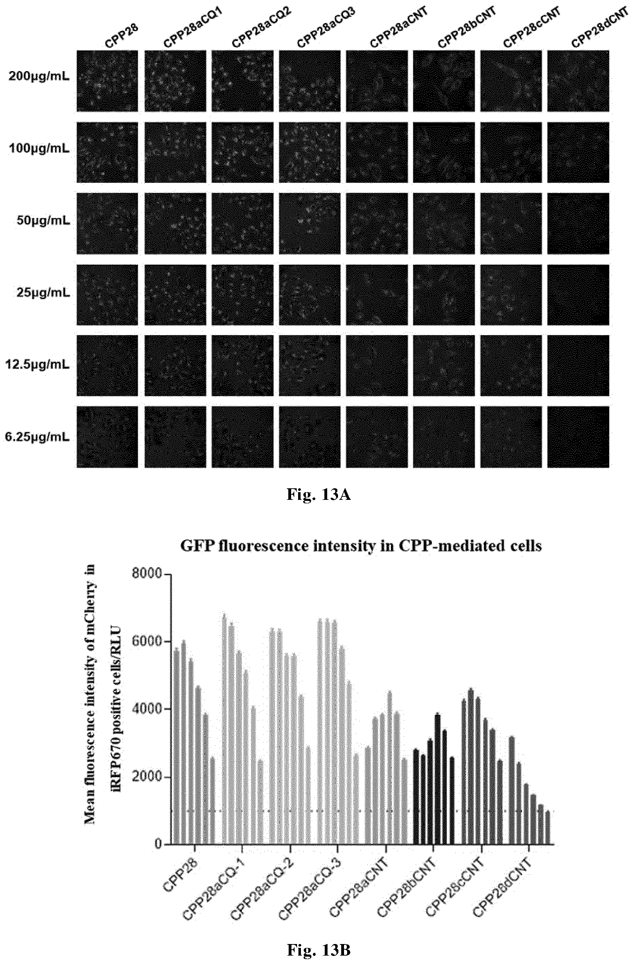

In addition, by the method described in Example 4, the process of cpp28, cpp28aCQ1, cpp28aCQ2, cpp28aCQ3, cpp28aCNT, cpp28bCNT, cpp28cCNT and cpp28dCNT delivering GFP protein into cells was observed by using the constructed Hela-p63mRb3 cell model, and their efficiencies and dose-dependent properties in delivery of protein into cells were evaluated. The experimental results were shown in Figs. 13A to 13B.

The results of Figs. 13A to 13B showed that cpp28aCQ1, cpp28aCQ2, and cpp28aCQ3 all had the ability to deliver GFP into cells, and their delivery efficiencies were comparable to, or even slightly better than that of cpp28. Further, cpp28aCNT, cpp28bCNT, cpp28cCNT and cpp28dCNT with combination of N-terminal truncation and C-terminal truncation also all had the ability to deliver GFP into cells, and the delivery efficiencies of cpp28aCNT, cpp28bCNT, and cpp28cCNT were equivalent to that of cpp28 at low concentration (12.5 µg/mL).

### Example 6: Analysis of recombinant proteins containing GFP and cpp28 or variants thereof

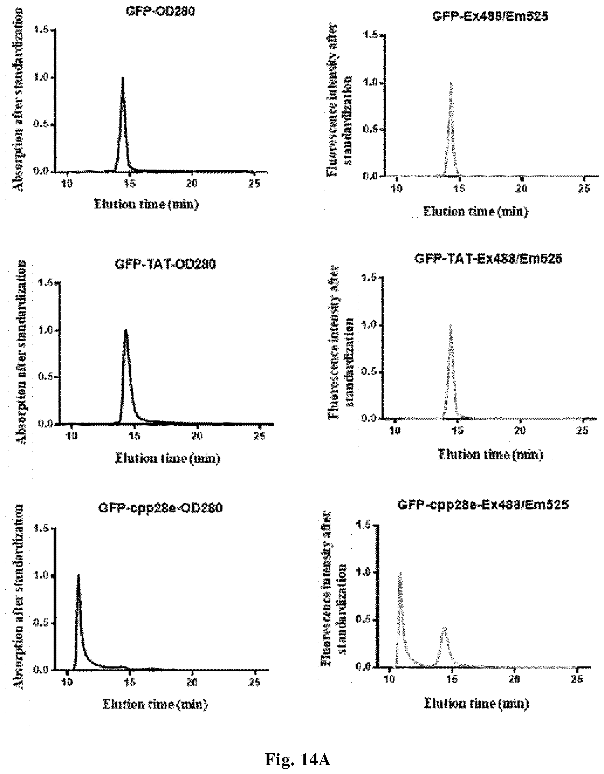

High-performance liquid fluorescence chromatography (HPLC) analysis was performed on the recombinant proteins containing GFP and cpp28 or variants thereof. The results were shown in Fig. 14A. The results showed that the peak time of GFP alone and GFP-TAT protein was about 14 minutes; while taking GFP-cpp28e as an example, the recombinant protein had two peaks with peak times of about 11 minutes and 14 minutes, respectively, and, the fluorescence detection analysis showed that the fluorescence peaks of GFP-cpp28e also appeared at their respective peak times, and the protein peak at about 11 minutes had stronger fluorescence value. This result indicated that GFP-cpp28e generated multimers.

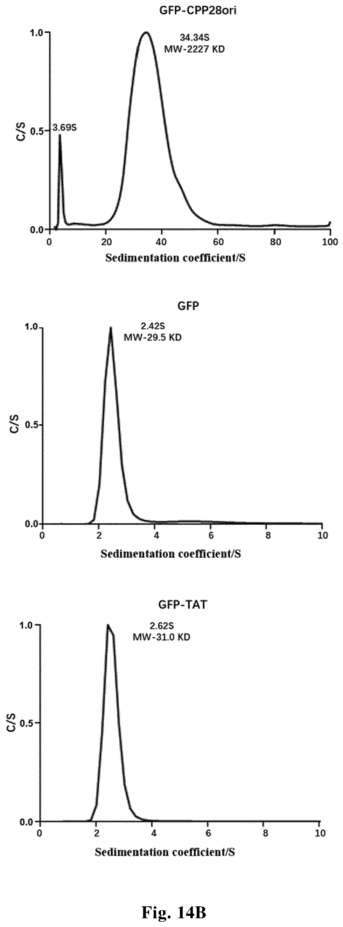

Further, taking GFP-cpp28, GFP and GFP-TAT as examples, these proteins were analyzed by analytical ultracentrifuge Optima XL-100 (AUC) to determine the sedimentation coefficients of these proteins. The results were shown in Fig. 14B. The results showed that the GFP-cpp28 with formation of multimer had a sedimentation coefficient of about 34S, and a relative molecular mass of about 2200kD; while the pure GFP and GFP-TAT proteins had a sedimentation coefficient of about 2.4S and a relative molecular mass of about 30kD. This result indicated that GFP-cpp28 formed a macromolecule of about 70 aggregates relative to pure GFP protein molecule.

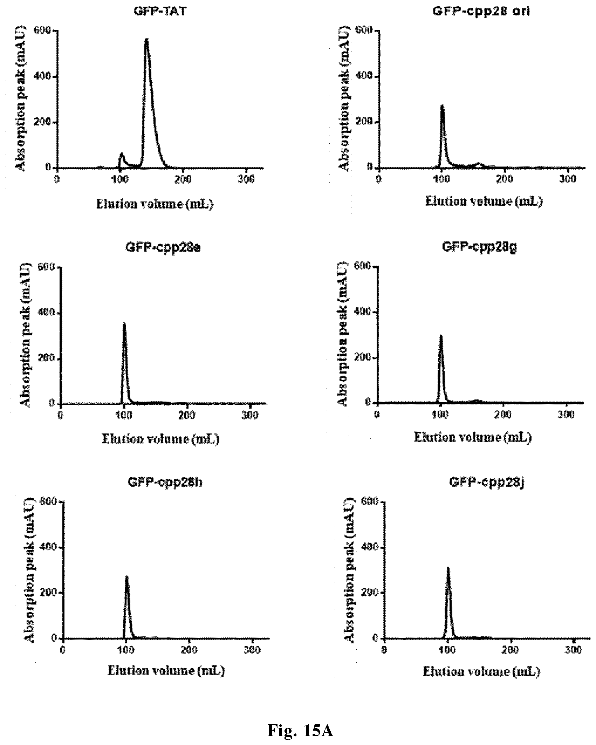

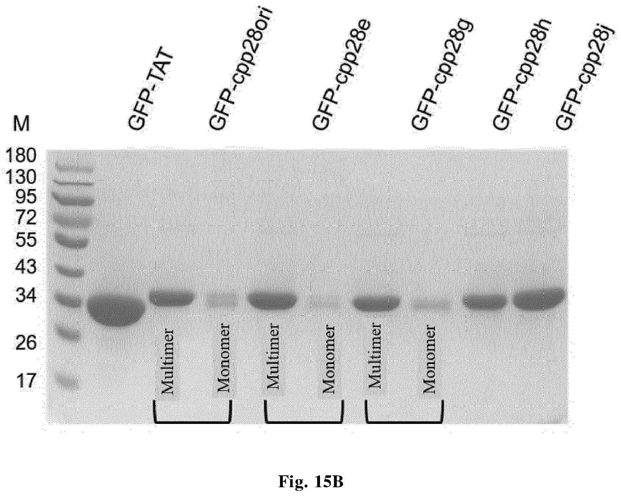

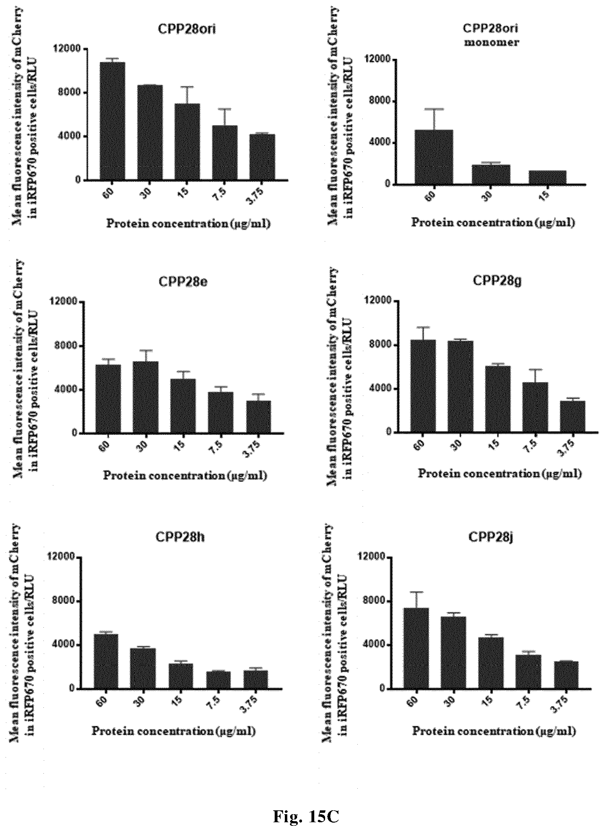

Based on the above conclusions, the inventors used Sephacryl^{™} High Resolution resins HiPrep^{™} Sephacryl HR columns 26/60 (320 ml) preparative molecular sieves to further purify the recombinant proteins (GFP-TAT, GFP-cpp28, GFP-cpp28e, GFP-cpp28g, GFP-cpp28h, GFP-cpp28j) obtained by nickle column affinity chromatography. The results were shown in Fig. 15A. The results showed that for the protein GFP-TAT, it mainly existed in the form of monomer; for the proteins GFP-cpp28, GFP-cpp28e, GFP-cpp28g, GFP-cpp28h and GFP-cpp28j, the target protein could be collected at two different peak times, and the percentage of protein with short peak time (multimer) was much higher than that of the protein with long peak time (monomer). This result was consistent with the results shown in Figs. 14A and 14B. Further, various protein fractions (multimeric fraction and monomeric fraction) collected were analyzed by SDS-PAGE. The results were shown in Fig. 15B. The results showed that after denaturation (i.e., multimers were dissociated into monomers), these proteins were all approximately 30 kd in size. Additionally, as described in Example 3, using the reporter system constructed in Example 1, the various protein fractions (multimeric fraction and monomeric fraction) collected were evaluated for their efficiency in delivering green fluorescent protein into cells. The experimental results were shown in Fig. 15C. The results showed that the cell entry efficiency of the multimeric protein was significantly higher than that of the monomeric protein.

### Example 6: Evaluation of cell entry efficiency of anti-CRISPR-cpp28ori protein

In this example, the inventors verified whether cpp28ori could deliver anti-CRISPR protein (AcrIIA4) into cells and exerted its effect of inhibiting gene editing of Cas9.

The CRISPR-cas9 system is currently a very popular gene editing system, and its high gene editing efficiency is also accompanied by off-target effects that trouble the researchers. A document published in Cell in 2017 (Cell. 2017 Jan 12; 168(1-2): 150-158.e10) reported that the protein AcrIIA4 (also called anti-CRISPR protein in the present application; SEQ ID NO: 44) can inhibit the action of CRISPR protein, and this protein can also exert an inhibitory effect in mammalian cells. In this example, the inventors prepared and purified an anti-CRISPR protein (anti-CRISPR-cpp28ori; SEQ ID NO: 45) fused to the cell-penetrating peptide cpp28 by a prokaryotic expression system, and evaluated its cell entry efficiency.

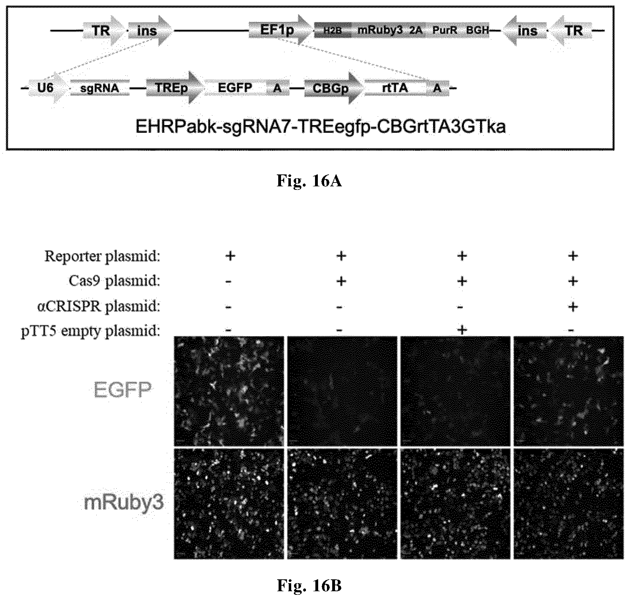

Briefly, the inventors constructed a fluorescent reporter system to verify the transmembrane effect of anti-CRISPR-cpp28ori protein. As shown in Fig. 16A, the fluorescent reporter system used a plasmid (hereinafter referred to as Reporter plasmid) that carried the red fluorescent mRuby3 gene, the sgRNA sequence for EGFP protein and the EGFP gene promoted by TRE promoter, wherein, EF1p/U6/TREp: promoter sequence; H2B: nucleotide sequence encoding nuclear localization signal; mRuby3: nucleotide sequence of mRuby3 gene; 2A: linking nucleotide sequence; BGH: transcription termination sequence; sgRNA: nucleotide sequence encoding sgRNA; EGFP: nucleotide sequence encoding EGFP protein; PurR: puromycin resistance gene; ins: insulation sequence; TR: transposition sequence.

When the Reporter plasmid and the plasmid carrying cas9 gene (addgene ID: 52961) (hereinafter referred to as cas9 plasmid) were co-transfected in the cells, the cells would express Cas9 protein (SEQ ID NO: 46), and the Cas9 protein would edit EGFP gene through the sgRNA sequence for EGFP protein, so that the cells could not express the EGFP protein normally. Thus, the cells would emit no or only very weak green fluorescence.

In addition, the inventors constructed pTT5-anti-CRISPR-cpp28ori plasmid (which was used to transfect eukaryotic cells and express anti-CRISPR-cpp28ori protein in the eukaryotic cells) and plasmids pTO-T7-his-anti-CRISPR and pTO-T7-his-anti-CRISPR-cpp28ori (which were used to transfect prokaryotic cells and express anti-CRISPR and anti-CRISPR-cpp28ori proteins carrying 6*His tag in the prokaryotic cells, wherein the anti-CRISPR protein and cpp28ori were ligated via a flexible linker represented by SEQ ID NO: 39).

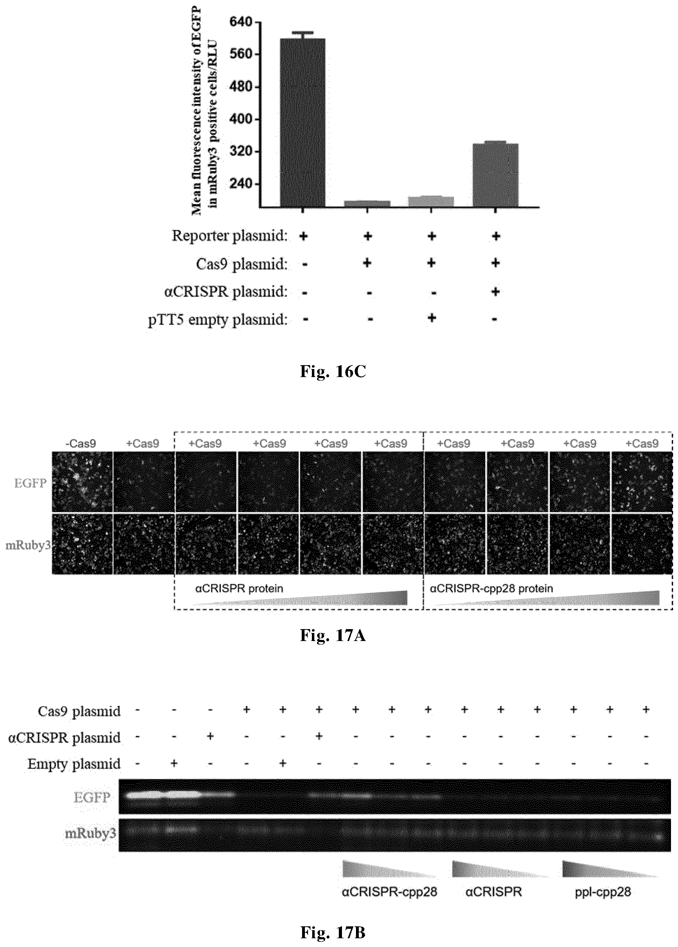

The reporter plasmid, cas9 plasmid and pTT5-anti-CRISPR-cpp28ori plasmid constructed as above were used to verify the function of the fluorescent reporter system in 293β5 cells. Briefly, 293β5 cells were transfected with the indicated plasmids or plasmid combinations. 48h after the transfection, the cells were cultured in DMEM medium containing doxorubicin for 12h, and then the intracellular EGFP fluorescence was photographed and analyzed using a laser confocal high-content imaging analysis system. The experimental results were shown in Figs. 16B to 16C. The results showed that: in the cells transfected with Reporter plasmid alone, obvious EGFP fluorescence expression could be observed; in the cells transfected with Reporter plasmid and cas9 plasmid at the same time, only a very weak green fluorescence was observed; in the cells transfected with Reporter plasmid, cas9 plasmid and pTT5 empty plasmid at the same time, only a very weak green fluorescence was observed; and, in the cells transfected with pTT5-anti-CRISPR-cpp28ori plasmid, Reporter plasmid and cas9 plasmid at the same time, a more obvious green fluorescence was observed. These results indicated that the Cas9 protein could play a gene editing function in the cells and inhibited the expression of EGFP protein; while the anti-CRISPR protein could inhibit the gene editing function of Cas9 protein and restored the expression of EGFP protein. The mean fluorescence intensity analysis results (Fig. 16C) showed that the anti-CRISPR restored about 40% of the EGFP fluorescence. Therefore, the constructed fluorescent reporter system could indicate the function of anti-CRISPR by EGFP fluorescence intensity.

According to the method described in Example 2, the anti-CRISPR and anti-CRISPR-cpp28ori proteins were expressed in *E. coli* and purified by nickel column affinity chromatography. The SDS-PAGE analysis showed that the purified anti-CRISPR protein had a purity of more than 95%, and the purified anti-CRISPR-cpp28ori protein had a purity of more than 90%, which could be used in the next cell experiments.

293β5 cells were simultaneously transfected with the Reporter plasmid and the cas9 plasmid. 12h after transfection, the medium was changed to a medium (DMEM, Gibco+10% Gibco FBS) containing the anti-CRISPR or anti-CRISPR-cpp28ori protein at the indicated concentration (20, 40, 80 or 100 µg/ml), and the cells were cultured continuously at 37°C for 12h. Subsequently, the medium was changed to a normal medium (DMEM, Gibco+10% Gibco FBS), and 48 h after transfection, the medium was changed to a DMEM medium containing doxorubicin. After culturing for another 12 hours, the EGFP fluorescence in the 293β5 cells was photographed and analyzed by a laser confocal high-content imaging analysis system. The experimental results were shown in Fig. 17A. The results showed that the addition of anti-CRISPR-cpp28ori protein to the medium could partially restore the intracellular EGFP fluorescence intensity, and with the increase of protein concentration, the percentage of EGFP fluorescence recovery increased. In contrast, the addition of anti-CRISPR protein to the medium did not restore the intracellular EGFP fluorescence intensity. This result indicated that cpp28ori could deliver anti-CRISPR protein into the cells, so that it could inhibit the function of Cas9 in the cells.

In another experiment, 293β5 cells were transfected simultaneously with the Reporter plasmid and the cas9 plasmid. 12 h after transfection, the medium was changed to a medium (DMEM, Gibco+10% Gibco FBS) containing anti-CRISPR or anti-CRISPR-cpp28ori or ctrl-protein-cpp28ori protein at the indicated concentration (100, 50 or 25 µg/ml), and the cells were continuously cultured at 37°C for 12h. Subsequently, the medium was changed to a normal medium (DMEM, Gibco+10% Gibco FBS), and 48 h after transfection, the medium was changed to a DMEM medium containing doxorubicin, and the culturing was continued for 12 h. In this experiment, negative control and positive control were also set as follows: in the negative control, the medium used did not contain recombinant protein, and the cells were transfected with Reporter plasmid, or Reporter plasmid and cas9 plasmid, or Reporter plasmid and plasmid expressing anti-CRISPR, or Reporter plasmid and empty plasmid, or Reporter plasmid, cas9 plasmid and empty plasmid; in the positive control group, the medium used did not contain recombinant protein, and the cells were transfected with Reporter plasmid, cas9 plasmid and plasmid expressing anti-CRISPR.

After incubation, the cells were lysed with DDM lysate, and the content of intracellular EGFP protein was detected by Native PAGE (native gel electrophoresis) and Western Blot. In the Native PAGE analysis, the mRuby3 fluorescent gene carried on the Reporter plasmid was used as the internal reference standard; in the Western Blot analysis, the gene Tubulin was used as the internal reference standard. The experimental results were shown in Figs. 17B to 17C.

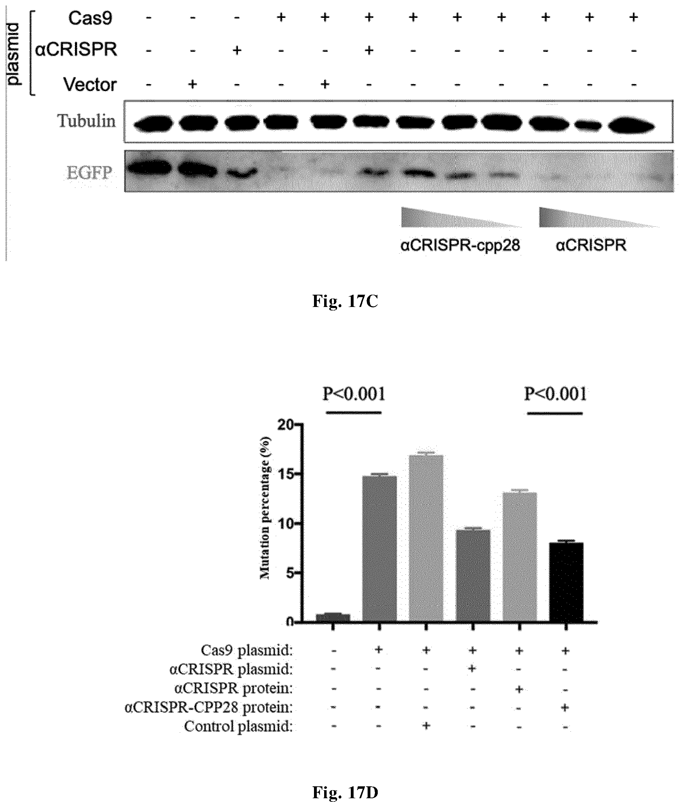

The results of Native PAGE analysis (Fig. 17B) showed that when the anti-CRISPR-cpp28ori plasmid was used for transfection or the anti-CRISPR-cpp28ori protein was added to the cell culture medium, a significant EGFP protein signal (green fluorescence) could be detected in the cells; and, with the increase of anti-CRISPR-cpp28ori protein concentration, the EGFP protein signal (green fluorescence) was enhanced. However, when the anti-CRISPR protein or ctrl-protein-cpp28ori protein was added to the cell culture medium, basically no or very weak EGFP protein signal was detected. The results of Western Blot analysis (Fig. 17C) were consistent with the results of Native PAGE analysis. The results of Figs. 17B to 17C showed that cpp28ori could deliver anti-CRISPR protein into the cells, so that it could inhibit the function of Cas9 in the cells.

In addition, QIAamp DNA blood Mini Kit (QIAGEN) was used to extract cellular genomic DNA from the cells, and PCR amplification was performed; in which, the two primers used were targeted to about 200bp positions of the upstream and downstream of sgRNA binding position of EGFP gene sequence, respectively. The PCR amplification products were recovered, and sequenced and analyzed based on the second-generation sequencing technology. The experimental results were shown in Fig. 17D. The results showed: (1) compared with the original EGFP gene sequence, the gene editing function of Cas9 could induce mutation of the target sequence (EGFP gene sequence), and the mutation ratio was about 15%; (2) the co-transfection of anti-CRISPR-cpp28ori plasmid or the addition of anti-CRISPR-cpp28ori protein in the medium could inhibit the function of Cas9 and reduce the mutation ratio of the target sequence (less than 10%); (3) the effect of anti-CRISPR-cpp28ori protein was significantly better than that of anti-CRISPR protein (p<0.001). This result indicated that cpp28ori could deliver anti-CRISPR protein into the cells, so that it could inhibit the function of Cas9 in the cells.

### Example 7: Evaluation of efficiency of cpp28ori to deliver antibodies into cells

In this example, the inventors verified whether cpp28ori could deliver antibodies into cells and exert antibody functions.

Through a flexible linker (SEQ ID NO: 39), cpp28ori was ligated to the C-terminal of Fc of anti-HBeAg chimeric antibody 2A7 (which VL and VH sequences were shown in SEQ ID NO: 47 and 48, respectively), anti-HBcAg chimeric antibody 16D5 (which VL and VH sequences were shown in SEQ ID NOs: 49 and 50, respectively), and anti-tyrosinase-related protein (TYRP1) chimeric antibody TA99 (which VL and VH sequences were shown in SEQ ID NOs: 51 and 52, respectively), thereby obtaining recombinant proteins 2A7-cpp28ori, 16D5-cpp28ori and TA99-cpp28ori. Among them, the antibody TA99 could specifically target the intracellular TYRP-1 antigen and reduce its expression; the 16D5 antibody could specifically target and clear the intracellular HBcAg antigen, and reduce the level of HBV DNA; and the 2A7 antibody could specifically target HBeAg (but not HBcAg).

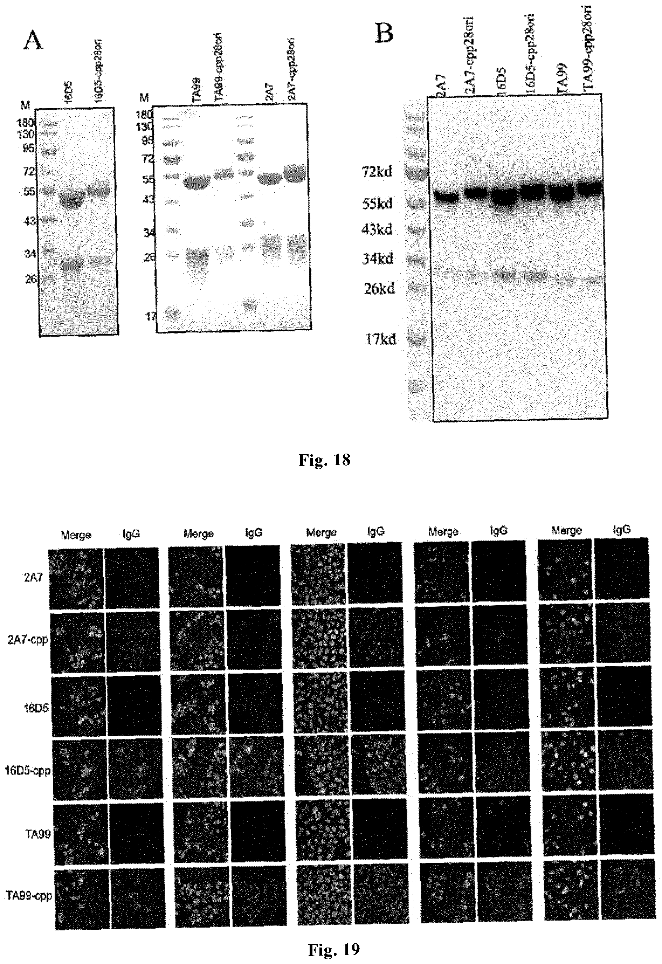

The antibodies 2A7, 16D5 and TA99 as well as the recombinant proteins 2A7-cpp28ori, 16D5-cpp28ori and TA99-cpp28ori were expressed in ExpiCHO cells by transient transfection. After 12 days, the cell supernatant was collected, and the antibody or recombinant protein expressed by the cells was purified by protein A column. The purified antibody or recombinant protein was detected by SDS-PAGE and Western blot (anti-human IgG). The experimental results were shown in Fig. 18. The results showed that the purified antibody or recombinant protein has a purity of more than 95% and could be used in the next cell experiments.

Five strains of HepG2-N10, HepG2-C3A, Hela, MNT-1 and A375 cells were inoculated in 96-well plates. After 12 h, the medium was removed, and a medium (DMEM, Gibco + 10% Gibco FBS) containing 100 µg/ml of 2A7-cpp28ori, 2A7, 16D5-cpp28ori, 16D5, TA99-cpp28ori or TA99 was added, and the cells were continuously cultured at 37°C for 6 h. Subsequently, the cells were washed three times with heparin, and intracellular antibodies were detected by immunofluorescence. The experimental results were shown in Fig. 19. The results showed that the antibodies fused to cpp28ori (2A7-cpp28ori, 16D5-cpp28ori and TA99-cpp28ori) could be detected in the various types of cells, while the antibodies (2A7, 16D5 and TA99) that were not fused to cpp28ori could not be detected in the cells. This showed that cpp28ori could effectively carry antibodies into various types of cells.

To verify whether the antibodies delivered into the cells by cpp28ori could still function, the following experiments were also performed.

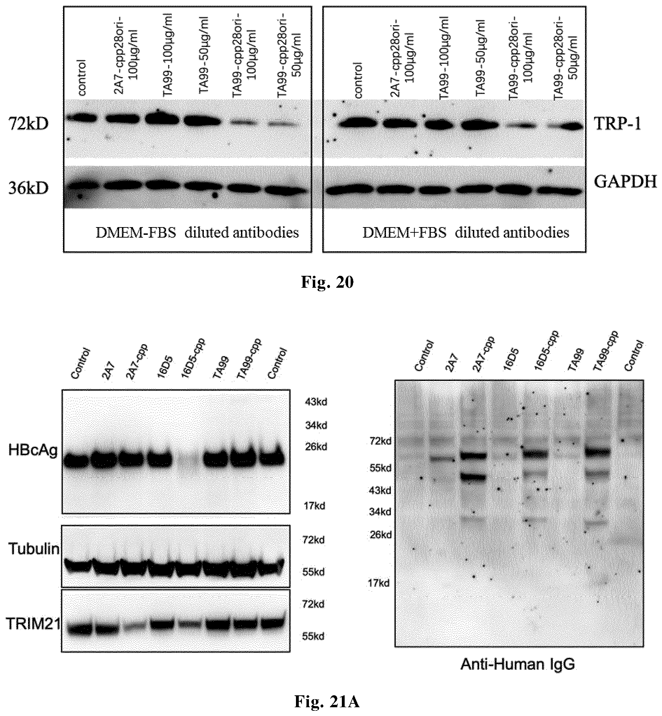

MNT-1 cells were inoculated at a density of 30,000 cells per well in 24-well plates. On the next day, the medium was removed and a medium (DMEM added with or without 10% Gibco FBS, Gibco) containing 2A7-cpp28ori (100 µg/ml; used as a control antibody), TA99 (100 µg/mL and 50 µg/mL), or TA99-cpp28ori (100 µg/mL and 50 µg/mL) was added, and the cells were continuously incubated for 6 h at 37°C. Subsequently, the medium was replaced with a normal medium (DMEM, Gibco+10% Gibco FBS), and the cells were continuously cultured for 48 h. Then, the cells were lysed with DDM lysate, and the lysate was subjected to western blot analysis to detect the level of TYRP-1 antigen therein. The experimental results were shown in Fig. 20. The results showed that the expression of TYRP-1 antigen was significantly reduced in the cells treated with TA99-cpp28ori. This indicated that cpp28ori could deliver TA99 antibody into the cells, and that the TA99-cpp28ori entered the cells could normally perform its biological function (i.e., could specifically target intracellular TYRP-1 antigen and reduce its expression).

HepG2-N10 cells were inoculated at a density of 30,000 cells per well in 24-well plates. On the next day, the medium was removed, a medium (DMEM, Gibco+10% Gibco FBS) containing 100 µg/ml of 2A7-cpp28ori, 2A7, 16D5-cpp28ori, 16D5, TA99-cpp28ori or TA99 antibody was added, and the cells were continuously cultured at 37°C for 6 h. Subsequently, the medium was replaced with a normal medium (DMEM, Gibco+10% Gibco FBS), and the cells were continuously cultured for 48 h. Then, the cells were lysed with DDM lysate, and the lysate was subjected to western blot analysis to detect the levels of HBcAg, TRIM21 and antibodies. In addition, the cell culture supernatant was collected, and the level of HBeAg antigen and the level of HBV DNA were detected. The experimental results were shown in Figs. 21A to 21B.

The results in Fig. 21A showed that the 2A7-cpp28ori, 16D5-cpp28ori and TA99-cpp28ori were detected in the cell lysates, indicating that these proteins were delivered into the cells. Further, the results in Fig. 21A showed that 16D5-cpp28ori could significantly clear the HBcAg antigen in the HepG2-N10 cells and reduce its intracellular level. Previous studies have shown that TRIM21 is a receptor for intracellular antibodies, which mediates the ubiquitination and degradation of the antibodies by binding to the Fc of antibodies. By detecting the level of TRIM21 protein in the cell lysate, it could be seen that 16D5-cpp28ori might reduce intracellular TRIM21 to achieve HBcAg clearance after entering the cells (Fig. 21A). In addition, the results in Fig. 21A also showed that 2A7-cpp28ori basically had no effect on the intracellular HBcAg level, but still significantly reduced the level of TRIM21.

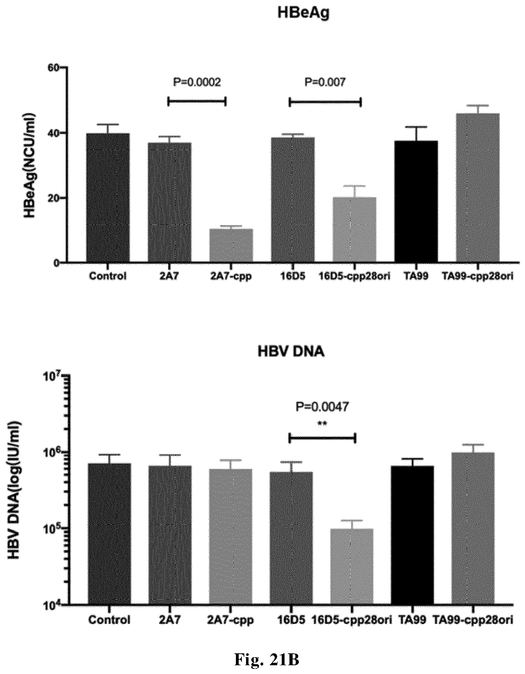

The results of Fig. 21B showed that the HBeAg level in the cell supernatant was significantly reduced under the action of 2A7-cpp28ori, and the treatment with 16D5-cpp28ori also inhibited the HBeAg level to a certain extent. In addition, the results in Fig. 21B also showed that the treatment with 16D5-cpp28ori was able to suppress HBV DNA level in cell supernatant, which was consistent with the previous findings that 16D5 antibody could reduce HBV DNA level in HBV transgenic mice.

The results in Figs. 21A to 21B showed that cpp28ori was not only capable of transmembrane delivery of various antibodies into the cells, but also able to precisely exert the specific function of each antibody. For example, the delivered antibodies could precisely recognize and target specific antigens (even HBeAg and HBcAg, which were two antigens with highly overlapping sequences).

### Example 8: Evaluation of efficiency of cpp28ori to deliver DNA into cells

In this example, the inventors verified whether cpp28ori could bind DNA and deliver it into cells to exert its function.

A previous study (Nat Methods. 2015 Nov;12(11):1085-90) showed that zinc finger protein (hereinafter referred to as ZF) could bind DNA efficiently. Therefore, a recombinant protein ZF-cpp28ori was constructed by ligating cpp28ori to the C-terminal of ZF protein (SEQ ID NO: 53) through a flexible linker (SEQ ID NO: 39).

Following the method described in Example 2, the ZF-cpp28ori was expressed in *E. coli* and purified by nickel column affinity chromatography. SDS-PAGE analysis showed that the purified ZF-cpp28ori protein had a purity of more than 90% and could be used in the next cell experiments. In addition, the binding sequence (SEQ ID NO: 54) of the zinc finger protein was inserted downstream of the mRuby3 gene on the pTT5-mRuby3 plasmid to obtain a plasmid pTT5-mRuby3-ZF motif.

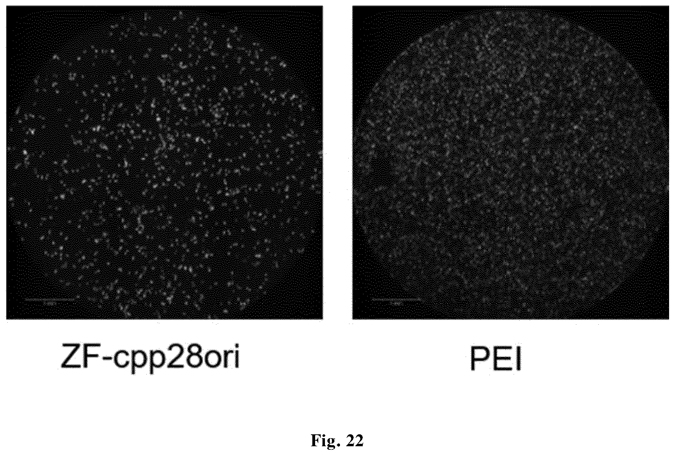

293β5 cells were inoculated at a density of 20,000 cells per well in 96-well plates. On the next day, the ZF-cpp28ori protein (60 µg/mL) and plasmid pTT5-mRuby3-ZF motif (0.8 µg) were diluted with a medium (DMEM, Gibco+10% Gibco FBS) in vitro, and pre-reacted at 37°C for 1 h. Then, the medium in the 96-well plate was removed, and the pre-reacted mixture was added to the cells. After 6 h, the pre-reacted mixture was removed, and a normal medium (DMEM, Gibco+10% Gibco FBS) was added, and the culturing was continued for 48 h. In addition, the plasmid pTT5-mRuby3-ZF motif was also transfected into 293β5 cells using PEI transfection reagent, which was used as a positive control. Then, the fluorescence of mRuby3 in the 293β5 cells was photographed and analyzed by a laser confocal high-content imaging analysis system. The experimental results were shown in Fig. 22. The results showed that the ZF-cpp28ori could effectively deliver the pTT5-mRuby3-ZF motif plasmid into the 293β5 cells to express red fluorescent protein mRuby3, and its delivery efficiency was higher than that of the PEI transfection reagent.

Although specific embodiments of the present invention have been described in detail, those skilled in the art will understand that various modifications and changes can be made to the details in light of all the teachings that have been disclosed, and that these changes are all within the scope of the present invention. The full scope of the present invention is given by the appended claims and any equivalents thereof.

## Claims

1. A cell-penetrating peptide or truncate thereof, wherein the cell-penetrating peptide has the structure represented by Formula I:
X₁X₂X₃X₄X₅X₆X₇X₈X₉X₁₀X₁₁PRRRX₁₆X₁₇X₁₈PRRRRX_{24Q}X₂₆PRRRRX₃₂X₃₃X₃₄X₃₅X₃₆X₃₇X₃₈X₃₉C Formula I
wherein,
X₁ to X₃ are each independently selected from (i) amino acid residue R; and (ii) amino acid residues (e.g., K or H) that are conservative substitutions relative to (i);
X₄ is selected from (i) amino acid residues R, C, G; and (ii) amino acid residues (e.g., K, H, N, Q, S, T, Y, W) that are conservatively substitutions relative to (i);
X₅ is selected from (i) amino acid residues R, N, G; and (ii) amino acid residues (e.g., K, H, C, Q, S, T, Y, W) that are conservatively substitutions relative to (i);
X₆ is selected from (i) amino acid residues R, G, P, S; and (ii) amino acid residues (e.g., K, H, N, Q, C, T, Y, W, A, V, L, I, M) that are conservative substitutions relative to (i);
X₇ is selected from (i) amino acid residues R, D, Q; and (ii) amino acid residues (e.g., K, H, N, G, C, S, T, Y, W, E) that are conservative substitutions relative to (i);
X₈ is selected from (i) amino acid residues R, A; and (ii) amino acid residues (e.g., K, H, V, L, I, M) that are conservative substitutions relative to (i);
X₉ is selected from (i) amino acid residues G, P, T; and (ii) amino acid residues (e.g., N, Q, S, C, Y, W, A, V, L, I, M) that are conservative substitutions relative to (i);
X₁₀ is selected from (i) amino acid residue R; and (ii) amino acid residues (e.g., K or H) that are conservative substitutions relative to (i);
X₁₁ is selected from (i) amino acid residues A, S, Q; and (ii) amino acid residues (e.g., V, L, I, M, N, G, T, C, Y, W) that are conservative substitutions relative to (i);
X₁₆ is selected from (i) amino acid residue T; and (ii) amino acid residues (e.g., N, Q, G, S, C, Y, W) that are conservative substitutions relative to (i);
X₁₇ is selected from (i) amino acid residue P; and (ii) amino acid residues (e.g., A, V, L, I, M) that are conservative substitutions relative to (i);
X₁₈ is selected from (i) amino acid residues S, Q; and (ii) amino acid residues (e.g., N, G, T, C, Y, W) that are conservative substitutions relative to (i);
X₂₄ is selected from (i) amino acid residue S; and (ii) amino acid residues (e.g., N, Q, G, T, C, Y, W) that are conservative substitutions relative to (i);
X₂₆ is selected from (i) amino acid residues S, C, Q; and (ii) amino acid residues (e.g., N, G, T, Y, W) that are conservative substitutions relative to (i);
X₃₂ is selected from (i) amino acid residues S, C; and (ii) amino acid residues (e.g., N, Q, G, T, Y, W) that are conservative substitutions relative to (i);
X₃₃ is selected from (i) amino acid residues Q, K; and (ii) amino acid residues (e.g., N, S, C, G, T, Y, W, R, H) that are conservative substitutions relative to (i);
X₃₄ is selected from (i) amino acid residues S, C; and (ii) amino acid residues (e.g., N, Q, G, T, Y, W) that are conservative substitutions relative to (i);
X₃₅ is selected from (i) amino acid residues R, P; and (ii) amino acid residues (e.g., K, H, A, V, L, I, M) that are conservative substitutions relative to (i);
X₃₆ is selected from (i) amino acid residues E, A; and (ii) amino acid residues (e.g., V, L, I, M, D) that are conservative substitutions relative to (i);
X₃₇ is selected from (i) amino acid residues P, S, C; and (ii) amino acid residues (e.g., A, V, L, I, M, N, Q, G, T, Y, W) that are conservative substitutions relative to (i);
X₃₈ is selected from (i) amino acid residues Q, S; and (ii) amino acid residues (e.g., N, C, G, T, Y, W) that are conservative substitutions relative to (i); and
X₃₉ is selected from (i) amino acid residues C, S, N; and (ii) amino acid residues (e.g., Q, G, T, Y, W) that are conservative substitutions relative to (i);
wherein, compared with the cell-penetrating peptide, the truncate is truncated by 1-10 (e.g., 1-5) amino acid residues at the N-terminal, and/or truncated by 1-14 (e.g., 1-8) amino acid residues at the C-terminal; and,
wherein, the cell-penetrating peptide or truncate thereof is able to perform transmembrane delivery of a biological molecule (e.g., a peptide of interest or nucleic acid of interest) into a cell.

2. The cell-penetrating peptide or truncate thereof according to claim 1, which has one or more features selected from the group consisting of:
(1) X₁ to X₃ are each independently selected from the group consisting of amino acid residues R, K and H; preferably, X₁ to X₃ are each independently selected from the group consisting of amino acid residues R and K; preferably, X₁ to X₃ are each independently amino acid residue R;
(2) X₄ is selected from the group consisting of amino acid residues R, C, G, K, H, N, Q, S, T, Y and W; preferably, X₄ is selected from the group consisting of amino acid residues R, C, G, K, N, Q, S and T; preferably, X₄ is selected from the group consisting of amino acid residues R, C, G and K; preferably, X₄ is selected from the group consisting of amino acid residues R, C and G;
(3) X₅ is selected from the group consisting of amino acid residues R, N, G, K, H, C, Q, S, T, Y and W; preferably, X₅ is selected from the group consisting of amino acid residues R, N, G, K, C, Q, S and T; preferably, X₅ is selected from the group consisting of amino acid residues R, N, G, K, C and Q; preferably, X₅ is selected from the group consisting of amino acid residues R, N and G;
(4) X₆ is selected from the group consisting of amino acid residues R, G, P, S, K, H, N, Q, C, T, Y, W, A, V, L, I and M; preferably, X₆ is selected from the group consisting of amino acid residues R, G, P, S, K, N, Q, C and T; X₆ is selected from the group consisting of amino acid residues R, G, P, S, K, C and T; preferably, X₆ is selected from the group consisting of amino acid residues R, G, P and S;
(5) X₇ is selected from the group consisting of amino acid residues R, D, Q, K, H, N, G, C, S, T, Y, W and E; preferably, X₇ is selected from the group consisting of amino acid residues R, D, Q, K, N, G, C, S, T and E; preferably, X₇ is selected from the group consisting of amino acid residues R, D, Q, K, N and E; preferably, X₇ is selected from the group consisting of amino acid residues R, D and Q;
(6) X₈ is selected from the group consisting of amino acid residues R, A, K, H, V, L, I, and M; preferably, X₈ is selected from the group consisting of amino acid residues R, A, K, V, L and I; preferably, X₈ is selected from the group consisting of amino acid residues R and A;
(7) X₉ is selected from the group consisting of amino acid residues G, P, T, N, Q, S, C, Y, W, A, V, L, I and M; preferably, X₉ is selected from the group consisting of amino acid residues G, P, T, N, Q, S and C; preferably, X₉ is selected from the group consisting of amino acid residues G, P, T, S and C; preferably, X₉ is selected from the group consisting of amino acid residues G, P and T;
(8) X₁₀ is selected from the group consisting of amino acid residues R, K and H; preferably, X₁₀ is selected from the group consisting of amino acid residues R and K; preferably, X₁₀ is amino acid residue R;
(9) X₁₁ is selected from the group consisting of amino acid residues A, S, V, L, I, M, N, Q, G, T, C, Y and W; preferably, X₁₁ is selected from the group consisting of amino acid residues A, S, V, L, I, N, Q, G, T and C; preferably, X₁₁ is selected from the group consisting of amino acid residues A, Q, S, V, L, I and T; preferably, X₁₁ is selected from the group consisting of amino acid residues A, Q and S;
(10) X₁₆ is selected from the group consisting of amino acid residues T, N, Q, G, S, C, Y and W; preferably, X₁₆ is selected from the group consisting of amino acid residues T, N, Q, G, S and C; preferably, X₁₆ is selected from the group consisting of amino acid residues T and S; preferably, X₁₆ is amino acid residue T;
(11) X₁₇ is selected from the group consisting of amino acid residues P, A, V, L, I and M; preferably, X₁₇ is amino acid residue P;
(12) Xis is selected from the group consisting of amino acid residues S, N, Q, G, T, C, Y and W; preferably, Xis is selected from the group consisting of amino acid residues S, N, Q, G, T and C; preferably, Xis is selected from the group consisting of amino acid residues S, Q and T; preferably, Xis is selected from the group consisting of amino acid residues S and Q;
(13) X₂₄ is selected from the group consisting of amino acid residues S, N, Q, G, T, C, Y and W; preferably, X₂₄ is selected from the group consisting of amino acid residues S, N, Q, G, T and C; preferably, X₂₄ is selected from the group consisting of amino acid residues S and T; preferably, X₂₄ is amino acid residue S;
(14) X₂₆ is selected from the group consisting of amino acid residues S, N, Q, G, T, C, Y and W; preferably, X₂₆ is selected from the group consisting of amino acid residues S, N, Q, G, T and C; preferably, X₂₆ selected from the group consisting of amino acid residues S, C and Q;
(15) X₃₂ is selected from the group consisting of amino acid residues S, C, N, Q, G, T, Y and W; preferably, X₃₂ is selected from the group consisting of amino acid residues S, C, N, Q, G and T; preferably, X₃₂ is selected from the group consisting of amino acid residues S, C, G and T; preferably, X₃₂ is selected from the group consisting of amino acid residues S and C;
(16) X₃₃ is selected from the group consisting of amino acid residues Q, K, N, S, C, G, T, Y, W, R and H; preferably, X₃₃ is selected from the group consisting of amino acid residues Q, K, N, S, C, G, T and R; preferably, X₃₃ is selected from the group consisting of amino acid residues Q, K, N and R; preferably, X₃₃ is selected from the group consisting of amino acid residues Q and K;
(17) X₃₄ is selected from the group consisting of amino acid residues S, C, N, Q, G, T, Y and W; preferably, X₃₄ is selected from the group consisting of amino acid residues S, C, N, Q, G and T; preferably, X₃₄ is selected from the group consisting of amino acid residues S, C, G and T; preferably, X₃₄ is selected from the group consisting of amino acid residues S and C;
(18) X₃₅ is selected from the group consisting of amino acid residues R, P, K, H, A, V, L, I and M; preferably, X₃₅ is selected from the group consisting of amino acid residues R, P, K and H; preferably, X₃₅ is selected from the group consisting of amino acid residues R, P and K; preferably, X₃₅ is selected from the group consisting of amino acid residues R and P;
(19) X₃₆ is selected from the group consisting of amino acid residues E, A, V, L, I, M and D; preferably, X₃₆ is selected from the group consisting of amino acid residues E, A, V, L, I and D; preferably, X₃₆ is selected from the group consisting of amino acid residues E and A;
(20) X₃₇ is selected from the group consisting of amino acid residues P, S, C, A, V, L, I, M, N, Q, G, T, Y and W; preferably, X₃₇ is selected from the group consisting of amino acid residues P, S, C, N, Q, G and T; preferably, X₃₇ is selected from the group consisting of amino acid residues P, S, C, G and T; preferably, X₃₇ is selected from the group consisting of amino acid residues P, S and C;
(21) X₃₈ is selected from the group consisting of amino acid residues Q, S, N, C, G, T, Y and W; preferably, X₃₈ is selected from the group consisting of amino acid residues Q, S, N, C, G and T; preferably, X₃₈ is selected from the group consisting of amino acid residues Q, S, N and T; preferably, X₃₈ is selected from the group consisting of amino acid residues Q and S; and
(22) X₃₉ is selected from the group consisting of amino acid residues C, S, N, Q, G, T, Y and W; preferably, X₃₉ is selected from the group consisting of amino acid residues C, S, N, Q, G and T; preferably, X₃₉ selected from the group consisting of amino acid residues C, S and N.

3. The cell-penetrating peptide or truncate thereof according to claim 1 or 2, wherein the cell-penetrating peptide has the structure represented by Formula II:
RRRX₄X₅X₆X₇X₈X₉RX₁₁PRRRTPSPRRRRSQSPRRRRX₃₂X₃₃X₃₄X₃₅X₃₆X₃₇X₃₈X₃₉C Formula II
wherein,
X₄ is selected from (i) amino acid residues R, C, G; and (ii) amino acid residues (e.g., K, H, N, Q, S, T, Y, W) that are conservative substitutions relative to (i);
X₅ is selected from (i) amino acid residues R, N, G; and (ii) amino acid residues (e.g., K, H, C, Q, S, T, Y, W) that are conservative substitutions relative to (i);
X₆ is selected from (i) amino acid residues R, G, P, S; and (ii) amino acid residues (e.g., K, H, N, Q, C, T, Y, W, A, V, L, I, M) that are conservative substitutions relative to (i);
X₇ is selected from (i) amino acid residues R, D, Q; and (ii) amino acid residues (e.g., K, H, N, G, C, S, T, Y, W, E) that are conservative substitutions relative to (i);
X₈ is selected from (i) amino acid residues R, A; and (ii) amino acid residues (e.g., K, H, V, L, I, M) that are conservative substitutions relative to (i);
X₉ is selected from (i) amino acid residues G, P, T; and (ii) amino acid residues (e.g., N, Q, S, C, Y, W, A, V, L, I, M) that are conservative substitutions relative to (i);
X₁₁ is selected from (i) amino acid residues A, S, Q; and (ii) amino acid residues (e.g., V, L, I, M, N, G, T, C, Y, W) that are conservative substitutions relative to (i);
X₃₂ is selected from (i) amino acid residues S, C; and (ii) amino acid residues (e.g., N, Q, G, T, Y, W) that are conservative substitutions relative to (i);
X₃₃ is selected from (i) amino acid residues Q, K; and (ii) amino acid residues (e.g., N, S, C, G, T, Y, W, R, H) that are conservative substitutions relative to (i);
X₃₄ is selected from (i) amino acid residues S, C; and (ii) amino acid residues (e.g., N, Q, G, T, Y, W) that are conservative substitutions relative to (i);
X₃₅ is selected from (i) amino acid residues R, P; and (ii) amino acid residues (e.g., K, H, A, V, L, I, M) that are conservative substitutions relative to (i);
X₃₆ is selected from (i) amino acid residues E, A; and (ii) amino acid residues (e.g., V, L, I, M, D) that are conservative substitutions relative to (i);
X₃₇ is selected from (i) amino acid residues P, S, C; and (ii) amino acid residues (e.g., A, V, L, I, M, N, Q, G, T, Y, W) that are conservative substitutions relative to (i);
X₃₈ is selected from (i) amino acid residues Q, S; and (ii) amino acid residues (e.g., N, C, G, T, Y, W) that are conservative substitutions relative to (i); and
X₃₉ is selected from (i) amino acid residues C, S, N; and (ii) amino acid residues (e.g., Q, G, T, Y, W) that are conservative substitutions relative to (i);
preferably, the cell-penetrating peptide has the structure represented by Formula II:
RRRX₄X₅X₆X₇X₈X₉RX₁₁PRRRTPSPRRRRSQSPRRRRX₃₂X₃₃X₃₄X₃₅X₃₆X₃₇X₃₈X₃₉C Formula II
wherein, X₄ is selected from the group consisting of amino acid residues R, C, G;
X₅ is selected from the group consisting of amino acid residues R, N, G;
X₆ is selected from the group consisting of amino acid residues R, G, P, S;
X₇ is selected from the group consisting of amino acid residues R, D, Q;
X₈ is selected from the group consisting of amino acid residues R, A;
X₉ is selected from the group consisting of amino acid residues G, P, T;
X₁₁ is selected from the group consisting of amino acid residues A, S, Q;
X₃₂ is selected from the group consisting of amino acid residues S, C;
X₃₃ is selected from the group consisting of amino acid residues Q, K;
X₃₄ is selected from the group consisting of amino acid residues S, C;
X₃₅ is selected from the group consisting of amino acid residues R, P;
X₃₆ is selected from the group consisting of amino acid residues E, A;
X₃₇ is selected from the group consisting of amino acid residues P, S, C;
X₃₈ is selected from the group consisting of amino acid residues Q, S; and
X₃₉ is selected from the group consisting of amino acid residues C, S, N.

4. The cell-penetrating peptide or truncate thereof according to any one of claims 1-3, wherein compared with the cell-penetrating peptide, the truncate is truncated by 1-10 amino acid residues (e.g., 1-5 amino acid residues) at the N-terminal, and/or truncated by 1-14 amino acid residues (e.g., 1-8 amino acid residues) at the C-terminal;
preferably, compared to the cell-penetrating peptide, the truncate is truncated by 1-10 amino acid residues at the N-terminal, for example, truncated by 1, 2, 3, 4, 5, 6, 7, 8, 9 or 10 amino acid residues at the N-terminal;
preferably, compared to the cell-penetrating peptide, the truncate is truncated by 1-14 amino acid residues at the C-terminal, for example, truncated by 1, 2, 3, 4, 5, 6, 7, 8, 9, 10, 11, 12, 13 or 14 amino acid residues at the C-terminal;
preferably, compared to the cell-penetrating peptide, the truncate is truncated by 1-10 amino acid residues (e.g., 1-5 amino acid residues) at the N-terminal, and truncated by 1-14 amino acid residues (e.g., 1-8 amino acid residues) at the C-terminal; for example, compared to the cell-penetrating peptide, the truncate is truncated by 5 or 10 amino acid residues at the N-terminal and truncated by 1-8 amino acid residues (e.g., 1, 3, 6 or 8 amino acid residues) at the C-terminal;
for example, compared to the cell-penetrating peptide, the truncate is truncated by 1-10 amino acid residues (e.g., 2, 3, 5, or 10 amino acid residues) at the N-terminal, and is not truncated or truncated by 1-8 amino acid residues (e.g., 1, 3, 6 or 8 amino acid residues) at the C-terminal;
for example, compared with the cell-penetrating peptide, the truncate is truncated by 5 amino acid residues at the N-terminal, and truncated by 1-14 amino acid residues (e.g., 1, 3, 6, 8 or 14 amino acid residues) at the C-terminal;
for example, compared to the cell-penetrating peptide, the truncate is truncated by 1-5 amino acid residues (e.g., 2, 3 or 5 amino acid residues) at the N-terminal, and is not truncated or truncated by 1-14 amino acid residues (e.g., 1, 3, 6, 8 or 14 amino acid residues) at the C-terminal;
for example, compared to the cell-penetrating peptide, the truncate is truncated by 14 amino acid residues at the C-terminal, and X₂₆ is amino acid residue C.

5. The cell-penetrating peptide or truncate thereof according to any one of claims 1-4, wherein the cell-penetrating peptide has the structure represented by Formula III:
RRRGX₅X₆RX₈X₉RSPRRRTPSPRRRRSQSPRRRRX₃₂QX₃₄X₃₅X₃₆X₃₇SNC Formula III
wherein,
X₅ is selected from (i) amino acid residues R, N, G; and (ii) amino acid residues (e.g., K, H, C, Q, S, T, Y, W) that are conservative substitutions relative to (i);
X₆ is selected from (i) amino acid residues R, G, P, S; and (ii) amino acid residues (e.g., K, H, N, Q, C, T, Y, W, A, V, L, I, M) that are conservative substitutions relative to (i);
X₈ is selected from (i) amino acid residues R, A; and (ii) amino acid residues (e.g., K, H, V, L, I, M) that are conservative substitutions relative to (i);
X₉ is selected from (i) amino acid residues G, P, T; and (ii) amino acid residues (e.g., N, Q, S, C, Y, W, A, V, L, I, M) that are conservative substitutions relative to (i);
X₃₂ and X₃₄ are each independently selected from (i) amino acid residues S, C; and (ii) amino acid residues (e.g., N, Q, G, T, Y, W) that are conservative substitutions relative to (i);
X₃₅ is selected from (i) amino acid residues R, P; and (ii) amino acid residues (e.g., K, H, A, V, L, I, M) that are conservative substitutions relative to (i);
X₃₆ is selected from (i) amino acid residues E, A; and (ii) amino acid residues (e.g., V, L, I, M, D) that are conservative substitutions relative to (i); and
X₃₇ is selected from (i) amino acid residues P, S, C; and (ii) amino acid residues (e.g., A, V, L, I, M, N, Q, G, T, Y, W) that are conservative substitutions relative to (i);
preferably, the cell-penetrating peptide or truncate thereof has one or more characteristics selected from the group consisting of:
(1) X₅ is selected from the group consisting of amino acid residues R, N, G, K, H, C, Q, S, T, Y and W; preferably, X₅ is selected from the group consisting of amino acid residues N, G, C, Q, S and T; preferably, X₅ is selected from the group consisting of amino acid residues N, G, C and Q; preferably, X₅ is selected from the group consisting of amino acid residues N and G;
(2) X₆ is selected from the group consisting of amino acid residues R, G, P, S, K, H, N, Q, C, T, Y, W, A, V, L, I and M; preferably, X₆ is selected from the group consisting of amino acid residues residues P, S, N, Q, C and T; X₆ is selected from the group consisting of amino acid residues P, S and T; preferably, X₆ is selected from the group consisting of amino acid residues P and S;
(3) X₈ is selected from the group consisting of amino acid residues R, A, K, H, V, L, I, and M; preferably, X₈ is selected from the group consisting of amino acid residues R, A, K, V, L and I; preferably, X₈ is selected from the group consisting of amino acid residues R and A; preferably, X₈ is amino acid residue A;
(4) X₉ is selected from the group consisting of amino acid residues G, P, T, N, Q, S, C, Y, W, A, V, L, I and M; preferably, X₉ is selected from the group consisting of amino acid residues G, P, T, N, Q, S and C; preferably, X₉ is selected from the group consisting of amino acid residues P, T and S; preferably, X₉ is selected from the group consisting of amino acid residues P and T;
(5) X₃₂ and X₃₄ are each independently selected from the group consisting of amino acid residues S, C, N, Q, G, T, Y and W; preferably, X₃₂ and X₃₄ are each independently selected from the group consisting of amino acid residues S, C, N, Q, G and T; preferably, X₃₂ and X₃₄ are each independently selected from the group consisting of amino acid residues S, C, G and T; preferably, X₃₂ and X₃₄ are each independently selected from the group consisting of amino acid residues S and C; preferably, X₃₂ and X₃₄ are each independently amino acid residue S;
(6) X₃₅ is selected from the group consisting of amino acid residues R, P, K, H, A, V, L, I and M; preferably, X₃₅ is selected from the group consisting of amino acid residues R, P, K and H; preferably, X₃₅ is selected from the group consisting of amino acid residues R, P and K; preferably, X₃₅ is selected from the group consisting of amino acid residues R and P; preferably, X₃₅ is amino acid residue P;
(7) X₃₆ is selected from the group consisting of amino acid residues E, A, V, L, I, M and D; preferably, X₃₆ is selected from the group consisting of amino acid residues E, A, V, L, I and D; preferably, X₃₆ is selected from the group consisting of amino acid residues E and A; preferably, X₃₆ is amino acid residue A; and
(8) X₃₇ is selected from the group consisting of amino acid residues P, S, C, A, V, L, I, M, N, Q, G, T, Y and W; preferably, X₃₇ is selected from the group consisting of amino acid residues P, S, C, N, Q, G and T; preferably, X₃₇ is selected from the group consisting of amino acid residues P, S, C, G and T; preferably, X₃₇ is selected from the group consisting of amino acid residues P, S and C; preferably, X₃₇ is selected from the group consisting of amino acid residues P and S;
preferably, the cell-penetrating peptide has an amino acid sequence selected from the group consisting of SEQ ID NOs: 20-21.

6. The cell-penetrating peptide or truncate thereof according to claim 1 or 2, wherein the truncate comprises a structure represented by Formula IV:
X₁₁PRRRTPX₁₈PRRRRSQX₂₆ Formula IV
wherein,
X₁₁ is selected from (i) amino acid residues S, Q, A; and (ii) amino acid residues (e.g., V, L, I, M, N, G, T, C, Y, W) that are conservative substitutions relative to (i);
Xis is selected from (i) amino acid residues S, Q; and (ii) amino acid residues (e.g., C, N, G, T, Y, W) that are conservative substitutions relative to (i);
X₂₆ is selected from (i) amino acid residues S, C, Q; and (ii) amino acid residues (e.g., N, G, T, Y, W) that are conservative substitutions relative to (i);
preferably, the truncate has one or more characteristics selected from the group consisting of:
(1) X₁₁ is selected from the group consisting of amino acid residues A, S, V, L, I, M, N, Q, G, T, C, Y and W; preferably, X₁₁ is selected from the group consisting of amino acid residues A, S, V, L, I, N, Q, G, T and C; preferably, X₁₁ is selected from the group consisting of amino acid residues A, Q, S, V, L, I and T; preferably, X₁₁ is selected from the group consisting of amino acid residues A, Q and S;
(2) Xis is selected from the group consisting of amino acid residues S, N, Q, G, T, C, Y and W; preferably, X₁₈ is selected from the group consisting of amino acid residues S, N, Q, G, T and C; preferably, Xis is selected from the group consisting of amino acid residues S, Q and T; preferably, Xis is selected from the group consisting of amino acid residues S and Q;
(3) X₂₆ is selected from the group consisting of amino acid residues S, N, Q, G, T, C, Y and W; preferably, X₂₆ is selected from the group consisting of amino acid residues S, N, Q, G, T and C; preferably, X₂₆ is selected from the group consisting of amino acid residues S, C and Q.

7. The cell-penetrating peptide or truncate thereof according to claim 1 or 2, wherein the truncate comprises a structure represented by Formula V:
X₆X₇RGRX₁₁PRRRTPX₁₈PRRRRSQX₂₆PRRRRX₃₂ Formula V
wherein,
X₆ is selected from (i) amino acid residues R, G; and (ii) amino acid residues (e.g., K, H, C, Q, S, T, Y, W, N) that are conservative substitutions relative to (i);
X₇ is selected from (i) amino acid residues R, D, Q; and (ii) amino acid residues (e.g., K, H, N, G, C, S, T, Y, W, E) that are conservative substitutions relative to (i);
X₁₁ is selected from (i) amino acid residues S, A, Q; and (ii) amino acid residues (e.g., V, L, I, M, N, G, T, C, Y, W) that are conservative substitutions relative to (i);
X₁₈ and X₂₆ are each independently selected from (i) amino acid residues S, Q; and (ii) amino acid residues (e.g., N, G, T, C, Y, W) that are conservative substitutions relative to (i);
X₃₂ is selected from (i) amino acid residues S, C; and (ii) amino acid residues (e.g., N, Q, G, T, Y, W) that are conservative substitutions relative to (i);
preferably, the truncate has one or more characteristics selected from the group consisting of:
(1) X₆ is selected from the group consisting of amino acid residues R, N, G, K, H, C, Q, S, T, Y and W; preferably, X₆ is selected from the group consisting of amino acid residues R, N, G, K, C, Q, S and T; preferably X₆ is selected from the group consisting of amino acid residues R, N, G, K, C and Q; preferably X₆ is selected from the group consisting of amino acid residues R and G; preferably X₆ is selected from amino acid residue R;
(2) X₇ is selected from the group consisting of amino acid residues R, D, Q, K, H, N, G, C, S, T, Y, W and E; preferably, X₇ is selected from the group consisting of amino acid residues R, D, Q, K, N, G, C, S, T and E; preferably, X₇ is selected from the group consisting of amino acid residues R, D, Q, K, N and E; preferably, X₇ is selected from the group consisting of amino acid residues R, D and Q; Preferably, X₇ is selected from amino acid residue R;
(3) X₁₁ is selected from the group consisting of amino acid residues A, S, V, L, I, M, N, Q, G, T, C, Y and W; preferably, X₁₁ is selected from the group consisting of amino acid residues A, S, V, L, I, N, Q, G, T and C; preferably, X₁₁ is selected from the group consisting of amino acid residues A, Q, S, V, L, I and T; preferably, X₁₁ is selected from the group consisting of amino acid residues A, Q and S; preferably, X₁₁ is selected from the group consisting of amino acid residues Q and S;
(4) X₁₈ and X₂₆ are each independently selected from the group consisting of amino acid residues S, N, Q, G, T, C, Y and W; preferably, X₁₈ and X₂₆ are each independently selected from the group consisting of amino acid residues S, N, Q, G, T and C; preferably, X₁₈ and X₂₆ are each independently selected from the group consisting of amino acid residues S, Q and T; preferably, X₁₈ and X₂₆ are each independently selected from the group consisting of amino acid residues Q and S;
(5) X₃₂ is selected from the group consisting of amino acid residues S, C, N, Q, G, T, Y and W; preferably, X₃₂ is selected from the group consisting of amino acid residues S, C, N, Q, G and T; preferably, X₃₂ is selected from the group consisting of amino acid residues S, C, G and T; preferably, X₃₂ is selected from the group consisting of amino acid residues S and C.

8. The cell-penetrating peptide or truncate thereof according to claim 1 or 2, wherein the truncate comprises a structure represented by Formula VI:
X₆X₇RGRX₁₁PRRRTPX₁₈PRRRRSQX₂₆PRRRRSX₃₃SRX₃₆X₃₇ Formula VI
wherein,
X₆ is selected from (i) amino acid residues R, G; and (ii) amino acid residues (e.g., K, H, C, Q, S, T, Y, W, N) that are conservative substitutions relative to (i);
X₇ is selected from (i) amino acid residues R, D, Q; and (ii) amino acid residues (e.g., K, H, N, G, C, S, T, Y, W, E) that are conservative substitutions relative to (i);
X₁₁ is selected from (i) amino acid residues S, A, Q; and (ii) amino acid residues (e.g., V, L, I, M, N, G, T, C, Y, W) that are conservative substitutions relative to (i);
X₁₈ and X₂₆ are each independently selected from (i) amino acid residues S, Q; and (ii) amino acid residues (e.g., N, G, T, C, Y, W) that are conservative substitutions relative to (i);
X₃₃ is selected from (i) amino acid residues Q, K; and (ii) amino acid residues (e.g., N, S, C, G, T, Y, W, R, H) that are conservative substitutions relative to (i);
X₃₆ is selected from (i) amino acid residues E, A; and (ii) amino acid residues (e.g., V, L, I, M, D) that are conservative substitutions relative to (i);
X₃₇ is selected from (i) amino acid residues S, C; and (ii) amino acid residues (e.g., N, Q, G, T, Y, W) that are conservative substitutions relative to (i);
preferably, the truncate has one or more characteristics selected from the group consisting of:
(1) X₆ is selected from the group consisting of amino acid residues R, N, G, K, H, C, Q, S, T, Y and W; preferably, X₆ is selected from the group consisting of amino acid residues R, N, G, K, C, Q, S and T; preferably X₆ is selected from the group consisting of amino acid residues R, N, G, K, C and Q; preferably X₆ is selected from the group consisting of amino acid residues R and G; preferably X₆ is selected from amino acid residue R;
(2) X₇ is selected from the group consisting of amino acid residues R, D, Q, K, H, N, G, C, S, T, Y, W and E; preferably, X₇ is selected from the group consisting of amino acid residues R, D, Q, K, N, G, C, S, T and E; preferably, X₇ is selected from the group consisting of amino acid residues R, D, Q, K, N and E; preferably, X₇ is selected from the group consisting of amino acid residues R, D and Q; Preferably, X₇ is selected from the amino acid residue R;
(3) X₁₁ is selected from the group consisting of amino acid residues A, S, V, L, I, M, N, Q, G, T, C, Y and W; preferably, X₁₁ is selected from the group consisting of amino acid residues A, S, V, L, I, N, Q, G, T and C; preferably, X₁₁ is selected from the group consisting of amino acid residues A, Q, S, V, L, I and T; preferably, X₁₁ is selected from the group consisting of amino acid residues A, Q and S; preferably, X₁₁ is selected from the group consisting of amino acid residues Q and S;
(4) X₁₈ and X₂₆ are each independently selected from the group consisting of amino acid residues S, N, Q, G, T, C, Y and W; preferably, X₁₈ and X₂₆ are each independently selected from the group consisting of amino acid residues S, N, Q, G, T and C; preferably, X₁₈ and X₂₆ are each independently selected from the group consisting of amino acid residues S, Q and T; preferably, X₁₈ and X₂₆ are each independently selected from the group consisting of amino acid residues Q and S;
(5) X₃₃ is selected from the group consisting of amino acid residues Q, K, N, S, C, G, T, Y, W, R and H; preferably, X₃₃ is selected from the group consisting of amino acid residues Q, K, N, S, C, G, T and R; preferably, X₃₃ is selected from the group consisting of amino acid residues Q, K, N and R; preferably, X₃₃ is selected from the group consisting of amino acid residues Q and K; preferably, X₃₃ is selected from amino acid residue Q;
(6) X₃₆ is selected from the group consisting of amino acid residues E, A, V, L, I, M and D; preferably, X₃₆ is selected from the group consisting of amino acid residues E, A, V, L, I and D; preferably, X₃₆ is selected from the group consisting of amino acid residues E and A; preferably, X₃₆ is selected from amino acid residue E;
(7) X₃₇ is selected from the group consisting of amino acid residues S, C, N, Q, G, T, Y and W; preferably, X₃₇ is selected from the group consisting of amino acid residues S, C, N, Q, G and T; preferably, X₃₇ is selected from the group consisting of amino acid residues S, C, G and T; preferably, X₃₇ is selected from the group consisting of amino acid residues S and C.

9. The cell-penetrating peptide or truncate thereof according to any one of claims 1-4, wherein the truncate has a structure represented by Formula VII:
RX₇RGRX₁₁PRRRTPSPRRRRSQSPRRRRX₃₂X₃₃X₃₄RX₃₆X₃₇QX₃₉ Formula VII
wherein,
X₇ is selected from (i) amino acid residues R, D, Q; and (ii) amino acid residues (e.g., K, H, N, G, C, S, T, Y, W, E) that are conservative substitutions relative to (i);
X₁₁ is selected from (i) amino acid residues A, S; and (ii) amino acid residues (e.g., V, L, I, M, N, Q, G, T, C, Y, W) that are conservative substitutions relative to (i);
X₃₂, X₃₄, X₃₇ and X₃₉ are each independently selected from (i) amino acid residues S, C; and (ii) amino acid residues (e.g., N, Q, G, T, Y, W) that are conservative substitutions with respect to (i);
X₃₃ is selected from (i) amino acid residues Q, K; and (ii) amino acid residues (e.g., N, S, C, G, T, Y, W, R, H) that are conservative substitutions relative to (i); and
X₃₆ is selected from (i) amino acid residues E, A; and (ii) amino acid residues (e.g., V, L, I, M, D) that are conservative substitutions relative to (i);
preferably, the truncate has one or more characteristics selected from the group consisting of:
(1) X₇ is selected from the group consisting of amino acid residues R, D, Q, K, H, N, G, C, S, T, Y, W and E; preferably, X₇ is selected from the group consisting of amino acid residues R, D, Q, K, N, G, C, S, T and E; preferably, X₇ is selected from the group consisting of amino acid residues R, D, Q, K, N and E; preferably, X₇ is selected from the group consisting of amino acid residues R, D and Q; Preferably, X₇ is selected from the group consisting of amino acid residues R and Q;
(2) X₁₁ is selected from the group consisting of amino acid residues A, S, V, L, I, M, N, Q, G, T, C, Y and W; preferably, X₁₁ is selected from the group consisting of amino acid residues A, S, V, L, I, N, Q, G, T and C; preferably, X₁₁ is selected from the group consisting of amino acid residues A, S, V, L, I and T; preferably, X₁₁ is selected from the group consisting of amino acid residues A and S;
(3) X₃₂, X₃₄, X₃₇ and X₃₉ are each independently selected from the group consisting of amino acid residues S, C, N, Q, G, T, Y and W; preferably, X₃₂, X₃₄, X₃₇ and X₃₉ are each independently selected from the group consisting of amino acid residues S, C, N, Q, G and T; preferably, X₃₂, X₃₄, X₃₇ and X₃₉ are each independently selected from the group consisting of amino acid residues S, C, G and T; preferably, X₃₂, X₃₄, X₃₇ and X₃₉ each independently selected from the group consisting of amino acid residues S and C;
(4) X₃₃ is selected from the group consisting of amino acid residues Q, K, N, S, C, G, T, Y, W, R and H; preferably, X₃₃ is selected from the group consisting of amino acid residues Q, K, N, S, C, G, T and R; preferably, X₃₃ is selected from the group consisting of amino acid residues Q, K, N and R; preferably, X₃₃ is selected from the group consisting of amino acid residues Q and K; and
(5) X₃₆ is selected from the group consisting of amino acid residues E, A, V, L, I, M and D; preferably, X₃₆ is selected from the group consisting of amino acid residues E, A, V, L, I and D; preferably, X₃₆ is selected from the group consisting of amino acid residues E and A;
preferably, the cell-penetrating peptide or truncate thereof has an amino acid sequence selected from the group consisting of SEQ ID NOs: 10-13, 15, 17-18, 20-21, 23-29 and 32-37.

10. A fusion protein, comprising the cell-penetrating peptide or truncate thereof according to any one of claims 1-9, and a peptide of interest;
preferably, the peptide of interest is directly covalently linked to the cell-penetrating peptide or truncate thereof; or, covalently linked via a peptide linker (e.g., a flexible peptide linker);
preferably, the cell-penetrating peptide or truncate thereof is linked to the N-terminal (optionally, via a peptide linker) or C-terminal (optionally, via a peptide linker) of the peptide of interest;
preferably, the peptide of interest is an antibody; preferably, the antibody is selected from the group consisting of anti-HBV antibody (e.g., anti-HBsAg antibody, anti-HBcAg antibody, anti-HBeAg antibody, etc.), anti-influenza virus antibody (e.g., anti-HA1 antibody, anti-HA2 antibody), anti-tumor antigen antibody (e.g., anti-p53 antibody, anti-kras antibody, anti-PRL-3 antibody), anti-immune checkpoint antibody (e.g., anti-PD1 or PDL1 antibody), anti-melanin synthesis-related antibody (e.g., tyrosinase-related protein TYRP1 antibody), anti-novel coronavirus antibody (e.g., anti-novel coronavirus S protein antibody, such as anti-S protein RBD or S1 or S2 antibody);
preferably, the peptide of interest is a protein related to gene editing; preferably, the protein is selected from the group consisting of Cas9 protein, AcrIIA4 protein, Cas13 protein, Cre recombinase and Flip recombinase;
preferably, the peptide of interest is an active or traceable protein; preferably, the protein is selected from the group consisting of fluorescent protein (e.g., green fluorescent protein), toxin protein (e.g., endotoxin), cytokine (e.g., interleukin or interferon, such as IL10 or IPNγ), immunomodulatory protein (e.g., PD1), enzyme (e.g., luciferase, nuclease, recombinase, methylase, protein kinase, etc.), signaling pathway-related molecular protein (e.g., β-catenin protein), cyclin (e.g., Cyclin D1 protein), transcription activator and transcription inhibitor;
preferably, the peptide of interest is a protein capable of binding a DNA molecule; preferably, the protein is selected from the group consisting of zinc finger protein and transcription activator-like effector nuclease (TALEN protein);
preferably, the peptide of interest has an amino acid sequence selected from the group consisting of: SEQ ID NOs: 1, 6-7, 43-44 and 46-53;
preferably, the fusion protein further comprises an additional domain; preferably, the fusion protein further comprises a tag domain (e.g., 6*His tag, HA tag, hapten tag, Strep tag, MBP tag, GST tag, etc.), and/or, an antibody heavy chain constant region; preferably, the additional domain (e.g., a tag domain) is located at the N-terminal or C-terminal of the fusion protein.

11. A conjugate, comprising the cell-penetrating peptide or truncate thereof according to any one of claims 1-9, and a molecule of interest (e.g., a protein of interest or a nucleic acid of interest);
preferably, the molecule of interest is directly covalently linked to the cell-penetrating peptide or truncate thereof or is covalently linked through a linker (e.g., a bifunctional linker or a peptide linker); or, the molecule of interest is non-covalently linked to the cell-penetrating peptide or truncate thereof;
preferably, the molecule of interest is a detectable label such as a fluorescent label (e.g., fluorescein, rhodamine, dansyl, phycoerythrin or Texas red), an enzyme label (e.g., horseradish peroxidase, alkaline phosphatase, luciferase, glucoamylase, lysozyme, glucose oxidase or β-D galactosidase), stable isotope or radioisotope, chromophore moiety, digoxigenin, biotin/avidin, DNA molecule or gold;
preferably, the molecule of interest is a cytotoxic agent; for example, paclitaxel, cytochalasin B, gramicidin D, ethidium bromide, ipecamine, mitomycin, etoposide, teniposide, vincristine, vinblastine, colchicine, doxorubicin, daunorubicin, dihydroxyanthracin diketone, mitoxantrone, mithramycin, actinomycin D, 1-dehydrotestosterone, glucocorticoid, procaine, tetracaine, lidocaine, propranolol, puromycin and analog thereof, antimetabolite (e.g., methotrexate, 6-mercaptopurine, 6-mercaptoguanine, cytosine arabinoside, 5-fluorouracil dcrbzine), alkylating agent (e.g., mustine, thiotepa chlorambucil, melphalan, carmustine (BSNU) and lomustine (CCNU), cyclophosphamide, Busulfan, dibromomannitol, streptozotocin, mitomycin C, and cis-dichlorodiamineplatinum (DDP)), anthracycline (e.g., idarubicin (formerly daunorubicin) and doxorubicin), antibiotic (e.g., genshammycin (formerly known as actinomycin), bleomycin, mithramycin, and anthiamycin (AMC)), as well as antimitotic agent (e.g., vincristine and vinblastine);
preferably, the molecule of interest is a protein or polypeptide;
preferably, the molecule of interest is an antibody; preferably, the antibody is selected from the group consisting of anti-HBV antibody (e.g., anti-HBsAg antibody, anti-HBcAg antibody, anti-HBeAg antibody, etc.), anti-influenza virus antibody (e.g., anti-HA1 antibody, anti-HA2 antibody), anti-tumor antigen antibody (e.g., anti-p53 antibody, anti-kras antibody, anti-PRL-3 antibody), anti-immune checkpoint antibody (e.g., anti-PD1 or PDL1 antibody), anti-melanin synthesis-related antibody (e.g., tyrosinase-related protein TYRP1 antibody), anti-novel coronavirus antibody (e.g., anti-novel coronavirus S protein antibody, such as anti-S protein RBD or S1 or S2 antibody);
preferably, the molecule of interest is a protein related to gene editing; preferably, the protein is selected from the group consisting of Cas9 protein, AcrIIA4 protein, Cas13 protein, Cre recombinase and Flip recombinase;
preferably, the molecule of interest is an active or traceable protein; preferably, the protein is selected from the group consisting of fluorescent protein (e.g., green fluorescent protein), toxin protein (e.g., endotoxin), cytokine (e.g., interleukin or interferon, such as IL10 or IPNγ), immunomodulatory protein (e.g., PD1), enzyme (e.g., luciferase, nuclease, recombinase, methylase, protein kinase, etc.), signaling pathway-related molecular protein (e.g., β-catenin protein), cyclin (e.g., Cyclin D1 protein), transcription activator and transcription inhibitor;
preferably, the molecule of interest is a protein capable of binding a DNA molecule; preferably, the protein is selected from the group consisting of zinc finger protein and transcription activator-like effector nuclease (TALEN protein);
preferably, the molecule of interest is a polypeptide having an amino acid sequence selected from the group consisting of SEQ ID NOs: 1, 6-7, 43-44 and 46-53.

12. A multimer, comprising the fusion protein according to claim 10 or the conjugate according to claim 11.

13. A complex, comprising the fusion protein according to claim 10 or the conjugate according to claim 11 or the multimer according to claim 12, and a component non-covalently bound or complexed with the fusion protein or conjugate or multimer;
preferably, the fusion protein or conjugate or multimer comprises a protein capable of binding a DNA molecule, which is linked to the cell-penetrating peptide or truncate thereof; and, the complex comprises a DNA molecule, which non-covalently binds or complexes with a protein capable of binding the DNA molecule;
preferably, the protein capable of binding the DNA molecule is covalently linked (optionally, via a linker) or non-covalently (optionally, via a linker) to the cell-penetrating peptide or truncate thereof;
preferably, the protein capable of binding the DNA molecule is linked to the N-terminal or C-terminal of the cell-penetrating peptide or truncate thereof;
preferably, the DNA molecule comprises a nucleotide sequence that is recognized and bound by the protein capable of binding the DNA molecule;
preferably, the protein capable of binding the DNA molecule is selected from the group consisting of zinc finger protein and transcription activator-like effector nuclease (TALEN protein); preferably, the nucleotide sequence is a binding sequence (e.g., SEQ ID NO: 54) of zinc finger protein.

14. An isolated nucleic acid molecule, comprising a nucleotide sequence encoding the cell-penetrating peptide or truncate thereof according to any one of claims 1-9 or the fusion protein according to claim 10.

15. A vector (e.g., a cloning vector or an expression vector), comprising the isolated nucleic acid molecule according to claim 14.

16. A host cell, comprising the isolated nucleic acid molecule according to claim 14 or the vector according to claim 15.

17. A method for preparing the cell-penetrating peptide or truncate thereof according to any one of claims 1-9 or the fusion protein according to claim 10, comprising, culturing the host cell according to claim 16 under conditions allowing the expression of the cell-penetrating peptide or truncate thereof or the fusion protein, and recovering the cell-penetrating peptide or truncate thereof or the fusion protein from a culture of the cultured host cell.

18. A composition, comprising the fusion protein according to claim 10 or the conjugate according to claim 11 or the multimer according to claim 12 or the complex according to claim 13 or the isolated nucleic acid molecule according to claim 14 or the vector according to claim 15.

19. A pharmaceutical composition, comprising the fusion protein according to claim 10 or the conjugate according to claim 11 or the multimer according to claim 12 or the complex according to claim 13, and a pharmaceutically acceptable carrier or excipient, wherein the fusion protein or the conjugate or the multimer or the complex comprises a therapeutically active polypeptide that is linked to the cell-penetrating peptide or truncate thereof;
preferably, the therapeutically active polypeptide is covalently linked (optionally, via a linker) or non-covalently (optionally, via a linker) to the cell-penetrating peptide or truncate thereof;
preferably, the therapeutically active polypeptide is linked to the N-terminal or C-terminal of the cell-penetrating peptide or truncate thereof.

20. A method for transmembrane delivery of a molecule of interest into a cell, comprising, linking the cell-penetrating peptide or truncate thereof according to any one of claims 1-9 to the molecule of interest, then contacting the molecule of interest with the cell;
preferably, the method comprises: (1) linking the molecule of interest to the cell-penetrating peptide or truncate thereof to obtain a conjugate; (2) contacting the conjugate with the cell, thereby delivering the molecule of interest into the cell through transmembrane delivery;
preferably, the molecule of interest is linked to the cell-penetrating peptide or truncate thereof by covalent binding, affinity binding, intercalation, coordinate binding, complexation, binding, mixing or addition;
preferably, in step (1), the molecule of interest is directly covalently linked to the cell-penetrating peptide or truncate thereof; or, the peptide of interest is covalently linked to the cell-penetrating peptide or truncate thereof through a linker (e.g., a bifunctional linker or a peptide linker);
preferably, in step (1), the molecule of interest is non-covalently linked to the cell-penetrating peptide or truncate thereof;
preferably, the molecule of interest is as defined in claim 11;
preferably, the molecule of interest is a peptide of interest, and the method comprises: (1) linking the molecule of interest with the cell-penetrating peptide or truncate thereof to obtain a fusion protein; (2) contacting the fusion protein with the cell, thereby delivering the molecule of interest into the cell through transmembrane delivery;
preferably, in step (1), the peptide of interest is directly covalently linked to the cell-penetrating peptide or truncate thereof; or, the peptide of interest is linked to the cell-penetrating peptide or truncate thereof through a peptide linker (e.g., a flexible peptide linker), so as to obtain a fusion protein;
preferably, in step (1), the cell-penetrating peptide or truncate thereof is linked to the N-terminal (optionally, via a peptide linker) or C-terminal (optionally, via a peptide) of the peptide of interest;
preferably, the peptide of interest is as defined in claim 10;
preferably, the molecule of interest is a nucleic acid of interest, and the method comprises: (1) linking a protein capable of binding the nucleic acid of interest to the cell-penetrating peptide or truncate thereof; (2) contacting the product of step (1) with the nucleic acid of interest to obtain a complex; and (3) contacting the complex with a cell, thereby delivering the nucleic acid of interest into the cell through transmembrane delivery;
preferably, the molecule of interest is a nucleic acid of interest, and the method comprises: (1) adding to the nucleic acid of interest a nucleotide sequence that can be recognized and bound by a DNA-binding protein; (2) linking the DNA-binding protein to the cell-penetrating peptide or truncate thereof; (3) contacting the product of step (1) with the product of step (2) to obtain a complex; and (4) contacting the complex with the cell, thereby delivering the nucleic acid of interest into the cell through transmembrane delivery.

21. Use of the cell-penetrating peptide or truncate thereof according to any one of claims 1-9 for transmembrane delivery of a molecule of interest into a cell.

22. Use of the cell-penetrating peptide or truncate thereof according to any one of claims 1-9 in the manufacture of a kit for transmembrane delivery of a molecule of interest into a cell.
